Europäisches Patentamt

European Patent Office

Office européen des brevets

(11) Veröffentlichungsnummer: **0 301 165**
**A2**

## (12) EUROPÄISCHE PATENTANMELDUNG

(21) Anmeldenummer: **88103775.8**

(22) Anmeldetag: **10.03.88**

(51) Int. Cl.⁴: **A61B 5/00 , A61M 5/14**

Die verdeckten Patentansprüche bzw. "Wesensmerkmale" Nr. 1-18, 20, 22, 23, 25-27, 29-32, 34-40, 42-87, 89-95 und 97-130 gelten durch Nichtzahlung der Anspruchsgebühren als verzichtet (Regel 31 (2) EPÜ).

(30) Priorität: **10.03.87 DE 3708031**
**17.03.87 DE 3708857**
**22.04.87 DE 3713846**
**08.09.87 DE 3730469**
**26.02.88 DE 3806574**

(43) Veröffentlichungstag der Anmeldung:
**01.02.89 Patentblatt 89/05**

(84) Benannte Vertragsstaaten:
**AT BE CH DE ES FR GB GR IT LI LU NL SE**

(71) Anmelder: **Wagner, Wolfgang, Dr.med.**
**Exerzierstrasse 1**
**D-1000 Berlin 65(DE)**

(72) Erfinder: **Wagner, Wolfgang, Dr.med.**
**Exerzierstrasse 1**
**D-1000 Berlin 65(DE)**

(54) **Einrichtung zur Stoffwechselkontrolle.**

(57) Einrichtung in Fortsetzung der Offenlegungsschrift WO 86/01728 und der Japanischen Anmeldung J 211352/86 zur Stoffwechselkontrolle insbesondere bei Zukkerkranken, welche die Substanzmessung, insbesondere der Blutglukose, allein, aber auch in Kombination mit einem von den Substanz-Meßergebnissen beeinflußten Injektor zum Gegenstand hat bei Einsatz einer Steuervorrichtung und eines Schaltgetriebes für den automatischen Funktionsablauf -wegen der offenbar noch nicht ausreichend gelösten Problematik der verletzungsfreien Substratbestimmung mittels Laser-Lichtes- bevorzugterweise über den Einsatz einer Art Sensorkanüle (oder eines Punktionsgriffels) zur verletzungsarmen Absaugung von Blut unter Anwendung eines Sogkanals -bevorzugterweise als Druckausgleichskanal bei Einsatz einer Saugglocke in Verbindung mit einer verläßlichen Unterdruckquelle um nach gesteuerter Einwirkungszeit des Blutes innerhalb einer Reaktionskammer auf eine Indikatorschicht bei einer hier bevorzugten optischen Version- das Blut von derselben zu entfernen und Lichtmeßwertänderungen der Indikatorschicht an einen Reflexphotometer zu vermitteln, welcher sich in getrenntem Gehäuse befinden kann; im Falle vorgesehener Stoffwechselverbesserung durch Injektion bevorzugt durch denselben der Blutgewinnung dienenden Kanülenträger über wenigstens eine vorzugsweise kanülennahe schlauchintegrierte Dosiervorrichtung bei Vorhandensein wenigstens zweier gleichwertiger Flüssigkeitsbehälter mit Ventilkupplung je Arzneisorte für eine restarme Verabreichung unterschiedlicher Flüssigkeitsmengen, unter Einschluß einer auch reflexphotometrischen Kontrolle zur Vermeidung einer Punktion krankhaft veränderter Haut.

## Vorrichtung zur Stoffwechselkontrolle:

### Einleitung zum Stand der Technik:

Die Erfindung bezieht sich auf das Gebiet der Medizintechnik, spezieller auf das Gebiet der Stoffwechseldiagnostik und Injektionstherapie in erster Linie des Diabetes mellitus beim Menschen.

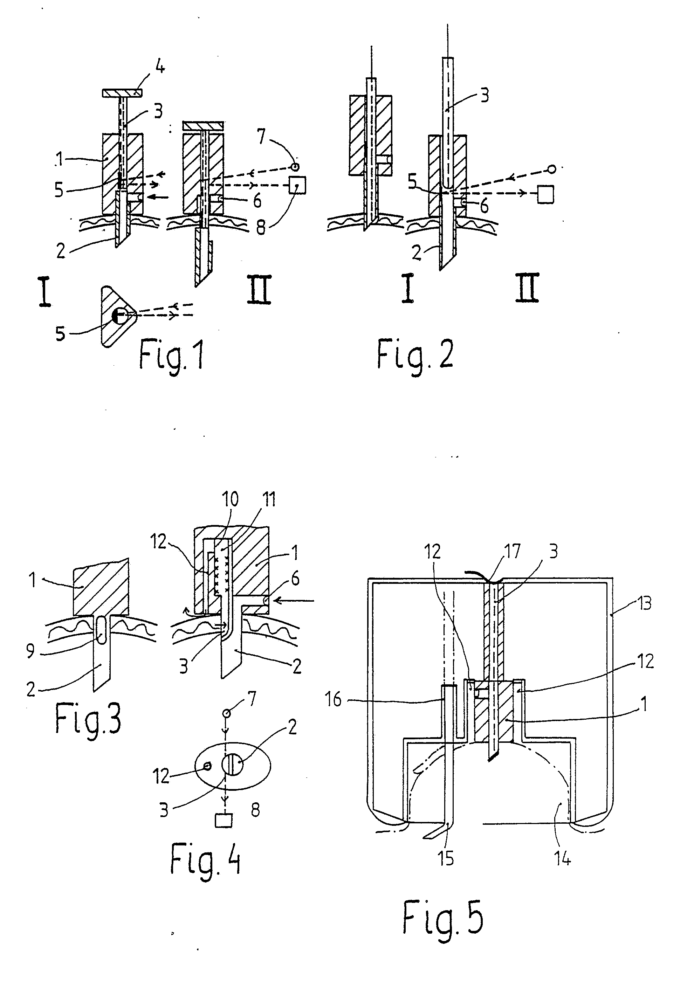

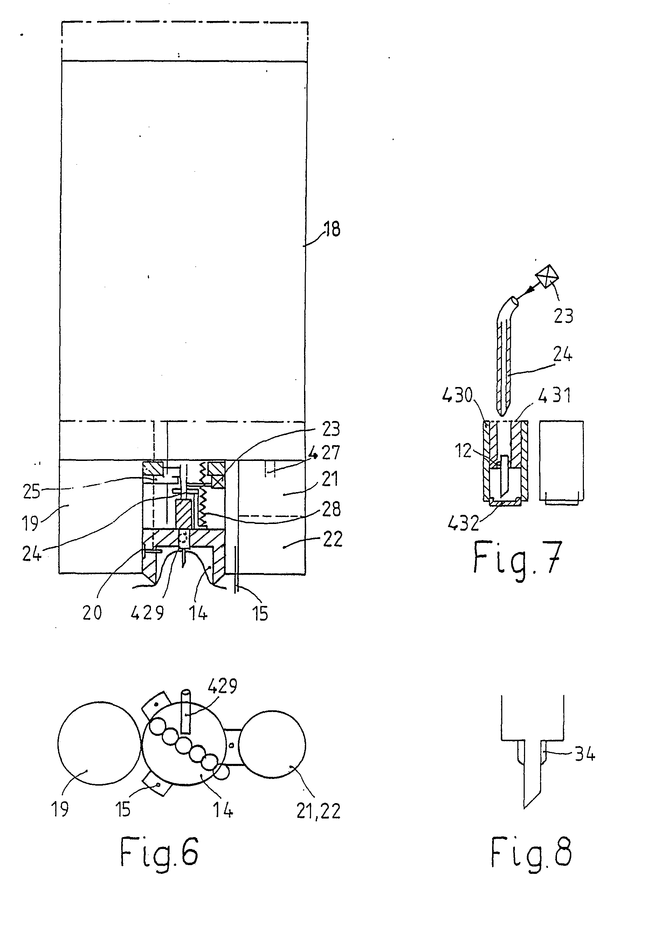

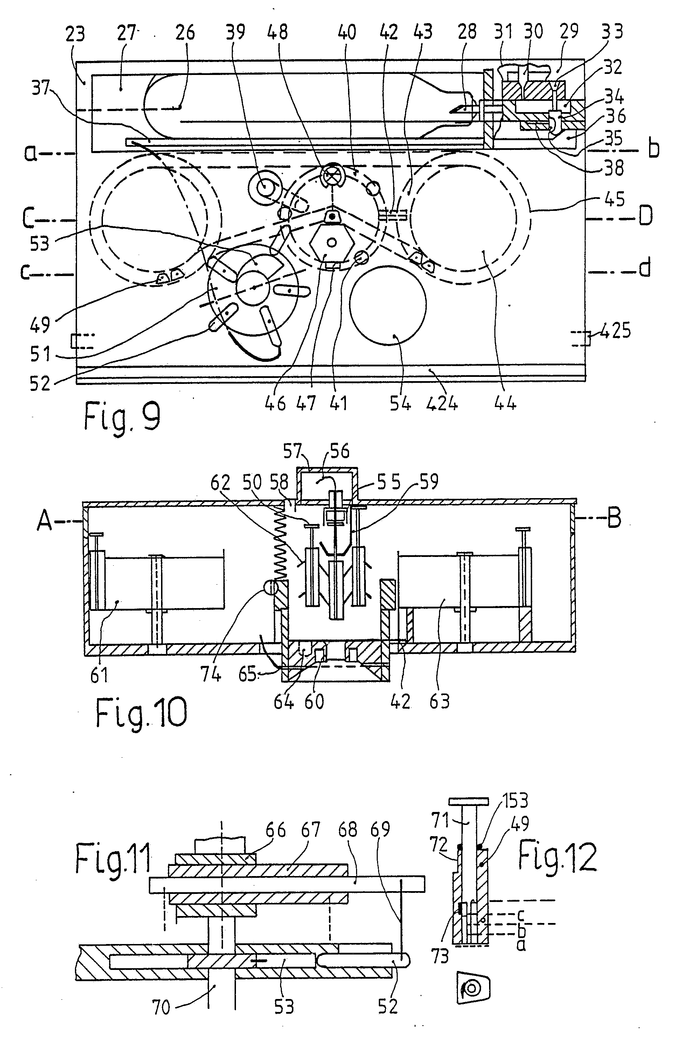

Die Technik der Sauginjektion wurde zunächst für die Anwendung an Schafen entwickelt, generell ist sie etwa seit 1932 bekannt, Überlegungen zum Einsatz beim Menschen beginnen mit den Anmeldungen DE P 25 51 991 "Dosierende Arzneispender für die perorale und Injektionsbehandlung"(1), P 25 51 992 "Dosierte Injektion aus Vorratsbehältern"(2) und P 25 51 993 "Sauginjektion mit Dosiervorrichtungen nach dem Wegwerfprinzip"(3) am 17.11.75 mit Priorität aus Anmeldungen in Großbritanien. In wenig glücklicher Beschreibung finden sich da Bemühungen, das Gebot der Sterilität zu berücksichtigen und dem Ziel der Einzelanwendung über längere Zeit näherzukommen. Es finden sich die verschiedensten Arten der Vakuumerzeugung von der Wasserstrahlpumpe(Fig.3, (3), der Gasstrahlpumpe(Fig.5), der druckgasbetriebenen Kolbenpumpe(Fig.11) und der federbetriebenen Pumpe mit Dreipunktauslöser vom Saugglockenrand aus (Fig. 12); auch die vertikal gestappelten "Säckchenkanülen" sind zu nennen(Fig.10a) bei horizontaler Reihung von Speichertöpfchen(Fig.12). Die Vakuumspeicherung wird prinzipiell zu Fig.5 (3) angegeben.

In (2) wird unter Fig.3 eine vorgefertigte Vakuumspeicherung in einer Kanüle beschrieben, um durch Blutrückfluß die sichere Lage der Kanüle im Blutge fäß beurteilen zu können, ohne mit einer Spritze einen Sog erzeugen zu müssen. Der Erfindungsgedanke war schon damals der, die Arznei luftfrei im Behälter zu stapeln und von dort durch eine Art Druckgaspolster innerhalb eines Kolbens, Faltenbalges oder einfach einer Gummiblase verdrängen zu lassen und nach Zwischenschaltung einer Dosiervorrichtung direkt in den Körper aus dem Behälter zu injizieren(2)(Fig.2a in(3). P 26 15 062 befaßte sich mit der "Grenzflächensicherung in Arzneidruckspeicherbehältern", P 26 20 358 brachte in den Fig.4 und 5 die Verschiebung der Saugglocke gegenüber einer feststehenden Kanüle(eine erste Lösung zur verletzungsfreien Hautdistanzierung nach der Injektion), P 26 50 450 befaßte sich mit der Längenverkürzung des Injektors durch teleskopartiges Ineinanderschieben von Teilen. P 27 40 328 vom 5.9.77 bringt dann eine Stufenspritze (ein Prinzip das inzwischen für Diabetiker im Handel ist); in Fig.10 findet sich ein automatischer Kanülenwechsel über einen schieber von vorne. P 30 19 589 und 32 05 827 greifen nochmals das Thema vom Speichervakuum auf, was dann im Europa-Patent 0103664 (erteilt am 30.12.86) sich wiederholt.

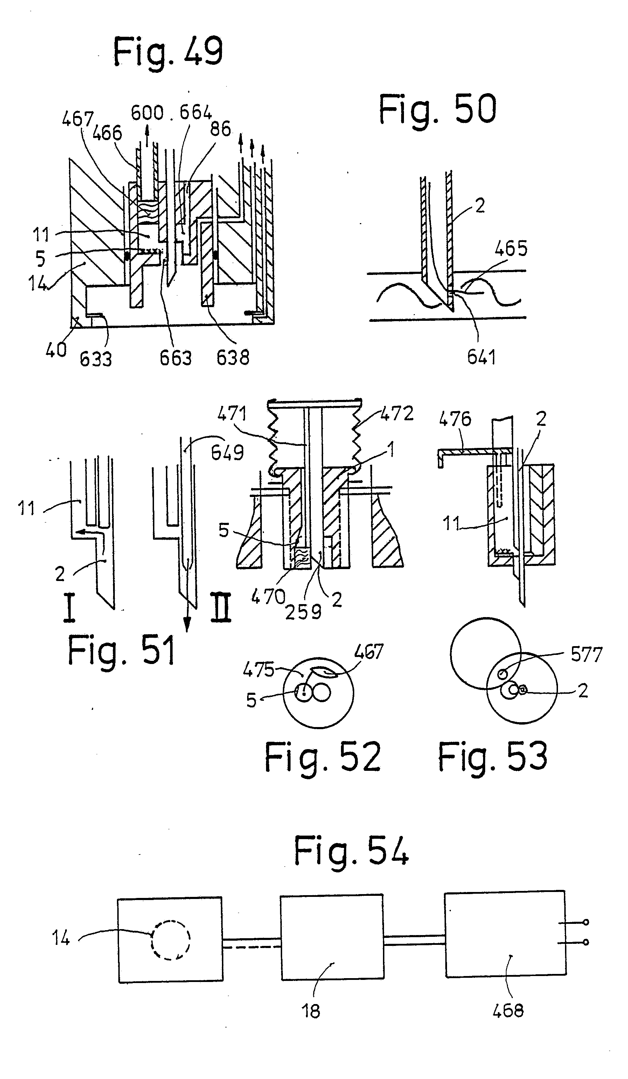

Zu dem heiklen Thema der "Compliance" der Mitarbeit des Patienten könnte auf P 2240 410 vom 14.8.72 zurückgegriffen werden, so es darum geht, Arzneimittel aus einem Behälter dosiert direkt in den Mund hinein unter Schluckaktkontrolle (nach Identitätskontrolle) abzugeben. Die aufgeworfenen Fragenkomplexe werden in der PCT-Anmeldung, veröffentlicht als WO 86/01728 vermehrt um das Problem der Stoffwechselkontrolle zugleich mit der Injektions mittels einer Sensorkanüle. In den Fig.50-56 werden schon Überlegungen zu einer optischen Lösung der Glukosebestimmung angestellt, ein Zugpflaster findet sich in Fig.104, während eine schlauchintegrierte Dosierpumpe erst in der erst in Japan veröffentlichten Schrift 211356/86 auftaucht, aber Reibungsprobleme aufwirft.

### Aufgabe:

Für die Stoffwechselkontrolle, insbesondere zur Diagnostik und Therapie des Diabetes, stellte ich mir in WO 86/01728 die Aufgabe, eine Einrichtung zu schaffen, welche es möglichst schmerzfrei gestattet, das Ergebnis häufiger Stoffwechseluntersuchungen -eventuell auch zeitproportional- sichtbar bzw.wahrnehmbar zu machen, wo erforderlich ergänzt durch in der Regel nahezu gleichzeitig mit den Messungen erfolgende, die Stoffwechsellage verbessernden Einspritzungen bei Anpassung der Dosierung an die gewonnen Meßergebnisse und Ausdehnung der Registrierung auf möglichst alle für die Injektionstherapie erheblichen Daten. Hierfür sind unentbehrliche Voraussetzungen die Verbesserung eines Sensors für das Meßsystem und solche Verbesserungen, welche eine Verkleinerung der Apparatur möglichst bis zum handlichen Taschenformat zulassen bei hoher Funktionssicherheit und leichter Bedienung, eine Aufgabe, welche eine erfinderische Verbesserung nicht nur vieler apparativer Einzelheiten, sondern auch von deren funktionellem Zusammenspiel unter Berücksichtigung der Natur besonders des Menschen zur Voraussetzung hat und damit oft auch eine Erweiterung des bekannten Oberbegriffes der Erfindung.

## Stand der Technik:

Die auf die Offenlegungsschrift WO 86/01728 basierende Erfindung weist noch erhebliche Mängel auf, denen diese Erfindung abhelfen soll.

Diese Mängel betreffen in erster Linie den Sensor, welcher der Gewinnung von Meßdaten im Körper dient. Selbstverständlich wäre die verletzungsfreie Messung mittels Laser-Lichtstrahles vorzuziehen, und es werden hier zu den bisherigen Angaben weitere Verbesserungsvorschläge gemacht.

Die "höhenverstellbare" gleitende Anordnung der die Haut innerhalb einer Saugglocke vermessenden Lichtschranken bedingt unnötigen Aufwand ebenso wie die "Hautvermessung" überhaupt.

Trotz solcher Verbesserungen erscheint es aber derzeit noch zweifelhaft, ob die direkte Stoffwechseluntersuchung mittels Laserlichtes nicht zu aufwendig für den Masseneinsatz am Patienten sein wird.

Es war deshalb vornehmliche Aufgabe vorliegender Erfindung, für die Messung eine Sensorkanüle zu schaffen, welche möglichst eng an den augenblicklichen Stand der Technik anknüpft. So sind Reflexphotometer für die optische Glukosebestimmung weit verbreitet, welche die Farbindikatorveränderungen eines Teststreifens benützen.

In WO 86/01728 ist zu Figur 53 ein in die Kanüle eingelegter Docht (oder eine Kapillare) mit Indikatorbeschichtung als Meßschicht beschrieben, bei welchen -mit Blut oder Körperflüssgkeit in Kontakt gebracht- die Farbänderung außerhalb der Kanüle reflexphotometrisch ausgewertet werden sollen; die offene Mulde der Fig.53 kann analog Fig.51 sinngemäß auch fehlen; denn die Kombination von Einzelmerkmalen der Erfindung konnte natürlich nicht in ganzer Breite beschrieben werden. Die in WO 86/01728 angegebenen Sensorkanülen sind in der angegebenen Anwendungsweise eigentlich nur bei besonderen Krankheitsumständen (wie Ödem oder Blutansammlung unter der Haut) einsetzbar.

Vorkehrungen gegen vorzeitige Abhebelung der Saugglocke von der Haut bei der Gerätehandhabung sind in J 211352/86 ebenso angegeben, wie Vorrichtungen zur Erkundigung der Eignung der Haut für Punktionszwecke. Viele dieser ersten Überlegungen zur Problemlösung blieben bis heute -insbesondere bezüglich der optischen Hautvermessung- noch zu umständlich und aufwendig.

Am wenigsten befriedigt die in WO 86/01728 beschriebene Lösung einer Nachfüllung nahezu entleerter Arzneibehälter zur Vermeidung des Verlustes größerer Restmengen bei wechselhaften Dosierungen.

## Lösung dergestellten Aufgabe:

Die gestellte Aufgabe wird entweder dadurch gelöst, daß messende Lichtstrahlen durch den in Höhe und Form mittels besonderer Vorrichtung für bestimmbare Zeit in Verbindung zum Gerät festgelegten Hautkuppenbereich geleitet werden durch welchen -falls zusätzlich beabsichtigt- die den Stoffwechsel korrigierende Flüssigkeit mittels einer Einspritzvorrichtung dosiert eingeführt wird.

Die gestellte Aufgabe kann aber auch dadurch gelöst werden, daß eine Art von Kanüle oder Punktionsgriffel mit Spitze dadurch als Sensor ausgebildet sind, daß sie so ausgestaltet werden, daß über eine Öffnung ihres durch die Haut eingeführten Schaftes wenigstens eine Hohlraumverbindung in eine Meßkammer für Gewebsflüssigkeits- oder Blutbestandteile führt, welche nach der Ableitung aus dem Kontaktbereich mit dem Körper mit Wirkstoffen in Kontakt gebracht werden, welche zur Erzeugung oder Vermittlung von Meßsignalen dienen; dabei erfolgt die Erzeugung dieser Signale vorzugsweise innerhalb eines Gehäusezusammenhanges mit einer Punktionsvorrichtung und und sind Punktionsgriffel oder Kanüle vorzugsweise in Mehrzahl gespeichert und werden nach überwiegend seitlich-paralleler Lagerung in Ketten -auch unverbunden- der Punktionsstelle zugeführt; die Fixation der Punktionsstelle auf der Haut erfolgt dabei bevorzugterweise durch eine erfindungsgemäß breit auf der Haut liegende Gehäusegestaltung während der Punktion und die Einrichtung ist -soweit eine Verbesserung des Stoffwechselzustandes mittels Einspritzung angestrebt wird, dadurch gekennzeichnet, daß die Einspritzung von wenigstens einer Flüssigkeit über wenigstens eine Dosiervorrichtung erfolgt, wobei für die Lagerung je einer Flüssigkeitssorte wenigstens zwei Behälter gleicher wesentlicher Beschaffenheit vorhanden sind, von denen nach Verbrauch des einen über eine Ventilvorrichtung der andere seine Flüssigkeit zur Injektion abgibt und wobei bestimmungsgemäß der jeweils verbrauchte Behälter durch einen neuen ersetzt wird, so daß ohne Verlust einer erheblichen Restmenge jederzeit eine beliebige Dosis eingespritzt werden kann. Vorzugsweise erfolgt die Hautpunktion bei einer mittels Zugwirkung hochgehobenen Haut; diese Zugwirkung kann beispielsweise über eine Adhäsivschicht, über eine Art Pflasterzug hervorgerufen werden, vorzugsweise aber mittels Sogeinwirkung auf die Haut innerhalb einer Saugglocke. Vorallen hierbei kann die Stärke der Sogwirkung reguliert und der Empfindlichkeit der Haut unter Verwendung eines verstellbaren Ventiles angepaßt werden. Eine optische Überwachung der Zug-oder Klemmwirkung (wo die Haut mittels seitlicher Klemmbacken fixiert wird) auf die Blutkapillaren ermöglicht eine Beschränkung der mechani-

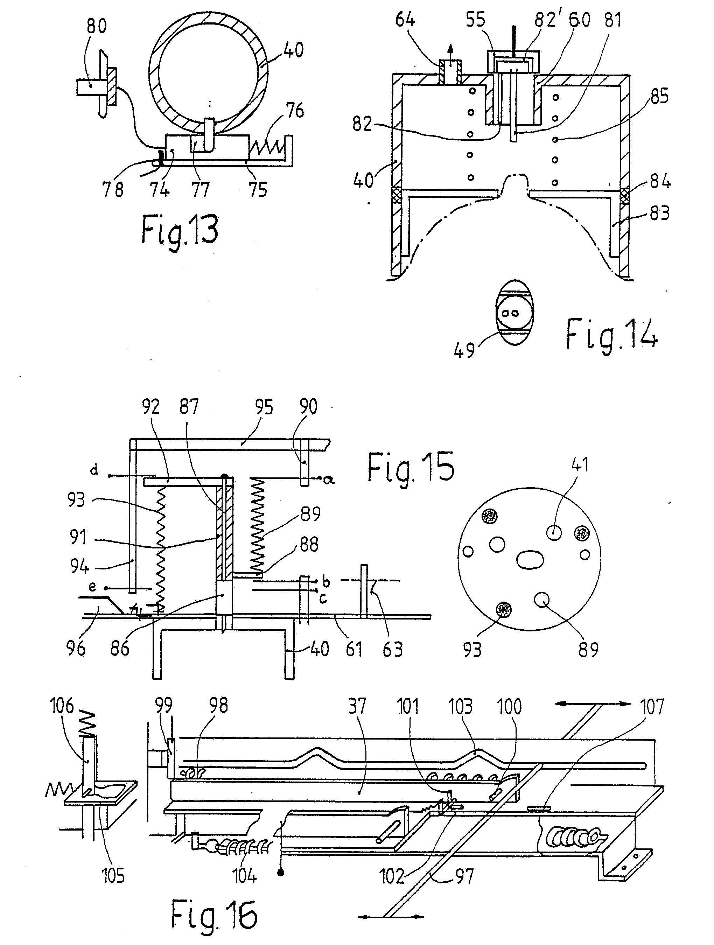

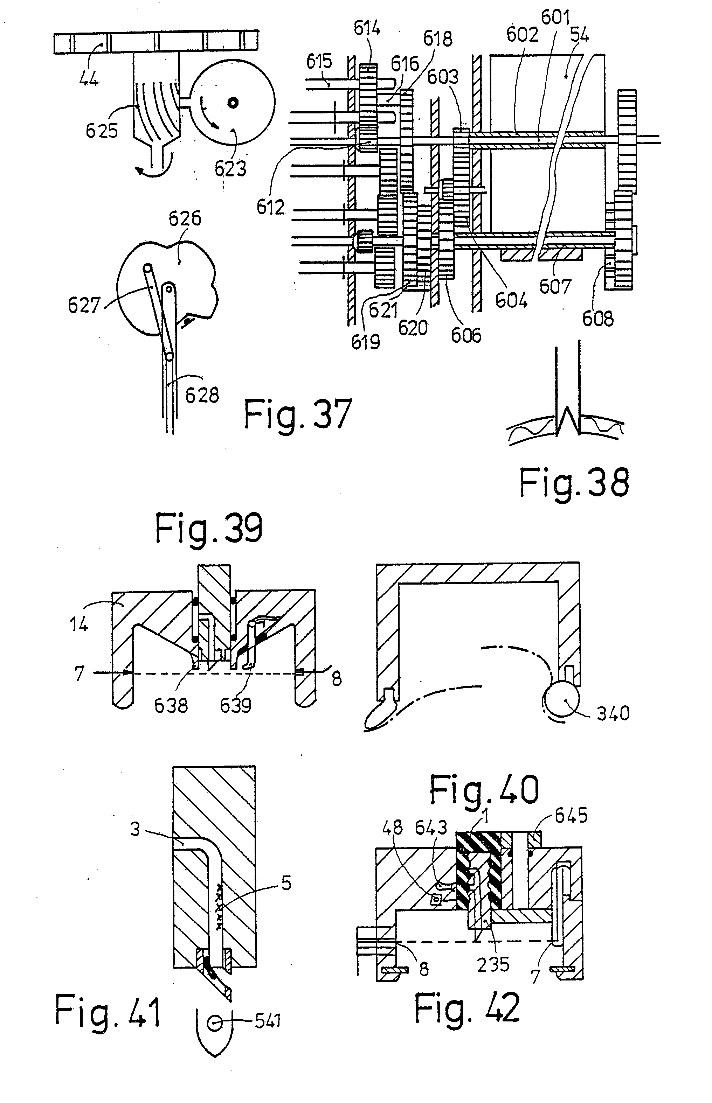

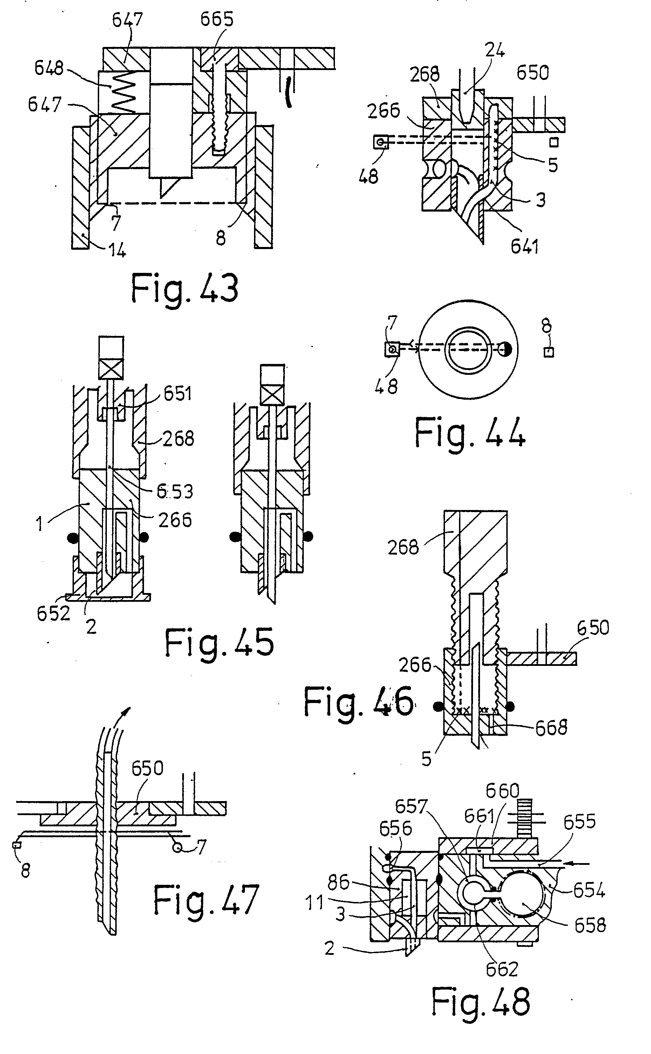

schen Hautbeanspruchung. Die Kanülenspitze oder die Spitze des Punktionsgriffels soll in den meisten Erfindungsbeispielen für die Materialgewinnungsphase lediglich bis in die Haut eindringen, was durch einen im Falle möglicher Injektion eventuell auf dem Kanülenschaft verschieblichen Teller hinter der Anschliffschräge oder durch nur kurzes Überragen des Kanülenendes über den unteren Punktionsgriffelrand oder durch zeitweise Abdeckung des Schaftes hinter der Kanülenöffnung mittels einer Art Hülse als Geräteteil und anders bewirkt werden kann. Da die Eindringtiefe der Haut in eine Saugglocke erheblich wechseln kann, und zwar beim Einzelwesen je nach Körperstelle und auch bei den verschiedenen Hautbeschaffenheiten verschiedener Personen, muß die Höheneinstellung der Kanülenspitze in vielen Fällen variiert werden. Dies geschieht dann erfindungsgemäß entweder dadurch, daß neben der durch den Punk tionsgriffel (hier und künftig oft gleichbedeutend mit Kanüle oder deren Abstützvorrichtung für die Haut)tief in das Zentrum der Saugglocke hineinragt und neben dem Zentrum genügend Platz für ein wulstartiges Ausweichen der unter Sogwirkung stehender Haut aufweist(Fig.39 u. 49). Eine zweite Lösung sieht die Annäherung des Punktionsgriffels oder einer zentralen Trägerhülse des Saugglockendaches an die bereits hochgezogene Hautkuppe vor (Fig.42 u.46); eine dritte Lösung beinhaltet die Heranführung des Saugglockendaches nach der Hautkuppenbildung-(Fig.14, Fig.43).

In letzterem Falle wird bevorzugterweise die Anordnung der optischen Testvorrichtung(Strahler und Sensor) zugleich mit dem Dach gesenkt. Auch die Absenkung einer zentralen Partie des Daches zusammen mit der seitlichen Trägerhülse der Lichtschrankenelemente ist zu erwähnen(Fig. 42), um nur einen Teil der ebenfalls schutzwürdigen Kombinationen zu erwähnen.

Um das Ausweichen der hochelastischen Haut vor der kurzen Kanülenspitze zu vermeiden, kann die Haut im Kuppenbereich durch eine Art Hülse vorgespannt werden; zur Ausnutzung der Kapillarüberfüllung in der wulstartig das Zentrum umgebenden Hautglocke ist die Aufhebung der Randklemmwirkung an jenem Hülsenrand nach der Punktion erforderlich, was durch Senkung des Punktionsgriffels bewirkt werden kann-(Fig.43,Fig.49).

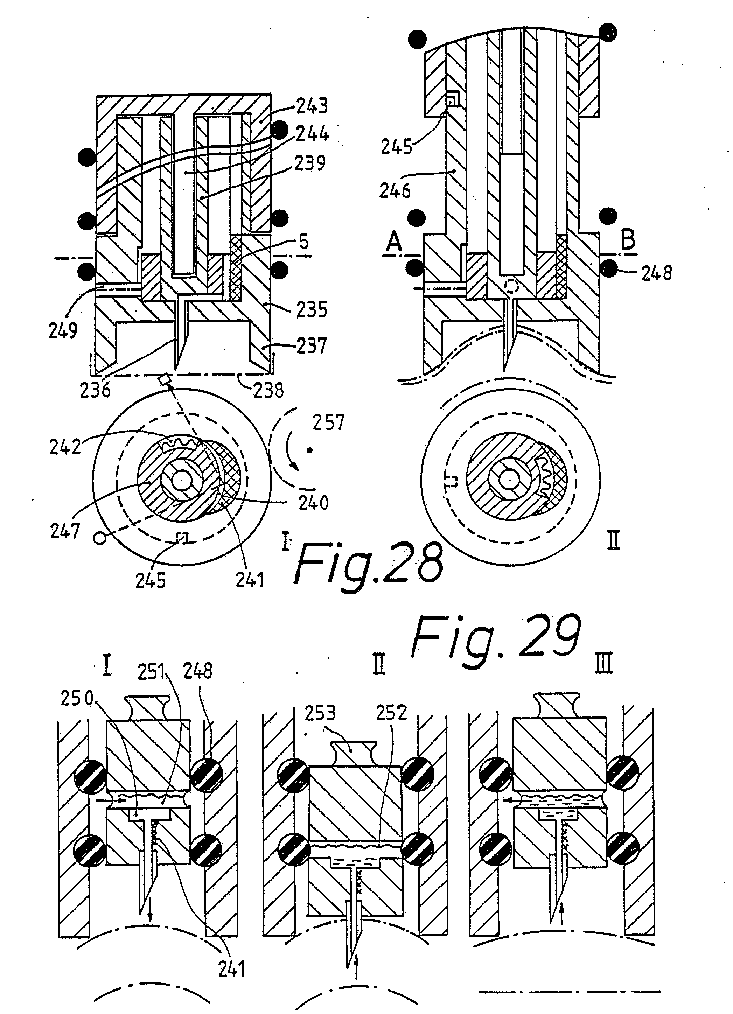

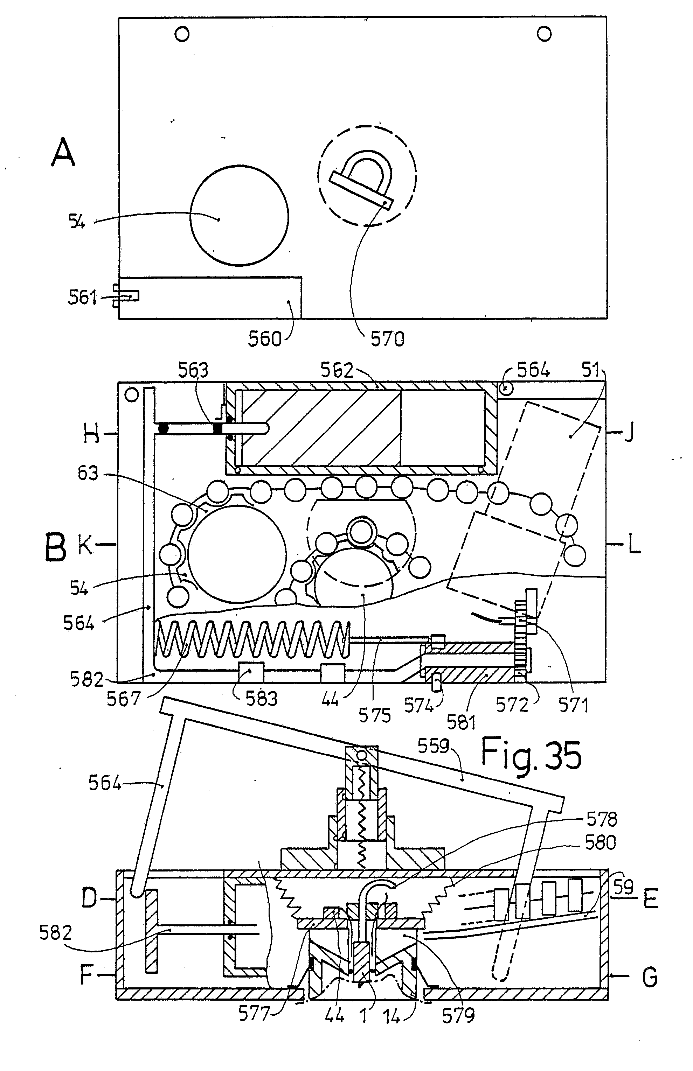

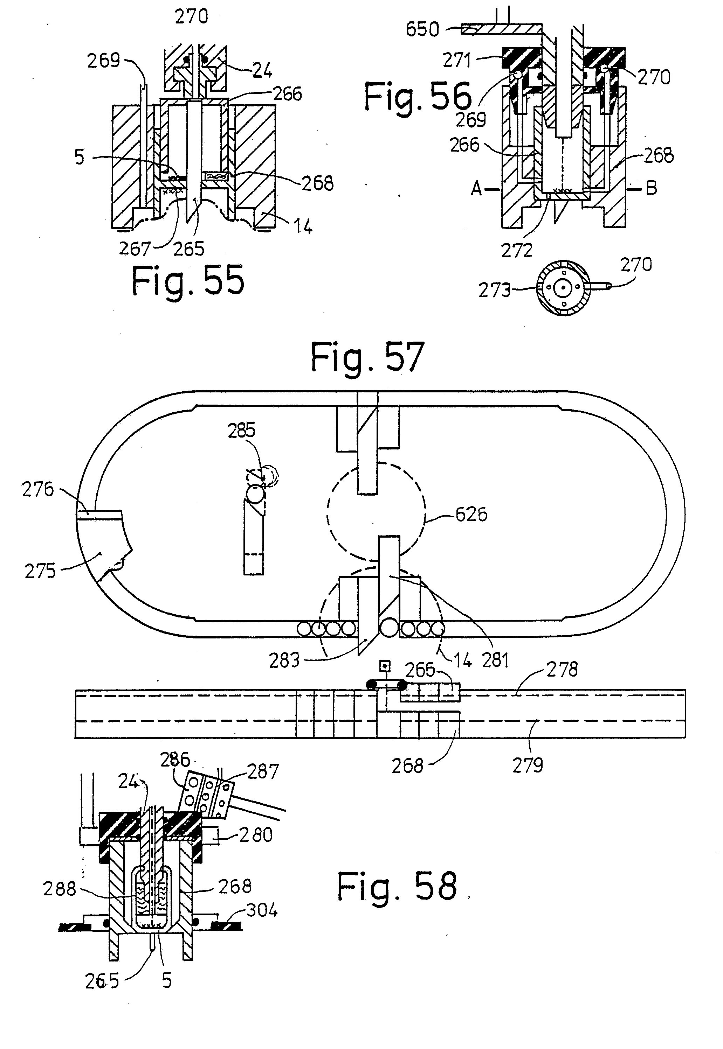

Das Hineinschlüpfen der Haut in eine Art Saugglocke unter Zugwirkung darf nicht durch den von der Willkür des Anwenders abhängigen Andruck des Saugglockenrandes abhängig sein, sondern wird tunlich über eine federgesteuerte Annäherung der Saugglocke bewirkt(Fig.35 u.36), wobei die Gehäuseabstützung mehr außerhalb des Saugglockenrandes erfolgt. Um ein vorzeitiges Abhebeln des Saugglockenrandes zu vermeiden und zur Erhöhung der Handlichkeit wird die Saugglocke mehr zentral der Breit seite des Gehäuses angeordnet vorzugsweise innerhalb einer Konkavität der Gehäuseoberfläche, welche auch dadurch in Anlehnung an die Wölbung von Körperteilen erzeugt werden kann, daß die Funktionselemente in parallel und gelenkartig miteinander verbundenen mehr oder weniger starren Gehäuseteilen angeordnet sind. Der Platzbedarf kann die Aufteilung der Bauelemente auf zwei während oder nach der Anwendung am Körper über Koppelungselemente miteinander in Verbindung zu setzende trennbare oder getrennte Gehäuse nötig machen. Die neue Gehäuseform bedingt für den Punktionsgriffelwechsel deren seitliche Anordnung innerhalb kreisförmiger oder schieberartiger Magazine oder vorzugsweise in kettenförmiger Anordnung auch ohne gesonderten Magazinbehälter zur Einführung in das Gehäuse. Dabei können Punktionsgriffel oder Kanülen innerhalb von jeweils einer gesonderten Hülse angeordnet sein, welche gegenseitig auch über (eventuell)elastischen Fäden untereinander verbunden sein können. Die Abdeckung des Punktionsgriffels gegen Staub kann durch eine elastische oder leicht zerstörbare Membran gebildet werden, welche von einem Stift oder einer Röhre durch brochen werden kann; aber auch Deckel können eingesetzt werden, insbesondere, wenn sie über eine Art Schwenkgelenk oder eine Fadenverbindung mit dem Griffelkörper auch nach Entfernung von Öffnungen (zur Entsorgung) verbunden bleiben. Der Kanülentransport in der Senkrechten über der Saugglocke erfolgt über einen Schiebestift oder ein Schieberohr, deren Ende entweder in eine trichterförmige Bohrung des Griffelkörpers eindringt oder der diesen (oder ein tellerartig verbreitertes Schaftende) kappenartig umfaßt. Dabei können Griffel und Schaft stufenweise gesenkt und wieder zurückgezogen werden. In vereinfachter Konstruktion eines unterdruckspeichernden Töpfchens mit Saugglocke kann eine Kanüle (oder Punktionsspitze) zur Blutentnahme verwendet werden; das Blut kann nach Abnahme des Töpfchens von der Haut ausgeblasen und der Messung zugeführt werden. Um eine Probe aus dem Körper innerhalb einer Saugglocke entnehmen zu können muß die Gegenöffnung zur Entnahmeöffnung der Kapillare in Verbindung mit dem Unterdruckraum stehen, um eine Fehlableitung des Blut-oder Flüssigkeitsstromes von der Verletzungsstelle auf die Haut zu vermeiden und die Kapillarfüllung möglich zu machen. Der Kontakt der entnommenen Körperbestandteile mit den Wirkstoffen oder Agentien kann vorzugweise innerhalb des Punktionsgriffels erfolgen. Es wird dabei unter Zurückhaltung von störenden Blutkörperchen eine Ultrafiltration durch eine Filtermembran beschrieben(Fig.48), wodurch im Gegenkompartiment eine Vermischung des Ultrafiltrats mit Reagentien ermöglicht wird und nach bestimmter Einwirkungszeit die Substratmessung beispielsweise im UV-Licht oder die Reflexfotometrie mit Laserlicht. Es kann aber auch Vollblut auf eine Farbindikatorschicht oder Meßzone (5) aufgebracht werden, insofern eine Vorrichtung vorhanden ist, die verdeckenden korpuskulären Bestandteile vor der Messung zu entfernen. Dies geschieht entweder dadurch, daß -beispielsweise mit der Höhenverschiebung des Schaftes gekoppelt und beispielsweise durch eine Art Faden- die Farbindikatorschicht abgewischt wird(Fig.52); es kann aber auch ein Luftzug mit oder ohne Vibration der Indikatorschicht erzeugt werden, welcher die Oberfläche der Farbindikatorschicht von Auflagerungen befreit. Vorzugsweise geschieht dies über eine Sogverbindung der Abnahmekapillare oder - noch besser-deren Verbindungskanal zum Vakuumraum(Fig.57)), indem nach der Blutansaugung die Kanalverbindung zum Vakuumraum für die Zeitdauer der gewünschten Bluteinwirkung auf die Farbindikatorschicht unterbrochen wird und wobei in einer für die Diagnostik bevorzugten Variation noch zusätzlich ein Vakuum-Reserveraum auch nach Wiederbelüftung der Saugglocke aufrechterhalten wird(Fig.29), welcher eine Schichtreinigung auch nach Trennung des Gerätes von der Haut bewirkt. Die Abweichung des Punktionsgriffelquerschnittes von der Kreisform (Fig.1) erlaubt es, auch innerhalb des Gerätes - bespiesweise am Kanülenschaft oder an einer Kapillare vorbei- den mittels Lichtleitfasern gebündelten Meßstrahlen den richtigen Auftreffwinkel auf die Farbindikatorschicht (Meßzone) darzubieten, so daß die Messung auch innerhalb des Gerätegehäuses erleichtert wird. Es kann hierzu auch eine ringförmig beschränkte Profiländerung des Griffels (vorallem für die Schieführung) dienen. Bei rotierenden Punktionsgriffeln kann die richtige Winkeleinstellung des Griffels zum Meßstrahl durch die Dämpfung von Suchstrahlen kontrolliert und die Rotation im richtigen Augenblick dann unterbrochen werden. Die Rotation zumindest der Anschliffzone der Kanüle, wobei an geeigneter Stelle im Schaftbereich auch eine gratförmige Schneidekante wenigstens vorhanden sein kann (oder die Rotation einer Lanzette) ist zweckmäßig, um eine für die Blutentnahme genügende Verletzung der Blutkapillaren zu erzielen. Dabei kann die Verletzung dadurch auf das kleinste Maß beschränkt werden, daß nach Überprüfung der Höhe der mittels einer Lichtschranke die Schneidbewegung abgebrochen wird( Fig.44, Querschnitt).

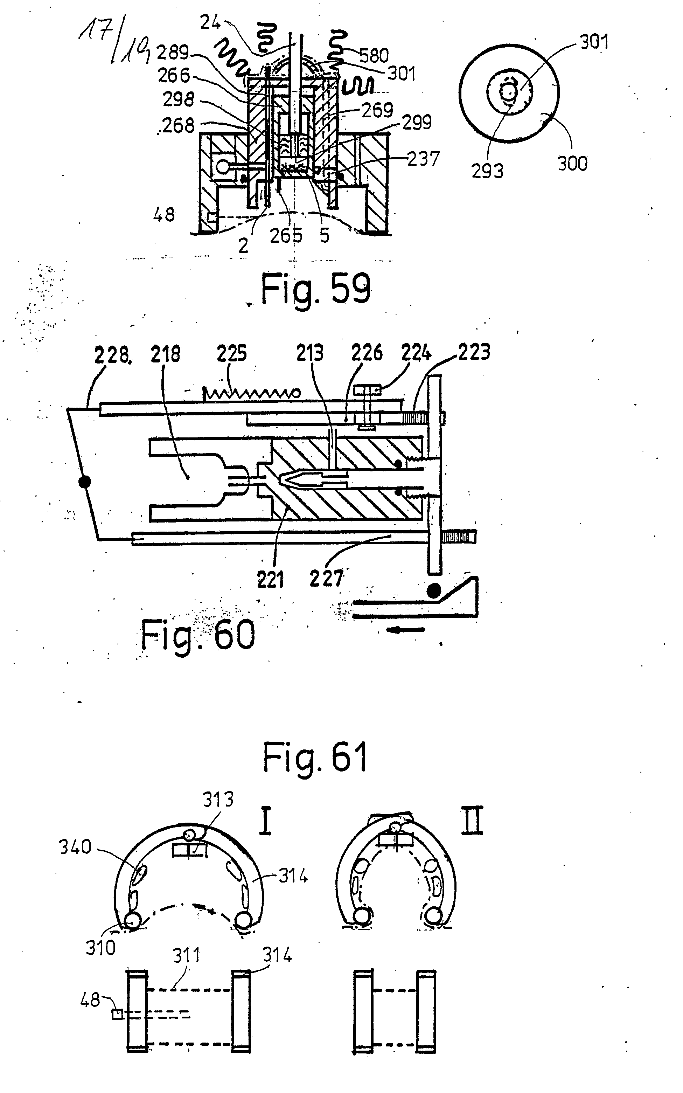

Vorteilhafterweise wird die Indikatorsubstanz der Meßzone in eine senkrechte von oben in den Kanülenkörper eingefügte sacklochartige Bohrung an deren Ende plaziert(Fig.49), die Reflexmessung erfolgt also von oben. Zur noch leichteren Justierung der Meßanordnung kann anstelle des exzentrischen Sackloches eine zentrale Bohrung in einer Trägerhülse für die Punktionsspitze -welche (auch wegen der geringeren Kosten) eine Lanzettspitze sien kann-seitenbeweglich innerhalb eines Aufnahmebechers angeordnet sein(Fig.60). Die für das Dichtungsproblem störende Rotation kann durch ein seitliches Ausschwingen(vorzugsweise der Trägerhülse für die Spitze) ersetzt werden. Die Blutaufnahme kann über Löcher an der Unterseite der Trägerhülse aus dem aus dem Lanzettstich austretenden Blutstropfen erfolgen. Diese Lösung berechtigt zur Hoffnung, daß derartige Vibrationskanülen, die funktionell aus einem stehenden und einem schwingenden Teil bestehen(wenn die Schwingung nicht über das Dach der Saugglocke vermittelt wird) auch in noch verkleinerter Einzelform im Gußverfahren hergestellt werden können, insbesondere wenn ein elastischer Werkstoff eingesetzt wird. In diesem Fall wird die Kanüle (oder auch der Punktionsgriffel ohne Injektionskanüle) von oben ausgeformt, und Aufnahmebecher und Trägerhülse bleiben über eine wenigstens zonenweise verdünnte Bodenmembran miteinander (und gegeneinander abkippbar im obigen Sinne) verbunden. Die Bodenlöcher, ja selbst seitliche tiefe Querdurchbrüche der Trägerhülsenwand zum seitlich unten im Aufnahmebecher liegenden Reserveraum, können entkernt werden. Dieser Reserveraum ersetzt dann den Wattering im dargestellten Beispiel und nimmt das Blut auf, welches aus dem zentralen Napf in der Meßzone übertritt oder dorthin nach der Einwirkungszeit ausgekippt wird. Ein besonderes exzentrisches Strahlenbündel kann durch eine Kanalbohrung so indirekt die Füllung der Meßkammer reflexphotometrisch orden und den weiteren Meßablauf (nach Leerwertbestimmung vor der Punktion, Blut einwirkung, -entfernung und Messung) einleiten. Stärkere Vibrationen nach Wiederbelüftung können das Blut aus dem Napf über der Meßzone auskippen, so daß es in den Reserveraum läuft. Die Schwingungen, aber auch ein (leicht in die Form mittels senkrechter Drähte einbringbares) System von Sogkanälen(Fig.57-(269,270) kann ebenso zur Reinigung der Meßzone eingesetzt werden, wie eine absenkbare Art Watteschicht mit zentralem Fenster für die Optik. Die Kammergröße kann oben auch durch ein Dichtkissen begrenzt sein(Fig.60(298), um den unterschiedlichsten Stellungen des Gerätes zur Senkrechten Rechnung zu tragen. Der Abschluß des Gußteiles erfolgt dann über eine (sogar stanzbare) Deckelmaske. Wegen des geringeren Durchmessers des Griffels wird die Funktion der Tülle für die Aufnahme der obersten Hautkuppe von einem besonderen Hautglockenrand übernommen; während im Bereich des Blutaustrittes aus der Haut der Boden des Punktionsgriffels, gegen die Sogwirkung

formstabsilierend zur Mitte hin etwas ansteigen kann. Pusierende Druckminima un den Griffel können den seitlichen Blutabfluß verhindern( Fig.49). Die Tülle mag entbehrlich sein, wenn das Blut über einen Schaft eingesogen wird. Auch die Deckel in Verbindung mit ihren Befestigungslaschen können mit dem Griffel in einer einzigen Form gefertigt werden.

Für Injektoren müssen Reaktionskammer oder Blutspeicherraum von dem Einspritzkanal getrennt werden, auch muß die Flüssigkeitsaustrittsöffnung in der Regel unter der Haut liegen. Die Blutableitung erfolgt aber innerhalb der Haut nach Verletzung der in deren unterem Anteil liegenden Gefäßnetzes; bei Punktion nur der Hautoberschicht wird in der Regel nur blutfreie Gewebsflüssigkeit gewonnen(was genutzt werden kann). Die Absaugöffnung(3) kann seitlich im Kanülenschaft angeordnet sein oder als getrennte Kapillare direkt unterhalb des Punktionsgriffelendes des beispielsweise in einer Art Mulde des Injektionskanülenschaftes(Fig.4). Es kann aber auch eine gesonderte feinere Injektionskanüle oder -kapillare innerhalb der Schneidkante der Punktionskanüle in Verbindung mit dem Flüssigkeits-oder Blutspeicherraum vorhanden sein, welche unter die Haut gesenkt (und eventuell auch wieder zurückgezogen werden kann (Fig.45).

Eine Art Diagnostikkapillare kann auch nur vorübergehend im Injektionskanal liegen und vor der Injektion zurückgezogen werden, wenn - beispielsweise über Rotation von Griffelteilen- ein Ventilabschluß beider Kanäle für die Zeit der Einspritzung bewirkt wird(Fig.44). Auch tangential zur Haut können eine Kapillare oder ein Draht zur Blutgewinnung (eventuell auch zur Fixierung der Haut) aus einer Kanüle vorgeschoben werden-(Fig.50).

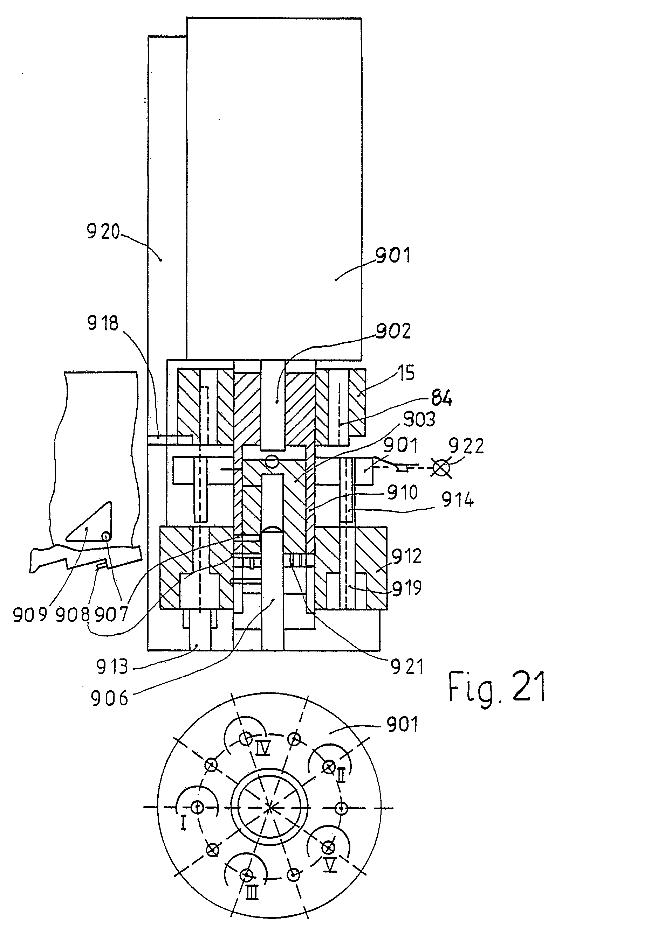

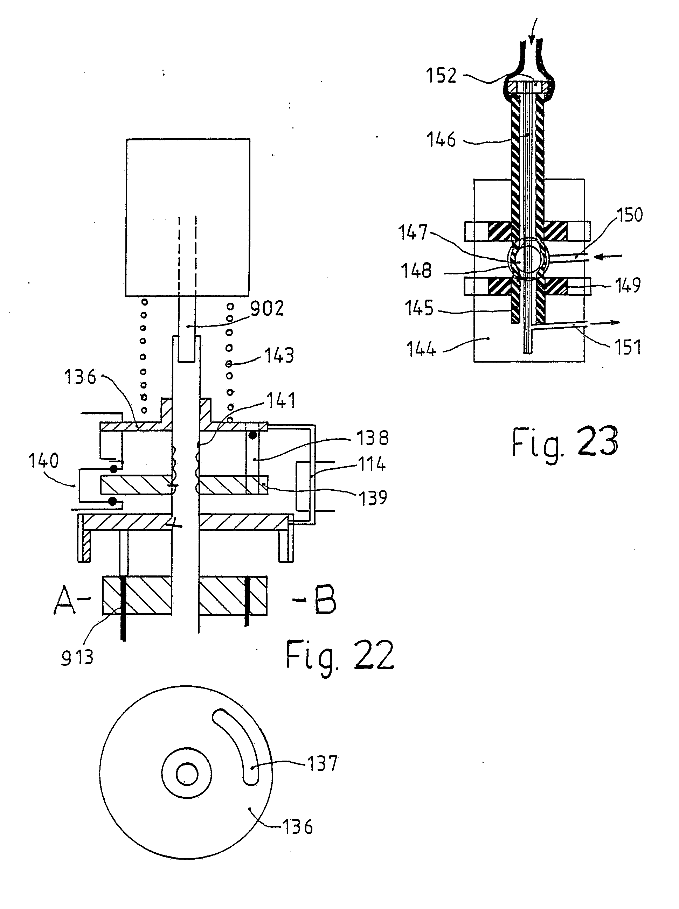

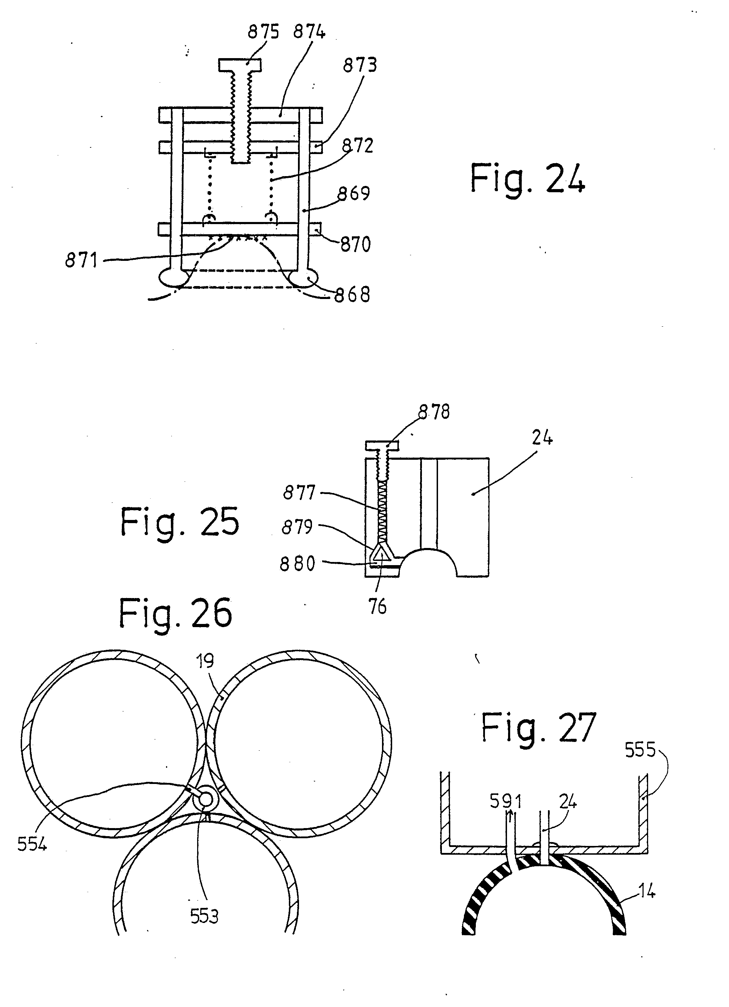

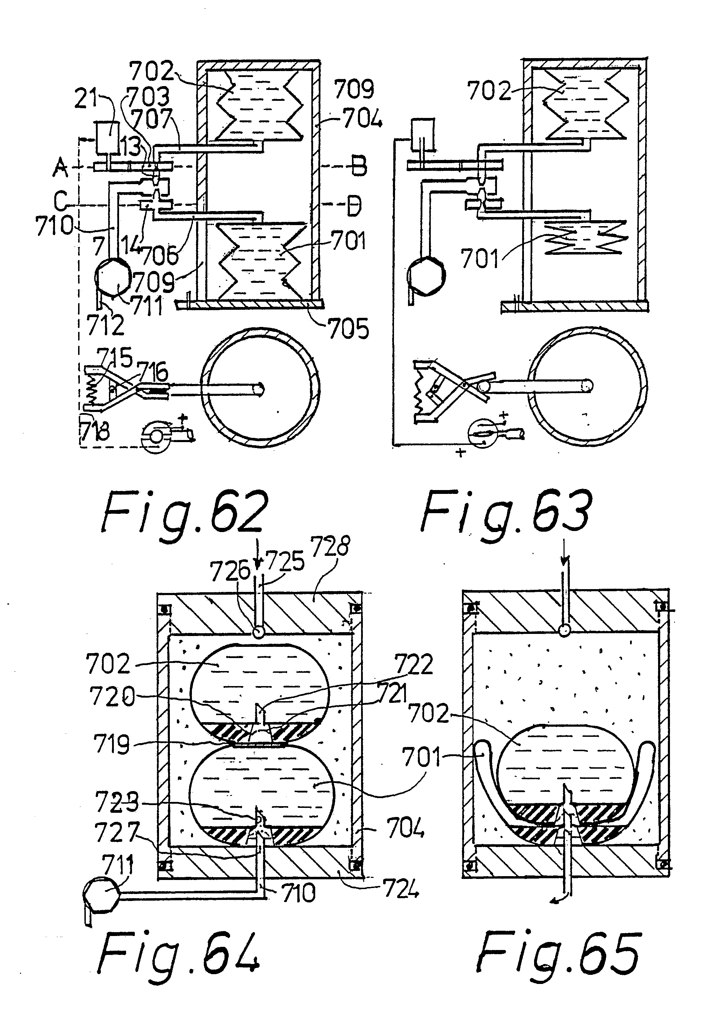

Die erfindungsgemäße Aufgabe. über Vorstufen zu einem vollautomatischen Diagnostikgerät sogar fakultativ verbunden mit einem Injektor zu gelangen, beinhaltet die Notwendigkeit vielfacher signalgesteuerter Schaltfunktionen, für welche der Gewichts- und Raumbedarf dadurch niedrig gehalten wird, daß nur ein elektrischer Antriebsmotor verwendet wird, welcher zugleich die Schalttakte, einschließlich etwaiger Dosierpumpentakte, als auch die Umschaltung auf neue Funktionsorgane bewirkt. Hierzu kann ein Magnet im Zusammenwirken mit einer Art Schaltgetriebe benutzt werden. Zur Betätigung des Schaltgetriebes wird eine verkürzte Stromzufuhr mit Wirkung eines Kurzhubes benutzt(Fig.21)oder die Magnetbetätigung in auf andere Weise bestimmten Sonderrhythmus(Fig.22), welcher die weitere Betäti gung einer bereits eingestellten Schaltfunktion ausschließt. Vorzugsweise können Schalttakte und Getriebeumstellung aber auch durch einen Rotationsmotor angetrieben werden(Fig.37)), welcher die Endfunktionen, sofern sie eine Hubwirkung voraussetzen, über eine Art Pleuelstange oder eine Hebelverdrängung durch angetriebene Keilführung bewirken, wobei die Kraftentfaltung bzw.Taktgeschwindigkeit über zwischengeschaltete Getriebeübersetzungen variiert werden können. Bei Einsatz linearer arbeitender Hubmagnete können selbstverständlich über eine Art Pleuelstangenantrieb auch Drehschalter betätigt werden. Solche Magnetschaltgetriebe könnten auch bei der Raumfahrt in Spielzeugmodellen und überall dort eingesetzt werden, wo Raum gespart werden muß. Motoren bedingen durch längere Wirkzeit geringeres Gewicht. Eine weitere wesentliche Raumersparnis wird durch die Art der Arzneibevorratung erzielt. Die Lösung in WO 86/01728, eine möglichst geringe Restmenge in Arzneibehältern bei welchselhafter Dosierung dadurch zu erzielen, daß ein zu großer Arzneirest im nahezu verbrauchten Behälter durch Rückpumpen aus einem neuen Behälter zu einer oder mehreren Volldosierungen wiederaufzufüllen, befriedigt weder ökonomisch noch im Hinblick auf die Handhabung und Sterilität. Schon bei kleineren Behältereinheiten ist Wirtschaftlichkeit gegeben, wenn diese jeweils wenigstens in doppelter Anzahl für jede Flüssigkeitssorte im Gehäuse lagern. wobei nach annähernder Entleerung des einen, der andere über einen Ventilmechanismus in Verbindung mit der Dosiervorrichtung gesetzt wird, was auch innerhalb der Zeit derselben Injektion geschehen kann. Nach entsprechender ein-oder mehrmaliger Betätigung einer Warnvorrichtung kann der verbrauchte Behälter durch einen neuen ersetzt und damit die ständige Injektionsbereitschaft bei verhältnismäßig kurzer Lagerzeit der Einzelbehälter gesichert werden(Fig.63-66). Ähnliche Vorratshandhabung kann auch im Hinblick auf die Zuschaltung von Unterdruckreserven an die Saugglocke -deren Erzeugung mittels Hitzeeinwirkung schon von altersher in der Form der Schröpfköpfe in die Medizin eingeführt wurde- oder der Zuschaltung von Druckgaspatronen zur Sogerzeugung nützlich sein (Fig.26). Erfindungsgemäß wird dem Nachteil der an und für sich handlicheren Anwendung eines begrenzten Vakuums gegenüber einem Dauervakuum dadurch ausgeglichen, daß bei über eine Meßvorrichtung erkennbar gemachtem vorzeitigem Druckanstieg im Saugglockenbereich manuell oder automatisch die Zuschaltung eines neuen"Unterdruckquantums" erfolgen kann. Neben der billigsten Möglichkeit der Betätigung einer Handpumpe wird auch ein $CO_2$-betriebenes Duplex-Pumpensystem als Beispiel beschrieben. Besonders durch Unterteilung des Saugglockenraumes in ringförmige Einzelzonen mit getrennter Kanalverbindung zu einer oder mehreren Vakuumquellen kann eine Art peristaltische(melkend nach oben verlaufende) Hautmassagewelle erzielt werden(Fig.49). Aber auch alternierend aufgepumpte Schlauchsegmente oberhalb von Klemmbakken für eine Hautfalte können ähnliches bewirken (Fig.62). Die Anwendung einer Dauersogquelle mittels einer elektromotorischen Pumpe könnte nach neuesten Versuchen wieder in Betracht gezogen werden.

Vollends läßt sich vorallem beim Diagnostikgerät eine Sogquelle ersparen, wenn die Kanülenspitze über den Hautbereich bis in die blutreichere Muskulatur solange weiter nach unten vorgeschoben wird, bis die notwendige Blutmenge in der Reaktionskammer vorhanden ist, welche auch aus dem Kanülenschaft selbst bestehen kann, wenn dieser wenigstens teilweise zur Mes sung durchsichtig gehalten ist(Fig.47), soweit nach Typ der Vorrichtung nicht eine Weiterleitung des Blutes zu einer Meßanordnung vorgesehen ist. Durch quere Sondierung der Haut mittels Draht oder Sonde(wie in Fig.57) kann die Hautkuppe festgehalten oder sogar eine solche wie durch eine Angel erzeugt werden. Auch Zentral- oder ringverschlußartige Vorrichtungen (einschließlich einer von zwei Seiten klammerartig wirkenden Klemmvorrichtung zur Erzeugung einer Hautfalte) können die angestrebte Hautkuppe erzeugen oder dazu verhelfen, daß Blut kuppenwärts ausgepreßt wird. Unschwer kann man sich mit den Kenntnissen der hier überwiegend beschriebenen Saugvorrichtungen- die Pflasterzugvorrichtung mit einer oder mehreren optischen Anlagen, die durch einen Pflasterschlitz (Fig.24,(71) hindurch wirken, und eine automatische Auslösung der Zugfedern(72) vorstellen. Auch die Kombination der geschilderten Vorrichtungen zur Hautkuppenherstellung kann eingesetzt werden(aber auch mit einem Saugnapf, der zunächst mittels Andruckes gegen die Haut evacuiert wird) Zur kanülennahen Dosierung (zur Vermeidung von Meßungenauigkeiten) wurde der Ventilmechanismus der im Schlauchende befindlichen Flüssigkeitsmeßkammer dadurch verbessert, daß die Meßspindel durch eine Kugel ersetzt wurde(Fig.23) oder eine Ventiltülle das Ende des Schlauches umkreist, während das Druckgaspolster sich innerhalb des Schlauches befindet( Fig.48).

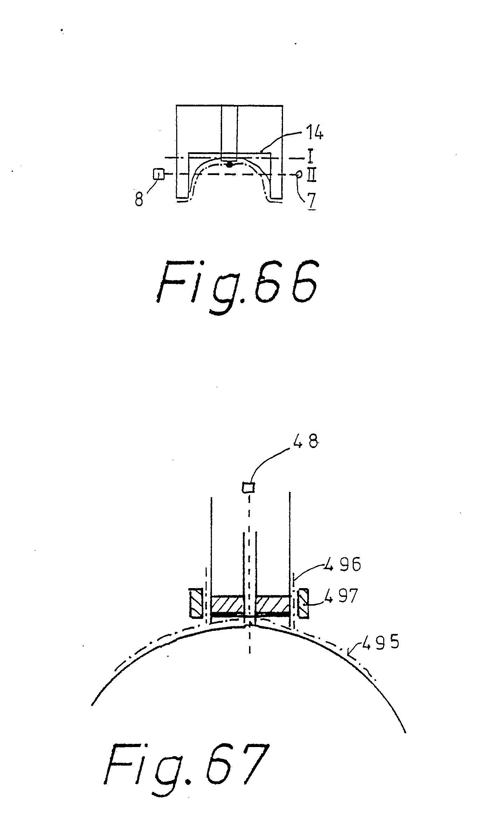

Die in der Anmeldung No 2113521/86 in Japan bereits veröffentlichten Grundeinrichtungen zur Überwachung des Hautbereiches der zu Messung oder Einspritzung bestimmt ist wurden dahingehend noch verbessert, daß die aufgeworfenen Probleme und der Rechenaufwand durch Nutzung einer formbestimmten Hautanlage im Kuppenbereich vereinfacht werden(Fig.66). Die Heranziehung eines wenigstens teilweise doppelseitig adhäsiven Pflasters zur nadellosen Überdruckinjektion ist (zunächst nur theoretisch) einleuchtender als Mittel der Hautfaltenanhebung als die Saugglocke-(Fig.67).

Wenn auch eine derartige Injektionseinrichtung außerhalb einer Vcrrichtung zur Hautfaltenbildung betrieben werden könnte (und auch ohne Stoffwechselmessung), so wäre die Abstellung der Gefahr unbeabsichtigter Pflasterablösung beim Zug am Injektor durch solche Vorrichtung doch zu bannen. Die Überdruckinjektion in eine Hautfalte bietet jedenfalls den Vorteil der Herstellung eines Abstandes zwischen gewebszereißendem Druckstrahl und tieferliegenden Gewebsschichten, wobei die Zugwirkung noch einen hautverdünnenden Effekt haben könnte.

Hinsichtlich der für eine "Sensorkanüle" oder einen Punktionsgriffel wichtigen optischen Kontrolle der Füllung der Reaktionskammer mit Blut oder Gewebsflüssigkeit bleibt nachzutragen, daß eine solche Kontrolle sich natürlich auch über einen Stromdurchlauf (oder eine Spannungsänderung) zwischen zwei elektrischen Kontakten vornehmen ließe. Hierzu müßten allerdings auch getrennte leitende Elemente oder kontakte innerhalb der Reaktionskammer oder deren Wandung vorgesehen sein, was die Kontrolle eher komplizieren würde. Das Vorhandensein einer Vorrichtung zur Reflex-Photometrie ist über eine senkrechte Bohrung (beispielsweise Fig.59) aber auch über Lichtleitfasern im Schiebestift möglich, ja die Strahlen können durch den Griffel oder an disem vorbei auch direkt gegen die Punktionsstelle auf der Haut gerichtet und von dort wieder zum Detektor zurückgeworfen werden; die Eignung der Haut (weil frei von ungewöhnlichen Reflexen durch Unebenheiten oder Pigmentveränderungen) zur Punktion ließe sich so überprüfen, aber auch der Höhenstand der Hautfalte oder Hautkuppe überhaupt. Eine solche funktionelle Konzentration kommt dem Erfindungsgedanken entgegen, wie er in der nachfolgenden Beschreibung der Zeichnungen in Einzelheiten erläutert wird.

### Kurze Beschreibung der Ausführungsbeispiele:

Fig.1 zeigt im Querschnitt im Maßstab 2 : 1 eine Sensorkanüle, deren Spitze zusammen mit der Diagnostikkapillare abgesenkt wird und erst nach Wiederanhebung der vorher durch die Kapillare verlegten seitlichen Einspritzkanal freigibt; I stellt das Stadium zur Blutaufnahme, II das Stadium zur Injektion dar; unter I ein Querschnitt in Höhe der Reaktionszone.

Fig.2 zeigt im Längsschnitt im Maßstab 2 : 1 eine andere Kanülenvariante, deren Spitze im Stadium I zur Blutansaugung in die Haut eingeführt und im Stadium II unter die Haut zur Injektion gesenkt wird.

Fig.3 zeigt eine Teilansicht einer Sensorkanüle im Längsschnitt Maßstab 4 : 1 mit seitlicher Öffnung im Kanülenschaft zur Aufnahme von Blut aus der Haut.

Fig.4 bringt oben im Längschnitt und darunter im Querschnitt im Maßstab 4 : 1 eine Sensorkanüle nach Art derjenigen in Fig.3, welche eine besondere Reaktionskammer in Verbindung mit einem Saugglockenraum aufweist.

Fig.5 zeigt im Längsschnitt im Maßstab 2 : 1 eine Sensorkanüle innerhalb einer Saugglocke mit Speichervakuum für den diagnostischen Einmalgebrauch.

Fig.6 zeigt im Umriß und im Saugglockenbereich im Längsschnitt in natürlicher Größe eine Diagnostikeinrichtung für den Gebrauch von Sensorkanülen. Unten ist im Querschnitt ein Teil eines kettenförmigen Kanülenmagazins abgebildet.

Fig.7 zeigt links im Längsschnitt rechts in einer Seitenansicht die Hülse eines Kanülenmagazins nach Fig.6, links mit Kanüle und Schaltstift darüber.

Fig.8 zeigt in Seitansicht den unteren Teil einer Sensorkanüle mit Sensorbeschichtung an der Kanülenbasis.

Fig.9 zeigt in einem Querschnitt längs der Schnittlinie A - B der Fig.10 eine Einrichtung zur Saugdiagnostik bei Sogerzeugung mittels Gasstrahlpumpe.

Fig.10 zeigt einen Querschnitt durch die Einrichtung zur Saugdiagnostik nach Fig.9.

Fig.11 zeigt im Längsschnitt im Maßstab 2 : 1 teilweise den Mechanismus für die Übersetzung von Magnethüben in Schalthübe verschiedener Höhe für eine Einrichtung nach Fig.9 und 10.

Fig.12 zeigt im Maßstab 2 : 1 oben im Längsschnitt unten im Querschnitt in Höhe der Meßzone einen Punktionsgriffel für die Verwendung innerhalb einer Einrichtung nach Fig.9 und 10.

Fig.13 zeigt eine mechanische Verriegelung der Saugglocke gegen federbewirkte Absenkung in eine funktionsbereite Stellung im Querschnitt in natürlicher Größe.

Fig.14 zeigt oben im Längsschnitt im Maßstab 2 : 1 einen Punktionsgriffel innerhalb einer Saugglocke mit Vorrichtung zur Hautannäherung zur Punktion. Unten im Querschnitt entlang der Schnittlinie A - B des Längsschnittes im Maßstab 4 : 1 zu sehen.

Fig.15 gibt schematisiert den Federmechanismus für die Höhenbewegungen der Saugglocke einer Einrichtung nach Fig.9 und 10 im Längsschnitt in natürlicher Größe wieder. Rechts wird im Querschnitt die Verteilung der Federn für die Saugglockenverschiebung gezeigt.

Fig.16 zeigt in seitlicher Draufsicht den Federspannmechanismus insbesondere für die Betätigung des Gasaustrittsventiles einer Einrichtung nach Fig.9 und 10; links wird ein beim Schlußanschlag des federgetriebenen Schiebers ausgelöste senkrechte Schaltbewegung demonstriert.

Fig.17 gibt ein von einem Elektromotor betriebenes Schaltgetriebe wieder, welches die verschiedenen Schaltfunktionen bedient; das Getriebe ist im Längsschnitt wiedergegeben.

Fig.18 beschreibt im Längsschnitt eine Vorrichtung, um den Arbeitshub (beispielsweise für die Dosierung mittels einer Vorrichtung nach Fig.23) ruckartig zu gestalten.

Fig.19 zeigt eine Vorrichtung wie in Fig.18, wobei jedoch beide Taktrichtungen ruckartig gestaltet werden (wie es für die Betätigung einer Dosiervorrichtung nach Fig.23 zweckmäßig ist).

Fig.20 zeigt im Längsschnitt eine Schaltvorrichtung entsprechend der Fig.17 mit einer Lösung für die Anpassung der Höhe der Schalthübe an die Funktionsnotwendigkeiten.

Fig.21 zeigt das Schaltgetriebe eines Hubmagneten im Längsschnitt, um eine reduzierte Hubbewegung zur Umschaltung zu benutzen.Unten ist ein Querschnitt in Höhe der Bowdenzuranschlüsse zu erkennen.

Fig.22 zeigt ein Magnetschaltgetriebe, welches bestimmte Taktfolgen der Magneterregung zur Umschaltung ausnutzt. Unten wird eine Draufsicht auf die Schwungscheibe gezeigt.

Fig.23 zeigt im Maßstab 2 : 1 im Schlauchende die kanülennahe gelagerte Dosiervorrichtung im Längsschnitt.

Fig.24 zeigt im Längsschnitt eine Vorrichtung zur Hautanhebung mittels veränderlicher Federspannung.

Fig.25 zeigt im Maßstab 1 : 2 eine Saugglocke mit einem regelbaren Ventil für die Sogstärke im Längsschnitt.

Fig.26 zeigt im Querschnitt in natürlicher Größe drei Vakuumspeicherräume, welche nacheinander über ein Ventil betätigt werden können.

Fig.27 zeigt eine gummielastische Saugglocke zur Betätigung als Sogquelle unter Andruck gegen die Haut in natürlicher Größe im Längsschnitt. Unten wird ein Querschnitt in Höhe der Meßzone(5) gezeigt.

Fig.28 zeigt im Längsschnitt im Maßstab 6 : 1 einen Punktionsgriffel für die Reflexfotometrie; I bringt das Stadium vor, II dasjenige nach bei Hautansaugung und während des Abwischvorganges der Meßzone.

Fig.29 zeigt einen Punktionsgriffel wie in Fig.28: I während der Hautanhebung mittels Soges II während der Blutansaugung
III bei der Wiederbelüftung und Blutabsaugung.

Fig.30 zeigt im Längsschnitt im Maßstab 4 : 1 einen Punktionsgriffel, welcher nach Blutaufnahme auch zur Einspritzung geeignet ist.

Fig.31 zeigt im Maßstab 2 : 1 eine Variante zu Fig.30 sowohl im Ventilmechanismus als in der Art der Annäherung der Kanüle an die Haut. Auch in der schematischen Teilansicht der Fig.32 ist die Hautannäherung Gegenstand der Darstellung: I Position zur Blutansaugung, II zur Einspritzung.

Fig.33 zeigt in natürlicher Größe im Längsschnitt eine Saugglocke mit Anordnung einer Lichtschranke im Kuppenbereich eines Gitters für die Aufnahme der Hautkuppe.

Fig.34 zeigt die Hautfestlegung innerhalb eines Gitters bei einer Kanüle ähnlich wie in den Figuren 31 und 32 im Längsschnitt in natürlicher Größe.

Fig.35 gibt die Draufsicht auf eine Einrichtung zur Saugdiagnostik bei einer Gehäusegestaltung ähnlich wie in Fig.9.

A gibt eine Draufsicht mit Aufbruch in der linken unteren Ecke.

B zeigt einen Querschnitt dieser Einrichtung,größtenteils(und hinten) entsprechend der Schnittlinie D - E des Längsschnittes C, vorn entsprechend der Schnittlinie F - G auf dem Längsschnitt C.

C , der Längsschnitt, zeigt einen linksseitigen Aufbruch längs der Schnittlinie H - J des Querschnittes B; rechtsseitig und hauptsächlich folgt die Darstellung der Schnittlinie K - L des Längsschnittes B. Der Anschnitt der Deckelwippe in C folgt in ganzer Ausdehnung der Schnittlinie H - J. diese Deckelwippe dient der Vakuumerzeugung.

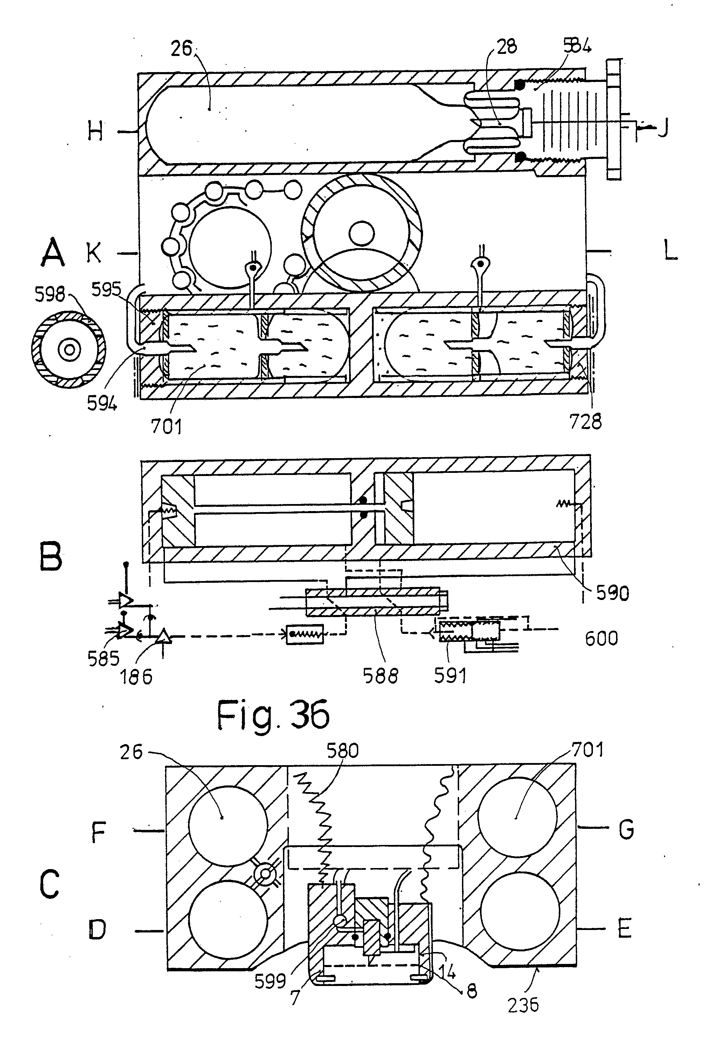

Fig.36 zeigt eine Einrichtung ähnlich derjenigen der Fig.35, welche jedoch auch zur Injektion geeignet ist. Die Vakuumerzeugung erfolgt mittels einer Kolben-Zylinder-Pumpe, die mittels Druckgases angetrieben wird.

A gibt einen Querschnitt in Höhe der Schnittlinie D - E des Transversalschnittes C wieder mit Druckgaspatrone, vereinfachter Saugglocke und Flüssigkeitsbehälter(Links im Detail im Sagitalschnitt erkennt man das Ineinandergreifen der Fortsätze der Kappen zweier Flüssigkeitsbehälter),

B zeigt einen Teilquerschnitt in Höhe der Schnittlinie F - G des Sagitalschnittes C mit der Vakuumpumpe; der Anschaulichkeit wegen wurde der zugehörige Ventilmechanismus nach vorn herausgedreht dargestellt.

C gibt einen Sagitalschnitt längs der Schnittlinie H - J des Querschnittes A.

Fig.37 zeigt im Längsschnitt im Maßstab 2 : 1 ein Schaltgetriebe, wie es in einer Einrichtung nach den Figuren 9- 11, 35 und 36 verwendet werden kann. Die Einzelheiten des Elektromotors wurden weggelassen.

Links oben wird in natürlicher Größe ein zugehöriger Schalter zur Betätigung des Kanülentransportes gezeigt, darunter eine Art Pleuelstangenschub unter Steuerung mittels einer Kurvenscheibe.

Figur 38 zeigt in teilweisem Querschnitt im Maßstab 3 : 1 eine doppelt angeschliffene Kanüle, welche zwar in die Haut eindringen, diese aber nicht durchdringen kann.

Fig.39 zeigt im Längsschnitt in etwa natürlicher Größe eine Saugglocke mit Sensorkanüle und deren Rotationsmechanismus.

Fig.40 zeigt im Längsschnitt etwa in natürlicher Größe eine Saugglocke mit pneumatischem Randwulst: links in schlaffem Zustand desselben vor , rechts bei Prallfüllung desselben nach der Hautanhebung.

Fig.41 zeigt im Längsschnitt im Maßstab 4 : 1 einen Punktionsgriffel mit Spitzenperforation für die Blutabsaugung nach oder während seiner Rotation (Unten die Spitze in Draufsicht um 90° gedreht).

Fig.42 zeigt in etwa natürlicher Größe eine besondere Randgestaltung einer Saugglocke und einen Mechanismus, um den die Spitze tragenden Teil des Punktionsgriffels der Haut entgegen herabzuschrauben.

Fig.43 zeigt in ähnlicher Darstellung wie in Fig.42 Mechanismen zur Senkung des Punktionsgriffels zugleich mit einer Lichtschranke; links unter Zugfederaufhängung des Saugglockendaches, rechts unter Herabschraubung desselben.

Fig.44 zeigt oben im Längsschnitt und unten im Querschnitt im Maßstab 4 : 1 eine zusammengesetzte Sensorkanüle mit der Möglichkeit der Rotation des unteren Teiles unter Ventilbetätigung vor der Einspritzung. Die Diagnostikkapillare liegt teilweise außerhalb des Kanülenschaftes. Der Querschnitt liegt in Höhe des Meßstrahles.

Fig.45 gibt eine ähnliche Kanüle wie diejenige der Fig.44 ähnlich wieder. Die Injektionskapillare liegt aber gänzlich innerhalb des Punktionsschaftes:

I in diesen zurückgezogen
II zur Einspritzung gesenkt.

Fig.46 zeigt wiederum im Längsschnitt im Maßstab von etwa 2 : 1 eine Kanüle, deren unterer Anteil während festem Dichtungssitz seines oberen inner halb der Saugglocke durch die Haut hinabgeschraubt werden kann.

Fig.47 zeigt im Längsschnitt etwa im Maßstab 4 : 1 eine Kanüle, welche auch ohne Anwendung von Zug oder Sog auf die Haut bohrerartig in diese zur Blutabsaugung gesenkt werden kann.

Fig.48 zeigt im Längsschnitt im Maßstab 2 : 1 eine Dosiervorrichtung ähnlich derjenigen der Fig.23 und eine Sensorkanülenvariante.

Fig.49 gibt etwa im Maßstab 2 : 1 im Längsschnitt eine Saugglocke mit mehreren Anlagekreisen für die Hautkuppenoberfläche wieder, welche über gesonderte Kanäle mit der Sogquelle verbunden sind; die Kanüle ist von einer Mulde umgeben, in die neben dem Kanülenschaft austretendes Blut eingesaugt werden kann. Die Meßzone kann sich in dieser Mulde oder einer an diese anschließende Reaktionskammer befinden.

Fig.50 zeigt im teilweisen Längsschnitt im Maßstab 4 : 1 den unteren Anteil eines Kanülenschaftes, durch dessen Loch gegenüber dem Anschliff ein Draht(oder eine Kapillare) quer zur Haut vorgeschoben ist.

Fig.51 gibt im Längsschnitt im Maßstab 2 : 1 den unteren Teil einer Kanüle mit anschließendem Kanal als Reaktionskammer wieder, wobei im Stadium I der Kanülenschaft durch ein hochelastisches Schläuchchen abgeschlossen wird, während im Stadium II durch Flüssigkeitsdruck das Schläuchchen gereckt und an seiner besonders dünnwandigen Spitze zur Injektion aufgeplatzt ist.

Fig.52 zeigt oben im Längsschnitt und unten im Querschnitt in Höhe der Schnittlinie A - B des Längssschnittes eine Kanüle mit absenkbarem Schaft, wobei der Raum zwischen Kanülenkörper und Schaft über einen Faltenbalg gedichtet ist, der auch als Luftpumpe eingesetzt werden kann. Die Meßzone innerhalb einer Trichterbohrung neben dem Schaft wird über eine Art Schwamm mit Blut in Kontakt gebracht. Benachbarte Kanülen sind über in Nuten verschiebliche Gelenkfortsätze verbunden.

Fig.53 bringt in natürlicher Größe oben im Längs-und unten im Querschnitt eine Sensorkanüle mit freiem Zugang zur Meßzone von oben und exzentrischer Lage der Schneidkante für die Blutgewinnung gesondert von einem Kanülenschaft; als weitere Neuheit wird ein exzentrisch gelagerter abklappbarer Deckel als Sterilitätsschutz der Kanülenbohrungen demonstriert.

Fig.54 zeigt in schematischer Blockdarstellung im Maßstab etwa 1 : 6 die Zusammenstellung des Diagnostik- und Injektionssystems verteilt auf drei Gehäuse.

Figur 55 bringt im Längsschnitt in natürlicher Größe eine (durch einen zweiten Sogkanal) zweistufige Saugglocke mit einem Punktionsgriffel im Innern, welcher eine Lanzette umschließt, die zur Punktion kurzzeitig in die Haut gesenkt wird. Das Blut kann entweder am Schlitz für die Lanzette vorbei auf eine nach oben gerichtete Meßzone aufgebracht werden oder erreicht diese Meßzone von unten.

Figur 56 stellt eine Rotationskanüle dar, deren Trägerhülse mit einer Lanzettspitze innerhalb eines in der Dichtung zur Saugglocke feststehenden Aufnahmebechers gedreht werden kann bei zentraler Lage des Sackloches für die Messung von oben. Unten sind im Querschnitt A - B durch den Längsschnitt die Bluteın- und -austrittskanäle in die Trägerhülse zu erkennen.

Figur 57 zeigt oben in einer Draufsicht, unten im Längsschnitt eine Variante des Kanülenwechselmagazins.

Figur 58 zeigt im Längsschnitt einen Punktionsgri fel mit Lanzettspitze dessen äußerer Aufnahmebecher gegenüber der feststehenden Trägerhülse in seitliche Schwingungen versetzt werden kann, um den Stichkanal bei Bedarf zu erweitern.

Figur 59 zeigt links im Längsschnitt eine Saugglocke mit einer anderen "Vibrationskanüle", wobei der Aufnahmebecher feststeht und die Trägerhülse in Schwingungen versetzt wird bei exzentrischer Lage der Lanzettspitze und des Schaftes der Injektionskanüle.
Rechts ist eine Draufsicht auf die Deckelpartie A - B wiedergegeben.
Die Balancierfedern sind links im Stadium noch der Saugglockenanhebung, rechts bei Hochstand derselben gezeichnet.

Fig.60 zeigt in einer Draufsicht die Steuervorrichttung zur Eröffnung eines Sitzventiles für eine Gaspumpenregulierung etwa in natürlicher Größe. Unten rechts wurde ein Keilschieber um 90 Winkelgrade herausgekippt dargestellt.

Fig.61 zeigt schematisiert oben im Längsschnitt und unten im Querschnitt eine Vorrichtung zur Quetschung einer Hautkuppe. I zeigt das Stadium bei geöffnetem Blendenraster, II das Stadium bei geschlossenem Ring-"Blenden"raster.

Fig.62 gibt in natürlicher Größe schematisch zwei Flüssigkeitsbehälter wieder, welche über eine Dosierpumpe nacheinander entleert werden. Die Flüssigkeitsbehälter werden gefüllt dargestellt; unten zeigt der Querschnitt in Höhe der Schnittlinie A - B des Längsschnittes wie der Durchfluß vom oberen Behälter zur Dosierpumpe gesperrt ist, während er vom unteren Behälter frei ist.

Fig.63 gibt die Vorrichtung nach Fig.62 wieder, nachdem der untere Behälter sich entleert wurde; der obere Zufluß ist jetzt freigegeben, der untere gesperrt, so daß der untere Behälter ersetzt werden kann.

Figur 64 und 65 geben eine Variante der Vorrichtung nach den Figuren 62 und 63 wieder, welche keiner Ventile bedarf.

Ausführliche Beschreibung der Ausführungsbeispiele:

Figur 1 zeigt im Längsschnitt in der Vergrößerung (Maßstab 2 : 1) eine Sensorkanüle bestehend aus Kanülenkörper(1) mit in eine zentrale Bohrung verschieblich bis zu einem Anschlag eingefügtem Schaft (2); oberhalb desselben befindet sich in dieser zentralen Bohrung des Kanülenkörpers mit dem Schaftende fest verbunden ein Docht als eine Art kapilläre Leitelemente(3) und eine Meßzone(5) für die Reflexfotometrie, wobei das freie aus dem Kanülenkörper herausragende Ende des Dochtes an eine .Querplatte(4) befestigt ist, welche zur Docht- und Kanülenverschiebung dient. Stadium A stellt den Zustand der Kanüle vor und während des Einstiches in die Haut dar, die vom Kanülenschaft durchdrungene Haut ist durch eine Wellenlinie deutlich gemacht. Der horizontale Pfeil von rechts gibt die Eintrittsstelle von Arznei oder Spülflüssigkeit(6) für die Abspülung von Blutbestandteilen vor der Messung an. Letzteres erfolgt im Stadium B nachdem die Querplatte(4) mit Docht und Kanüle gesenkt und dadurch der Docht mit der hyperämischen Haut in Berührung gebracht wurde. In diesem Falle wurde zur Demonstration ihrer Beweglichkeit die Meßzone innerhalb des Hautverletzungsringes liegend dargestellt. Zur Messung wird an der Querplatte der Docht wieder zurückgezogen, so daß das unter A dargestellte Stadium wiedererreicht wird. Man erkennt den Strahlenweg des Lichtes, während Lichtquelle(7) und -empfänger(8) rechts im Stadium B eingezeichnet sind. Das freie untere Ende des Dochtes kann eine besondere gegen Flüssigkeitsdurchtritt dichtende Abschlußschicht aufweisen. Die Injektion erfolgt bei Bedarf über die Eintrittsstelle(6).

Unter dem Längsschnitt ist ein Quersschnitt durch den Kanülenkörper im Bereich der Reagentien tragenden Meßzone(5) angegeben, welcher garantiert, daß die Meßzone sich in bestimmter Winkellage zur Photometeranordnung befindet.

Figur 2 zeigt -wiederum im Längsschnitt und im Maßstab 2 : 1 - eine andere Lösung einer aus Fig.48,49 aus WO 86/01728 abgewandelten Kanüle mit seitlicher Eintrittsstelle(6) für die Arznei. Der Kanülenschaft ist im Kanülenkörper festmontiert, und die kapillären Leitelemente(3) reichen vorn im Stadium A bis zur Kanülenspitze, deren seitliche schräge Öffnungsfläche innerhalb der Haut festgehalten werden muß für die Aufnahme von Blut in die kapillären Leitelemente. Im Stadium B wird der Docht als kapillären Leitlelemente zurückgezogen, wobei dessen blutgetränktes Ende über der Meßzone(5) sich abstreift. Es kann nach bestimmter Einwirkungszeit der Blutbestandteile auf die Meßzone von oben über die den Docht eine Freispülung erfolgen, oder aber der Docht kann nach unten geschoben und spitzenwärts aus der Kanüle herausgezogen werden, wobei das nicht blutgetränkte Ende die Meßzone abstreift und säubert. Zur etwaigen Injektion muß der obere Teil der zentralen Bohrung des Kanülenkörpers natürlich verschlossen werden.

Figur 3 zeigt in teilweisem Längsschnitt etwa im Maßstab 3 : 1 einen Kanülenkörper(1) mit Schaft(2), welcher an seiner Basis eine Schlitzung-(9) aufweist. Im Inneren der Kanüle kann sich hinter dem Schlitz ein Docht befinden, auch das Kanülenlumen selbst kann bei entsprechender Enge als Kapillare saugend zur Blutaufnahme aus dem Hautverletzungsring wirken.

Figur 4 zeigt wiederum in einem partiellen Längsschnitt (oben) eine Kanülenkörper(1). In einer Einbuchtung an der Basis des Kanülenschaftes(2) liegen Kapillaren als Leitelemente(3), welche sich in den Hautverletzungsring öffnen. Von dort steigt Blut über ein nicht dargestelltes Filter für Blutkörper chen in einen Sammelraum(11), dessen Wand mit Chemikalien beschichtet ist(z.B.NADH und Glucoseoxydase). Die vollständige Sammelraumfüllung wird begünstigt durch die Unterdruckwirkung über den Druckausgleichskanal(12), wobei Flüssigkeit in dem Sammelraum durch das nur für Gas durchlässige Filter(10) zurückgehalten wird. Da die diaphanische optische Durchleuchtung des Sammelraumes zur Glukosebestimmung mittels U.V.-Lichtes(7,8) in beiden Richtungen erfolgen kann, wurde ein ovalärer Kanülenquerschnitt gewählt (unten).

Die Figur 5 gibt einen Längsschnitt im Maßstabe 2:1 durch ein Unterdruckspeichertöpfchen an, wie es im Europatent No 0103664(Fig.3,4) beschrieben ist. Der Kanülenkörper(1) mag eingesteckt oder fest eingebaut sein. Die Außenkammer-(13) für die Unterdruckbevorratung wird zentral von einer Röhre durchzogen, welche einen Docht als kapilläre Leitelemente(3) umschließt, der bis zur (nicht dargestellten) Fensterung(gemäß Fig.3) reicht. Ein Druckausgleichskanal (12) ist auch hier vorgesehen. Bei Andruck der elastischen Zunge am Bodenende nimmt der Auslösestift (16), welcher innerhalb des Ventilrohres(16) gelagert ist, Spannung auf bis zum Druchbruch seines oberen Endes durch die Verschlußmembran des Ventilrohres. Während des jetzt erfolgenden Druckausgleiches zwischen Vakuumspeicher und Saugglocke-(14) wird die Haut in letztere angehoben und von dem Kanülenschaft durchdrungen. Der Docht innerhalb des Kanülenschaftes nimmt in der hyperämischen Phase Blut auf und kann nach Wiederbelüftung durch Abzug des Membrandeckels (17) mit diesem entfernt werden. Das gewonnene Blut kann bei dieser Anordnung innerhalb oder außerhalb des unterdruckbewahrenden Speichertöpfchens auf eine Meßzone gebracht oder in einem Reagenzglas (bei Standardisierung der Kontaktzeit) eluiert werden. Ein komplettes Glukose-Meßgerät auf Saugbasis wird oben im Längsschnitt und unten im Querschnitt auf der Höhe der (unvollständig wiedergegebenen) Kette der Kanülenhülsen(30) in den Grundzügen wiedergegeben. Das Gerät leitet sich von dem Injektorbeispiel Fig.82,83 aus WO 86/01728 ab, wo die Bauteile und deren Funktion näher erläutert sind. Durch Wegfall von Insulinbehälter und Dosiervorrichtung gestaltet sich das Gerät, dessen Größe in erster Linie durch das Reflexphotometer(18) bestimmt wird, handlicher. Der Saugglocke(14) für den Dauergebrauch ist ein Heißluftkessel(19) angelagert bei Verbindung desselben zur Saugglocke über das Ventil(20). Die Aufheizung erfolgt über Heizdrähte im Kesselinneren über das Lichtnetz; nach Abkühlung bleibt der Unterdruck bis zur Geräteanwendung gespeichert.

Abgebildet ist das Gerät während der Hautansaugung nach Senkung des Reflexfotometers aus der höheren (streich-punktiert gezeichneten) Position. Das Absenken des Reflexphotometers gegen die Druckfeder((28), welche dem Vortrieb des Rohres mit dem Wiederbelüftungskanal-zugleich Schiebestift(24) für die Kanülen-dient, ist erst möglich, wenn über gleichmäßige Druckbetätigung der drei Auslösestifte(15) um die Saugglocke der Schaltring(16) gedreht wurde (dieser Mechanismus ist zu Fig.84 in WO86/01728 näher beschrieben). Die Druckfeder(28) bewirkt erst dann die Absenkung des Kanülenschaftes in die Haut, wenn der Raster(25) durch den Elektromagneten (21), welcher über den Kondensator(22) gespeist wird, betätigt wurde, dies geschieht über den Magnetstößel (27) über einen nicht dargestellten Bowdenzug. Letzterer führt einen Konusstift in die Bohrung der Lamelle des in die Achseneinkerbung des Schiebestiftes (24) einrastenden Sperrlamelle, welche die Bewegung des Schiebestiftes mit Wiederbelüftungskanal freigibt. Der Elektromagnet gibt seinen Hub mittelbar über einen Schaltstift weiter dessen Querstift nach jedem Hub eine Winkeldrehung um die Achse durchführt(ein Mechanismus ähnlich wie in den Aufrollungen der Fig.26 und 84 I in WO86/01728 darge stellt), so daß auch die Öffnung des Wiederbelüftungsventiles(23) über denselben Hubmagneten(21) bewirkt werden kann, wobei zugleich der Sperraster für die Rückwärtsbewegung des Reflexphotometers von der Saugglocke weg unter Rückzug der Kanüle aus der Haut ausgelöst wird. Mit der Öffnung des Wiederbelüftungsventiles(23) tritt ein Luftstrom über die Meßzone im Kanülenkörper und reinigt diese von Blutbestandteilen. Die reflexphotometrische Messung erfolgt über das Lichtleitkabel(429), welches mit dem Reflexphotometer(18) verbunden ist(nicht dargestellt). Der Mechanismus des Kanülenwechsels durch Horizontalverschiebung der Kette der Kanülenhülsen(30,Fig.7), welche zweckmäßigerweise in sich geschlossen ist, ist nicht dargestellt. Mittels des Schiebstiftes(24) kann die Kanüle nach Gebrauch wieder in die Kanülenhülse-(430) zurückgezogen und dort abgestreift werden. Nach Weitertransport der Kette kommt eine unverbrauchte Kanüle über die die Zentralbohrung in der Saugglocke zu liegen und verschließt diese Zentralbohrung nach Absenkung hermetisch. Die Zweckmäßigkeit der Neuerung im Verfahren des Kanülenwechsels ergibt sich- neben Zwängen der Gerätegestaltung- auch aus der Möglichkeit, die Sensorkanüle mit den erfindungsmäßig neuen Eigenschaften auch am schaftabgewandten Ende für Manipulationen zugänglich zu halten (wo bei der "Stapelkanüle" aus WO86/01728 der Schaft der nachfolgenden Kanüle gelagert ist).

Figur 7 zeigt im Maßstab 2 : 1 zwei der in Kette angeordneten Kanülen, die linke davon im Längsschnitt. Der Kanülenkörper mit Schaft und hier schräg verlaufendem Druckausgleichskanal(12) liegt innerhalb der Kanülenhülse(430), welche oben von einer dehnelastischen Membran(431) und unten von einem Haftdeckel(432) steril verschlossen wird. Diese sterile Lagerung bis unmittelbar vor dem Gebrauch ist ein wesentlicher Vorteil der neuen Ausgestal tung der Erfindung, ohne welche eine derartige Sensorkanüle nicht allgemein eingesetzt werden kann. Die Vertikalbewegung der Kanüle mittels des Schiebestiftes(24) und dessen Wiederbelüftungskanal im Zentrum in Schlauchverbindung mit dem Wiederbelüftungsventil(23) wurden zu Fig.6 bereits beschrieben.

Figur 8 zeigt in einem teilweisen Längsschnitt im Maßstab 3 : 1 einen Trägerkörper mit Sensorborste bzw.(wenn man sich die Borste durchbohrt vorstellt) einen Kanülenkörper mit Schaft, wobei Borste oder Schaft von einem Mantelring(34) umgeben sind, welcher die Eigenschaften eines passiven elektrischen Sensors aufweist, wie er schon in WO86/01728 beschrieben wurde.

Figur 9 zeigt einen deckelnahen Querschnitt (A-B in Fig 10) eines Beispieles einer Vorrichtung zur Saugdiagnostik in natürlicher Größe. Der Gehäusekasten (555) gliedert sich in drei in sich starre Teilräume, welche gegen einander bei entsprechender Elastizität von Gehäuseboden und - deckel längs der (strichpunktierten) Faltlinien a-b und c-d parallel ihrer Längsachse etwas umgebogen werden können. Der Gehäusedeckel(424) ist um seine Scharniere(425) hochgeschlagen. Im oberen Teilraum ist die Druckgasflasche(26) erkennbar, an deren Ende die Quellplatte(27) aus Agar-Agar, welche sich unter Einfluß zugegebenen Wassers ausgedehnt und den Öffnungsdorn(28) durch die Weicheisenplatte der Druckgasflasche gedrückt hat. Das ausgetretene Druckgas, Kohiensäure, befindet sich im Gasstauraum(29); das am Bimetallbügel(31) befestigte kleine Sitzventil(30) dichtet von innen den kleinen Kanal zur Rückstaukammer(32) ab. Das große Sitzventil(33) wird durch Vorschub des eingeschliffenen Stößels-(34), welcher von der Keilschrägen(36) des Schiebers(37) einwärts verschoben wird, geöffnet. so daß die Mulde (35) des Stössels den Gasaustritt zum Gasaustrittskanal(38) zur Gasstrahlpumpe(39) freigibt

Im mittleren Teilraum ist gestrichelt der Saugglockenrand(40) dargestellt, gegen den die drei Rückstellfedern(41) anlehnen. Vom Saugglockenrand aus erstreckt sich der Führungsstift(42) in das Zick-Zack-Profil der Kulisse der Kanülentransportscheibe(44), von diesem wird das "Kanülen"-Körper-Transportband bewegt und wird vor dem sechskantigen Mitnehmerrad(46) mit der stellungsstabilisierenden Plattfeder(47) vor der Lichtquelle mit Detektor(48) vorbeibewegt, um über das linksseitige Transportrad im Kreis zurückgeführt zu werden. Die "Kanülen"-körper, hier eigentlich Träger von einer Borste oder einem Dorn mit anschliessender "Textil"-Zone und/oder Draht oder Plastikfaden, sind über Fäden strickleiterartig miteinander verbunden, welche durch Bohrungen-(49,Fig.4) des Körpers hindurchgeleitet werden. Die Magnetschaltvorrichtung besteht aus einem Elektromotor(51) und fünf Teleskop-Rohrsegment-Schieber, welche nacheinander durch die Keilschräge der Schaltnocke(53) an der Schaltwelle betätigt werden. Von den Bowdenzügen zu den Funktionsschaltern ist nur derjenige zum Schieber-(37) für die Gasventilöffnung zur Gasstrahlpumpe dargestellt. Die Batterie(54) für die Stromversorgung steht links neben dem Raum für die elektronische Steuerung, welche -ebenso wie die Verkabelung- nicht dargestellt wurde.

Die Figur 10 gibt einen Längsschnitt durch die Vorrichtung nach Fig.9 (in der Schnittlinie C-D dort) wieder. Das obere Ende des "Schaftes" der Kanüle oder des Punktionsgriffels weist einen Teller(50) als Verbreiterung auf. Die Kappe(55) für die Aufnahme desselben ist mittels des Bowdenkabels(56) hochgezogen, dessen Biegung und Fortsetzung innerhalb einer schrägliegenden Deckelausbuchtung liegt bei Endigung mit vier Zügeln an jeweils zwei entgegengesetzten Enden der Teleskop-Rohrsegment-Stutzen(Fig.11), welche ihrer seits mit den vier zugeordneten Teleskop-Rohrsegmentschiebern(Fig.9,52), über welchen sie in der Deckelausbuchtungs(57) liegen, verbunden sind. Die Gasaustrittsöffnung(58) für die Gasstrahlpumpe(Fig.9,39) liegt dicht an der Kante der Deckelausbuchtung, damit sie nicht von der bedienenden Hand verschlossen werden kann. Es ist dargestellt, wie durch die vom Deckel ausgehende Führungsschiene(59) der mittlere Punktionsgriffel, welcher über dem Trichter(60) der Saugglokkenöffnung liegt, unter das Niveau der übrigen Griffel gesenkt ist. letztere stehen auf dem feststehenden Transportsteg(61) und sind durch die Haltefäden (62) untereinander verbunden. Das rechte Transportrad(63) für die Punktionsgriffel weist an der Außenseite ein Zick-Zack-Profil auf, welches bei Eingriff des drehgesichert sich nach oben und unten bewegenden Führungsstiftes(42) von der Saugglocke aus bei jedem Hub derselben, d.h. in dessen Anfangsphase bis zum Kontakt des unteren Griffelendes mit dem Trichter(60) der Saugglocke, eine Sektordrehung der Trommel an der Transportscheibe(44) und damit den Austausch eines gebrauchten Griffels gegen einen ungebrauchten am Tiefpunkt der Führungsschiene(59) bewirkt. Im Saugglockenbereich ist noch die Einmündungsstelle(64) des Sogschlauches von der Gasstrahlpumpe zu erkennen sowie die Bohrung für die Einführung der Lichtleitfasern(65), welche man sich aber als um etwa 45° gedreht vorzustellen hat.

Die Figur 11 zeigt einer Vergrößerung von 2 : 1 im Detail den Längsschnitt durch einen Teleskop-Rohrstutzen mit der in der Haltebuchse(66) verschieblichen Schiebehülse(67), in welcher wiederum die Stange(68) gleitet. Letztere steht über die Lasche(69) in starrer Verbindung mit dem Teleskop-Rohr-Schieber(52) für die Abwärtsbewegung des Bowdenkabels(56). Der benachbarte Teleskop-Rohr-Schieber wiederum sorgte für eine Rechtsverschiebung der Schiebehülse(67) über die gestrichelt dargestellte starre Laschenverbindung. Linksseitig ist eine Laschenverbindung gestrichelt angedeutet, welche über eine Verschiebung des Teleskop-Rohr-Schiebers eine Linksverschiebung der Stange bewirkt. Die Laschenverbindung zur linken Seite der Schiebehülse für die Linksverschiebung ist weggelassen. Die Ausweichbewegung des Teleskop-Rohr-Schiebers erfolgt jeweils unter Einwirkung der Schaltnocke(53). Die Haltebuchse(66) ist auf der Achse(70) für den Schaltnockenantrieb montiert. Weggelassen sind die Ringrasten auf den Stangen(68) und die dort einrastenden Federn der Schiebehülsen (67), welche der Stabilisierung der einzelnen Schaltstellungen dienen.

Die Figur 12 zeigt etwa im Maßstab 2 :1 einen Längsschnitt durch einen Punktionsgriffel mit Teller(50), Schaft(71) mit Anschliffzone(a),Abwischzone(b) und Akzeptor-Zone(c) sowie der Meßzone(73) innerhalb der Griffelbohrung und der Einkerbung(72) für die Führungsschiene(Fig.9,59).

Die Figur 13 beschreibt im Querschnitt einen Riegel, (Fig.9,74), welcher auf einer Halteschiene gelagert ist und durch die Druckfeder(76) nach Durchtritt eines Querstiftes an der Saugglocke(40) durch eine Lücke(77) mit überdachter Keilschräge die Saugglocke an der Rückkehr nach unten unter dem Einfluß der Rückstellfedern(Fig.9,41) hindert. Die Rastbewegung des Riegels bewirkt eine Stromunterbrechung am Schaltkontakt(78), welche der elektronischen Steuereinheit das Signal zur Eröffnung des Druckgasventiles übermittelt. Bei manueller Betätigung des Druckknopfes(80) in der Gehäusewand wird über Vermittlung eines Bowdenzuges der Riegel gegen die Druckfeder verschoben und die Lücke(77) läßt den Querstift nach unten durchtreten, so daß sich die Saugglocke unter Wirkung der Rückstellfedern senkt.

Die Figur 14 beschreibt einen Punktionsgriffel mit eingelassener unten etwa 2mm vorstehender Kapillare (81), Belüftungsbohrung(82) und am Kopfende eine den Spaltraum zwischen Kapillare und Belüftungsbohrung dichtend überbrückende Deckelkappe(82) sowie die Kappe(55) zur Beförderung des Punktionsgriffels. Wie unten auf einem Querschnitt zu erkennen ist, wurde dem Punktionsgriffel ein ovalärer Querschnitt gegeben mit zwei Bohrungen(49) für die Haltefäden, während die Deckelkappe oben kreisförmig ist. Die Darstellung im Längsschnitt in der Vergrößerung 1 : 2 umfaßt auch eine Saugglocke(40) mit Trichter(60) zur Aufnahme des Punktionsgriffels und eine Schale(83) mit zentraler Öffnung für den Hautdurchtritt, welche vom Permanent-Magnetring(84) festgehalten wird. Nachdem die Rollbewegung der sich unter Sog aus der Einmündungsstelle (64) des Sogschlauches innerhalb der zentralen Öffnung der Schale hebenden Haut zur Ruhe gekommen ist, wird dieser zentrale Hautbereich durch Anhebung der Schale mit der Haut in die Kapillare gehoben, welche die Haut senkrecht durchbohren kann. Schnittverletzungen durch die Kapillare bei seitlichem Wegziehen der Saugglocke sind bei der hohen Biegsamkeit der Kapillare nicht zu befürchten. Diese Biegsamkeit verlangt aber auch die genau senkrechte Einstichrichtung. Die weiche Druckfeder bringt die Kappe, soweit die Schwerkraft dies nicht bewirkt, wieder in Kontakt mit dem Permanent-Magnetring-(84).

Die Figur 15 gibt einen schematischen Längsschnitt durch eine Saugglocke und die Anordnung von Druckfedern und deren Sperraster(a,b,c,d,e) bei mit der Saugglocke verbundenem Transportsteg(61) samt Transporttrommeln(63). Die Sensorkanüle(86) oder der Punktionsgriffel wird vom Zentralstift(87) gehalten, der von der Rohrmanschette(91) umgeben ist. Letztere trägt an drei auf dem Kreisumfang verteilten an ihm befestigten Auflageplatten(88) die Druckfedern(89) (siehe rechts abgebildeter Querschnitt). Der Distanzierungsstift(90) vom Deckel aus hält den Sperraster (a). Die Sperraster(b,c) für das andere Druckfeder ende sind an einem Distanzierungsstift auf dem Transportsteg(61) verbunden. Am oberen Ende der Rohrmanschette(91) liegen auf drei Querarmen(92) je eine Druckfeder(93) von unten an, deren unteres Ende dem Transportsteg(61) aufliegt. Der obere Sperraster(d) zu diesen Federn sowie der untere Sperraster(e) befinden sich an dem längeren Haltesteg(94) vom Deckel aus (wobei unter Deckel hier nicht der Gehäusedeckel (24), sondern eine gesonderte Abdeckplatte(95) verstanden werden soll. Nur im Querschnitt rechts sind die beiden Rückstellfedern(41) für die Saugglocke abgebildet. Bei Anhebung der Saugglocke durch Hautandruck werden die oberen Druckfederenden(93,89) durch die Sperraster(d,a) festgehalten, die Druckfedern werden gespannt. Wird der Sperraster(b) gelöst - beispielsweise durch Bowdenzugbewegung von einer Magnetschaltvorrichtung aus- so senkt sich die Kanüle bis zur Raststellung(c), nach Auslösung von (c) senkt sich die Kanüle vollständig. Jetzt wird der Sperraster(a) entblockt, um die Aufwärtsbewegung der Kanüle nicht zu behindern, welche nach Lösung des Sperrasters(d) erfolgt. Nach Entblockung von (e) erfolgt die Senkung der Saugglocke unter dem Einfluß ihrer beiden Rückstellfedern(41), siehe Querschnitt rechts). Die Einschiebung des Keilhebels(96) von rechts führt zuletzt zur Wiederanhebung der Saugglocke.

die Figur 16 gibt in perspektivischer Seitenansicht zwei Schieber einer Federspannvorrichtung wieder, welche das Spannen von Federn mittels der Bewegung eines einzigen Drahtbügels in linker Pfeilrichtung erlaubt. Der Drahtbügel liegt der Front des Schiebers (37), beispielsweise Fig.9) an, dessen Linksverschiebung die Druckfeder(98) verkürzt, welcher der Sperrschieber(99) nachgeordnet ist. Der Knebel(100) am Schieber stößt gegen den Sperrwinkel(101) und legt diesen nach links um, wobei dessen zweiter Schenkel sich gegen die Querplatte des Schiebers stützt -nach wiederaufrichtung durch die Spannfeder(102), so daß der Schieberknebel unter Einfluß der Druckfeder nicht nach rechts zurückkehren kann. Der Drahtbügel wird über eine Ausschwingung in der Schlitzführung der hinteren Seitenwand( die vordere wurde nicht gezeichnet) hochgehoben und damit von der Frontkante des Schiebers abgehoben; er kommt dann auf die Frontkante des zweiten Schiebers zur Anlage und kann diesen nach links mitnehmen. Diesmal wird die Zugfeder(104) bis zur Feststellung des Knebels am Raster(105),symbolisch) gespannt. Solche Federspannvorgänge lassen sich entlang der gesammten Gehäuselänge fortführen. Durch eine gleichmäßig wirkende Rückfeder-(ähnlich bei einem Rollbandmaß) wird der Drahtbügel nach dem Spannvorgang wieder nach rechts in die Ausgan lage zurückgeführt. Wird der Sperrschieber(99) angehoben so bewegt sich der Schieber(37) in Richtung der Sperrplatte des Rasters(105) und löst die Sperrung einer von oben nach unten gefederten Bewegung des Stiftes(106), indem dessen Querstift in der erweiterteten Bohrung der Sperrplatte keinen Halt mehr findet. Während eines kleinen "Überschubes" über die für die Auslösung notwendige Strecke hinaus, kommt der Schlitz(107) unter den zweiten Schenkel des Sperrwinkels(101), wobei die in der Druckfeder(98) noch gespeicherte Schubkraft, diejenige der Spannfeder(102) übertrifft, so daß der Knebel(100) den Sperrwinkel überholen kann und der Schieber nach rechts in die Ausgangslage zurückkehrt.

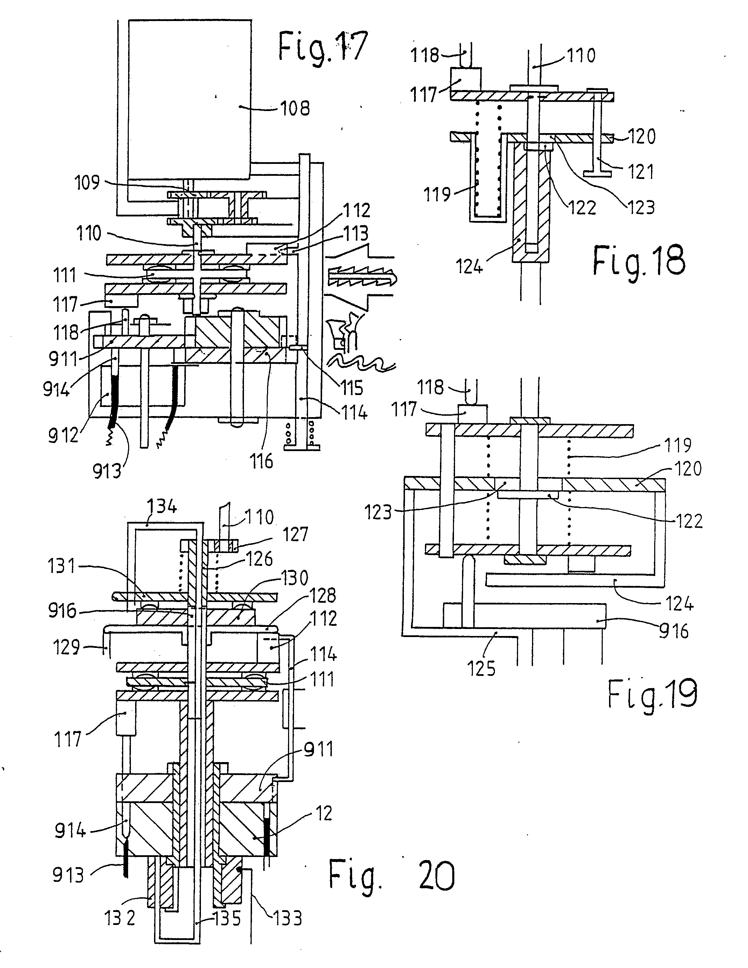

Dienen schon die Einzelheiten der Figuren 15 und 16 hauptsächlich der Verwirklichung eines messenden Sauginjektors innerhalb eines flachen Gehäuses, so gilt dies noch mehr für die Beispiele einer elektrischen Magnetschaltvorrichtung, wie sie in den Figuren 17 bis 22 beschrieben werden.

Figur 17 gibt im Längsschnitt eine Magnetschaltvorrichtung wieder unter Verwendung des Elektromotors(108), welcher über das Getriebe(109) eine langsamere Drehung der Antriebswelle(110) erreicht. Letztere treibt das Antriebsrad(111) mit beiderseitigen Sperrklinken, welche im Zusammenwirken mit Sperrverzahnungen an den benachbarten Schieben deren Mitnahme jeweils nur in entgegengesetzten Bewegungsrichtungen erlauben. So wird die obere Scheibe mit der der Keilnoppe(112) nur bei Drehung nach links mitgenommen( siehe seitliche Profildarstellung rechtsstehend), wobei der Querstift (113) an der nach unten gefederten Schaltstange(114) angehoben wird und damit auch der Querstift(115), welcher in das seitliche Zick-Zack-Profil des Hilfsrades (116) eingreift, das über eine Verzahnung die Schaltscheibe(911) antriebt. Nach jedem einzelnen Hub der Schaltstange kommt durch Sektorbewegung der Schaltscheibe, der von dieser ausgehende Stößel(914) über die jeweils nächste Bohrung mit einem Bowdenzug-(913) innerhalb der feststehenden Trommel(912) zu liegen. Die Umkehrung der Motordrehung in Drehrichtung nach rechts aber bewirkt die Mitnahme der unteren Scheibe mit der Keilnoppe(117), welche über Vortrieb des Arbeitsstößels(118) eine Senkung der Schaltscheibe(911) bewirkt und damit auch eine Senkung des an ihr befestigten Stößels-(914), welcher den Bowdenzug nach unten drückt und durch dessen Rückfederung wieder in die Ausganglage gehoben wird. Schalt- oder Arbeitstakte können je nach Strompolung des Motors nun frei gewählt werden.

Die Figur 18 gibt im Längsschnitt eine Ergänzung zur Vorrichtung nach Fig.17 zum Zwecke einer ruckartigen Gestaltung des Arbeitshubes. Zwar wird die Scheibe mit der Keilnoppe(117) durch den in diesem Falle stehenden Arbeitsstößel-(118) bei Drehung der Ar beitswelle(110)nur langsam vorgetrieben, aber die Vortriebsbewegung wird durch Senkung der Scheibe gegen die Druckfeder-(119) aufgenommen, während die Scheibe (120) zwar über die Schiebestange(121) in der Drehung mitgenommen, die Senkung aber solange nicht mitmachen kann, wie diese auf dem Querstift(122) aufliegt. Sobald aber -wie dargestellt- ein Schlitz in der Scheibe(120) über den Querstift zu liegen kommt, kann letztere plötzlich unter Wirkung der Druckfeder(119) tiefer treten und gibt ihre Senkungsbewegung über die Hülse(124) an den Funktionsabnehmer weiter.

Die Figur 19 zeigt im Längsschnitt eine Vorrichtung wie in Figur 10, jedoch sind beide Taktbewegungen ruckartig gestaltet worden und mit gleicher (Feder)-Kraft ausgestattet. Die Scheibe(120) ist diesmal noch oberhalb des Querstiftes(122) stehend abgebildet, wird durch den Schlitz(123) aber in diesem Augenblick noch unter Wirkung der gespannten Feder(119) tiefertreten. Inzwischen wird auch die untere Keilnoppe Spannung der unteren Feder bewirken und es zur ruckartigen Rückkehr der Scheibe(120) nach oben kommen. Durch Annäherung des eisernen Gestänges bzw. der Platten-(124,125) wechselseitig an den Permanentmagnet-(916) kommt es zu einer Lagestabilierung in den Endlagen(bei Nachlassen der Federspannung).

Die Figur 20 ziegt im Längsschnitt eine weitere Ausgestaltung der Vorrichtung nach Fig.17. Wie in den beiden vorangehenden Figuren ist der Motor und das Schaltgetriebe weggelassen worden. Es geht um die Anpassung der Höhe der Arbeitshübe in den einzelnen Schaltpositionen an wechselnde Bedürfnisse. Über die Antriebswelle(110) wird über das Zahnrad(127) die Drehung auf das Achsrohr(126) mittels dessen Zahnrad übertragen und dem Antriebsrad(111) mitgeteilt. In bei Figur 17 bereits geschilderter Weise werden die Drehungen nach rechts über die Keilnoppe(117) in Hubbewegungen des Bowdenzuges übersetzt. Auch die Drehung der Schaltscheibe(911) erfolgt in bekannter Weise über die Schaltstange(114) (hier allerdings ohne Vermittlung eines Zwischenrades). Die Keilnoppe (112) hebt aber zusätzlich den in der Schiene(129) gegen Drehung gesicherten Hebel-(128) und damit das Ritzel(130) mit Innenverzahnung in einen Zahneingriff auf der Antriebswelle. Aber nur die Gegenbewegung (also in der ursprünglichen Arbeitsdrehrichtung nach rechts) wird über Sperrzahnkranz und Klinke der Scheibe(131) mitgeteilt, deren Drehung über den Bügel(134), die zentrale Welle und den Bügel(135) auf das Stellrad (132) mit am Außenrand an-und absteigender Schneckenrille übertragen wird. Der feststehende Haken(133) in der Schneckenrille bewirkt die Annäherung und Entfernung von Trommel und Schaltscheibe an die Keilnoppe (117), wobei diese Entfernung die Arbeitshubhöhe des Stößels(914) bestimmt. Die Umkehrung der Motorbewegung nach links oder ein Weiterdrehen ohne Bewegungsumkehr schaltet das Ritzel(130) von der Motordrehung ab.

Die Figur 21 zeigt im Maßstab 2 : 1 im Längsschnitt eine Erfindungslösung unter Streckendifferenzierung; d.h. die Unterscheidung zwischen Arbeitstakt eines Hub-Magneten und Schalttakt geschieht über unterschiedliche Hubhöhen des Magneten. Unten ist ein Querschnitt in Höhe der Schnittlinie A - B dargestellt, linksseitig eine Aufrollung im Fensterbereich der Pendelhülse(oben) und der von dieser betätigten Schalthülse (unten).

Der Elektromagnet(21) steht mit seinem Ankerstößel(902) über der Pendelhülse(903), welche über dem feststehender Bolzen(906) drehbar und höhenverschieblich ist.

Der Querstift(907) im Bolzen greift durch den dreiecksförmigen Fensterschlitz(909) der Pendelhülse hinein, während die unten an der Pendelhülse befestigte Feder zunge(908) in den sägeblattartig gestalteten Unterrand der Schalthülse-(910) eingreift. Der Rotorbalken(911) ist drehbar über der feststehenden Scheibe(912) mit den Gewindebohrungen drehbar. In die Gewindebohrungen sind Bowdenzüge(913) eingeschraubt. Der Rotorbalken ist fest mit der Schalthülse(910) verbunden und trägt die beiden Stößel(914), deren rechter über der Bohrung(919) steht, während er linke über dem Seelenende eines Bowdenzuges liegt. Der Trägerring(915) trägt die Permanentmagnete(84) und wird durch Stifte(918) in der Haltebrücke(920) unter dem Elektromagneten in der Höhe fixiert.

Im Querschnitt erkennt man daß jedem Bowdenkabelende eine Bohrung(919) gegenüber liegt zur Unterstützung der Führung bei der Betätigung des Bowdenzuges durch Absenkung des gegenüberliegenden Stößels(914) trotz exzentrischer Druckwirkung auf diesen. Diese Betätigung geschieht, indem der Ankerstößel(902) gesenkt wird während der Anregung des Elektromagnetes(21). Die eiserne Scheibe am Rotorbalken(911), die eigentlich in Kontakt mit den Permanentmagneten-(84) steht wird von letzteren zuerst losgerissen, dann erst dringen die Stößel in die darunterliegenden Bohrungen ein und verhindern von nun an eine Drehung des Rotorbalkens und damit der Schalthülse(910). Durch die Fensterschrägung-(siehe Aufrollung links) bewirkt der Querstift(907) eine Drehung der Pendelhülse(903) in Uhrzeigerrichtung unter Anspannung der Torsionsfeder(921) zwischen derselben und der Schalthülse. In deren sägezahnartige Randausnehmungen rückt dabei die von der Pendelhülse ausgehende Federzunge-(908) um einen Zahn zurück, wobei der Steilrand der Flanke der Ausnehmung eine Rückdrehung der Pendelhülse unter Wirkung der Torsionsfeder verhindert. Weitere Arbeitakte des Magneten können in der eingestellten Schaltposition getätigt werden, wenn über den höhen justierbare Schalter(922) die Stromzufuhr zum Elektromagneten so vorzeitig eingeschaltet wird, daß die beiden Stößel(914) die Bohrungen in feststehenden Trommel(912) nicht verlassen. Wird die Stromeinschaltung durch den Schalter(922) durch Abstellung desselben verhindert, so verlassen die Stößel(914) die Bohrungen und die Torsionsfeder(921) dreht die Pendelhülse gegen den Uhrzeiger unter Mitnahme der Schalthülse und damit der Schaltscheibe mit den Stößeln(914), welche über ein anderes Bohrungspaar zu liegen kommen. Unter vorzeitiger Einschaltung des Elektromagneten über die Lichtschranke kann jetzt eine beliebige Zahl von Betätigungen des anderen Bowdenkabels erzielt werden. Die Rückfederung der Bowdenkabel bewirkt nach Stromabschaltung zum Elektromagneten die Anhebung der Stößel bis in die abgebildete Höhe. Unter Wirkung der Permanentmagnete erfolgt dann der Resthub und damit die Entfernung von den Bowdenkabelenden. (Bei entsprechender Genauigkeit der Oberflächengestaltung der Trommel(912) kann der Mechanismus der Trägerschiebe mit den Permanentmagneten eingespart werden). Die Rotation der Pendelhülse trotz Gegendruckes des Ankerstößels(902) wird durch eine kleine Kugel zwischen beiden erleichtert. Die Betätigung des Schalters(922) erfolgt noch unter Wirkung des Elektromagneten und bewirkt eine Abbremsung der Ankerbewegung nach oben. Wird die Stromeinschaltung -z.B. durch Abschaltung eines Thyristorsnicht wirksam, so bewirkt die Anziehung durch die Permanentmagnete den Resthub, wonach eine Rotation von deren Trägerscheibe zusammen mit der Scheibe(911) unter Wirkung der Torsionsfeder-(921) statthat.

Die Figur 22 zeigt oben im Längsschnitt eine Magnetschaltvorrichtung, bei welcher Unterschiede in der Ankergeschwindigkeit zur Umschaltung auf andere Funktionen benutzt werden. Unten ist eine Draufsicht auf die Mitnehmerscheibe(136) wiedergegeben mit dem Langloch(137) für den Durchlass des Sperrstiftes(138). Zur Reibungsherabsetzung endet dieser in einer Kugel. Auch die Schwungscheibe(139) wird in der Haltegabel(140) unter Kugellagerung in der Höhe festgehalten. Die unter Einwirkung der Senkung des Ankerstößels(902) unter Vermittlung einer Art vertikal unterbrochener Schneckenführung(141) bei Stifteingriff von der Schwungscheibe her in diese erzielte Rotation letzterer wird bei höherer Ankergeschwindigkeit beschleunigt, so daß während der Ankerrückbewegung durch Druckwirkung von Bowdenzug(913) her dann der Schlitz des Langloches(137) vom Sperrstift erreicht wird. Letzterer kann durchtreten und unter Wirkung der Druckfeder(143) kommt es zu einer Absenkung der Mitnehmerscheibe(136) und zur Betätigung der Schaltstange(114) dessen Einwirkung auf das Zick-Zack-Profil an der Außenseite der Schaltscheibe(911) deren Sektorbewegung und damit die Umschaltung veranlaßt. Die Arbeitstaktfunktion wurde schon zu Figur 17 beschrieben.

Es versteht sich, daß die Anordnung durch Anbringung eines Ankers mit Unruhe etwa bei entsprechender Verzahnung am Außenrand der Schwungscheibe auch zur taktzahlgesteuerten Umschaltung benutzt werden kann. Nach einem gewöhnlichen Arbeitstakt würde die Schwungscheibe sich wieder in die Ausgangslage zurückbewegt haben. Bei der kürzeren Abfolge einer bestimmten Zahl von Arbeitstakten könnte die Rückkehr um jeweils gleiche Differenzstrecken vermindert werden, welche Differenzen sich soweit aufsummieren könnten, daß der Sperrstift das Langloch erreicht und die Funktionsumschaltung bewirkt wird.

Die Figur 23 zeigt in einer Vergrößerung 2 : 1 im Längsschnitt eine schlauchintegrierte Impuls-Dosierpumpe. In einen Kunststoffblock(144) befindet sich eingegossen ein Silikongummi-Schlauchende(145), in dessen Innerem ein Stift-(146) locker gelagert ist, welcher innerhalb einer kugeligen Kammer(148) im Kunststoffblock eine Kugel(147) von etwas geringerem Radius aufweist, so daß -bei Verdünnung des Silikongummischlauches im Kammerbereich noch ein Spaltraum verbleibt. Die Kugelkammer wird nach oben und nach unten von ringförmigen Schlauchmanschetten(149) eingefaßt. Über die Bohrung(150) ist der Kammerraum mit einem Druckgaspolster(nicht dargestellt) verbunden, während in Nähe des Schlauchendes ein Flüssigkeitsaustrittskanal(151) zur Kanüle hin vorhanden ist. Eine Querplatte(152) mit Öffnungen für den Flüssigkeitsdurchtritt von oben innerhalb des Schlauches dient der Befestigung mit dem antreibenden Hubmagnet. Die Flüssigkeit tritt von oben unter Druckwirkung ein und kann bei gesenkter Kugel(147) in den Kammerspaltraum eintreten, aber wegen Verlegung des Schlauchkanals nach unten nicht weiter fließen. Das umgebende Druckgas im Kammerspaltraum würde durch die Flüssigkeit höheren Druckes verdrängt. Wird der Stift nach oben verschoben, so wird der Flüssigkeitszufluß zur Kammer oben abgesperrt, aber der Flüssigkeitsaustritt unten frei gegen. Dieser erfolgt unter Einfluß des Druckgaspolsters auf die Schlauchwandung im Kammerbereich.

Varianten, wie die Erzeugung von Schubbewegungen mittels eines Motors in Verbindung mit einer Pleuelstange oder die Wahl der Kreisform auch für das untere Punktionsgriffelende zur leichteren Saugglokkenabdichtung oder die Überlappung von Teilen benachbarter Gehäuseteilräume ,sollen einbezogen sein, ebenso die Messung der Öffnungszeiten des Gasaustrittsventiles und deren Addition zur Berechnung des Zeitpunktes der Druckgaserschöpfung. Die Kombination dieser Geräteform mit einem diaphanischen, punktionsfreien Meßgerät ist vorteilhaft(W086/01728) Figur 24 zeigt in natürlicher Größe im Längsschnitt eine Vorrichtung zur Hautanhebung unter Zugwirkung mit regulierbarer Zugstärke. Von einem die Hautkuppe begrenzenden Einfassungsring(868) gehen nach oben vier Gehäusestifte(869) aus, längs derer die Zugplatte(870) höhenverschieblich angeordnet ist, indem die Gehäusestifte durch Bohrungen der Zugplatte hindurchtreten. Mit dieser über die Zugfeder(872) verbunden ist ähnlich und verschieblich die Gegenplatte(873) angeordnet, mit der zentralen am Ende durch zwei Benzing-Sicherungen drehbar befestigten Einstellschraube(875), welche innerhalb eines Gewindes der Deckplatte-(874) höhenregulierbar ist. Die Hautadhesion erfolgt an dem doppelseitig wirkenden Pflaster(871) unterhalb der Zugplatte. Zum Gebrauch wird nach Pflasterbefestigung die Zugplatte gegen die Haut gedrückt und dann losgelassen, wobei die Zugfeder die Hautanspannung je nach Höhenstellung der Gegenplatte stärker oder schwächer bewirkt.

Figur 25 gibt im Längsschnitt eine Saugglocke-(24) in natürlicher Größe wieder mit zentralem Kanülenmagazin.

Der Ventilkegel(876) ist über die Spannfeder-(877) mit luftdicht abdeckbaren Justierschraube-(878) verbunden. Mittels der Federspannung wird festgelegt, bei welchem Unterdruck in der Saugglocke, der Ventilkegel aus dem Ventilsitz gezogen wird, so daß solange Luft in die Saugglocke eintritt, bis der Unterdruck auf das eingestellte Maß abgesunken ist. Die Ventilsitzstellung kann durch einen Elektrokontakt kontrolliert werden und zur Drosselung eines Vakuumgenerators benutzt werden. Andererseits kann auch das mechanische Überdruckventil durch ein elektrisch betätigtes (beispielsweise über ein Magnetschaltwerk) ersetzt sein und der Unterdruck in der Saugglocke über eine op tische Hautüberwachung (beispielsweise unter Korrelierung der Zuschaltung der Vakuumquelle mit dem Eintritt einer optimalen Hyperämie) ähnlich wie in Fig.10(65) dargestellt, gesteuert werden, um eine unnötige mechanische Hautreizung bis hin zur Hämatombildung(Blutunterlaufung) zu vermeiden.

Figur 26 zeigt im schematischen Querschnitt in natürlicher Größe drei elektrisch aufheizbare Heißluftkessel(19) zur Unterdruckspeicherung, welche ein vom Magnetschaltwerk durch Rotation betätigtes Ventil(153) umschließen; letzteres erlaubt die Erneuerung des Unterdruckes innerhalb der Saugglocke bei Bedarf noch während ein und derselben Geräteanwendung. Die Luft aus der Saugglocke erreicht über einen Schlauch, das Rohrsegment(155) des Ventiles(153) über den Kanal(154) den abgekühlten linken oberen Heißluftkessel bei der gezeichneten Ventilstellung.

Figur 27 zeigt im Längsschnitt den unteren Teil des Gehäusekastens(555) eines Gerätes zur Saugdiagnostik über einer halbkugelförmigen Saugglocke (14) aus weich-elastischem Material. Das zur Annäherung an die Haut absenkbare Schiebestift(24) enthält unten den Punktionsgriffel oder Kanülenkörper (1), welcher mittels des Schiebestiftes(24) abgesenkt und gehoben werden kann. Über den Schaft(2) führt der Druckausgleichskanal(12) über eine Bohrung im Führungsstift(42) und eine Schlauchverbindung über die Unterdruck-Meß-und-Regelvorrichtung-(591) zur Vakuumquelle. Wenn auch der manuelle Andruck dieser Saugglocke gegen die Haut bei vorrübergehender Abplattung der Saugglocke genügt, um eine Hautansaugung zu erzielen, so bietet die Unterdruckkontrolle allein Gewähr dafür, daß die Blutgewinnung vor Ablösung der Saugglocke von der Haut abgeschlossen ist, abgesehen von der Blutmengenkontrolle mittels optischer Vorrichtung.

Mit Figur 28 wird die Beschreibung verbesserter Punktionsgriffel begonnen. Die Darstellung erfolgt im Längsschnitt im Maßstab 6:1. Linkseitig (I) ist ein Punktionsgriffel vor der Benutzung dargestellt, er ist (siehe die Abbruchlinien) in der Höhe auf etwa die Hälfte verkürzt und besteht aus einem zylindrischen Kanülenträger(235), in dessen Boden zentrisch die Kanüle(236) eingelassen ist, deren Anschrägung nur so weit nach unten reicht, daß die Öffnung der Kanülenbohrung (auch bei dünner Hautbeschaffenheit) noch innerhalb der Haut liegt. Über den Rand einer becherförmigen Leiste(237) ist eine leicht zerstörbare Staubschutzfolie(238) gespannt. Die Kanüle öffnet sich nach oben in einen kapillären Spaltraum unter dem Binnenzylinder-(239) und dann in den Spaltraum(240), welcher zwischen der in exzentrischer Nischenbohrung eingebrachten stoffwechselbeeinflußbarem Meßzone(5) und einer Abplattung des Binnenzylinders sich erstreckt (siehe auch unten im Querschnitt).

Im Querschnitt ist auch die Wischzone(242) erkennbar, welche während einer Sektordrehung des Binnenzylinders im Uhrzeigersinn mit weichem, saugendem Oberflächenbelag über die Meßzone streicht und dabei von unten über die Kanüle eingedrungenes Blut aus dem Meßbereich entfernt.

Diese Drehphase ist rechts(II) oben wieder im Längsschnitt und unten im Querschnitt(A-B) dargestellt. Die Drehung des Binnenzylinders(239) erfolgt über die Innennoppe(245) des auf den Kanülenträger(235) aufgeschobenen Hüllzylinders-(243), welche in eine spiralige Außennut im Kopfzylinder(246) des Kanülenträgers eingreift. Über einen Kantstift(244) nimmt der Hüllzylinder bei seiner Drehung den Binnenzylinder mit, ohne ihn aber nach oben anzuheben. Die basale Trommel(247) des Binnenzylinders dient - wie beschrieben- der Funktion der Reinigung der Meßzone. Die reflexphotometrische Strahlenführung-(gestrichelt im Querschnitt I) erlaubt vor der Blutansaugung und nach Beseitigung des Blutes, da dann die Wischzone im Uhrzeigersinne weitergedreht wurde. Der Innenraum des Punktionsgriffels steht mit dem Saugglockenraum über den Kanal(249) in Verbindung. Dichtungsringe (248) in der Aufnahmebohrung für Punktionsgriffel im Zentrum des Saugglockendaches sorgen für die notwendige Unterdruckerhaltung im Saugglockenbereich. Bei II erkennt man, wie die Staubschutzfolie durch die angehobene Haut durch die Kanülenspitze nach oben gehoben wurde.

Die Figur 29 bringt im Längsschnitt im Maßstab 6 : 1 ein Beispiel eines vorteilhaften Punktionsgriffels unter Anwendung von Sog zur Entfernung des Blutes von der Meßbelagschicht(241). Zwischen den Dichtungsringen (248) wird ein Vakuumreserveraum eingeschlossen, wobei der Unterdruck sich aus der Saugglocke über die Kanüle und die Meßkapillare darüber mit dem Kalibersprung der Überlaufkammer(250) und über die Querbohrung (251) mit Docht(252) der Außenkammer mitteilt. (Die Pfeile in Phase I zeigen den Weg der Luft an). Durch einen klemmenden Rohransatz, welcher in den Rastnippel(253) eingreift, wird der Punktionsgriffel nun gesenkt (Phase II), so daß ein Dichtring die Querbohrungsöffnungen abdichtet. Der Pfeil zeigt den Weg des aufsteigenden Blutes durch die Meßkapillare in Richtung auf die Überlaufkammer an.

Wird nach der Wiederbelüftung der Saugglocke der Punktionsgriffel wieder angehoben bis die Querbohrung zwischen den Dichtringen liegt, so wird durch die Querbohrung das Blut durch das gespeicherte Vakuum zwischen den Dichtringen aus der Meßkapillare nach oben und außen abgesaugt und wird vom Docht(252) aufgesaugt(III); jetzt kann die Messung erfolgen.

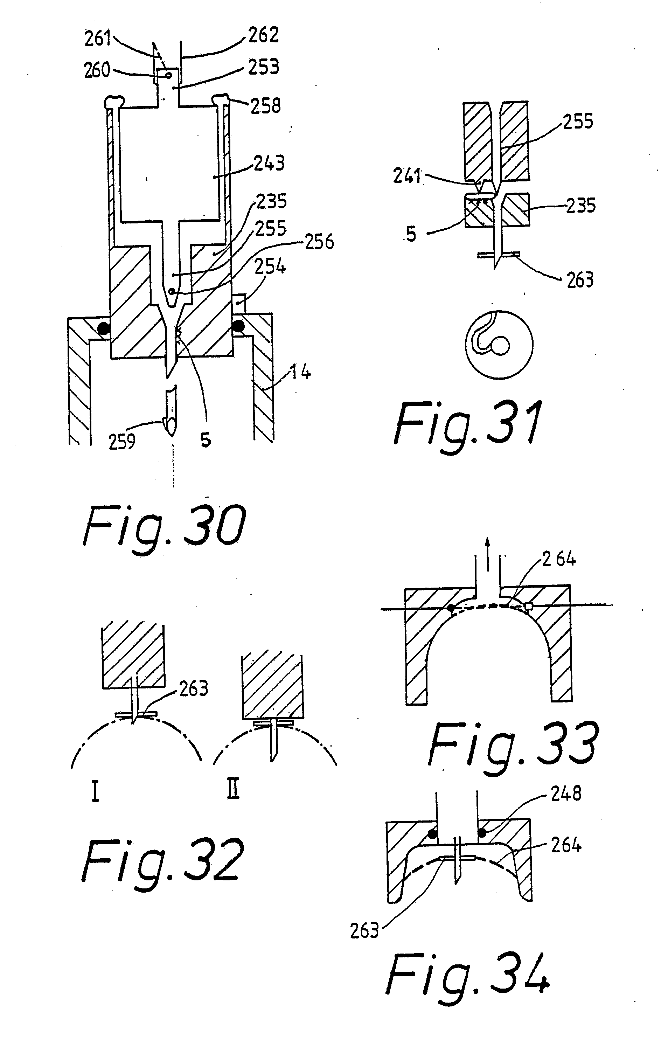

Figur 30 zeigt in schematischem Längsschnitt im Maßstab von etwa 3 : 1 einen Punktionsgriffel mit Vakuumreserveraum innerhalb des Punktionsgriffels. Dieser ist in die Saugglocke(14) eingeschoben, wobei ein Randring oder Noppen(254) als Vorsprünge an der Außenwandung das Tiefertreten unter Vakuumeinfluß verhindern. Der Hüllzylinder-(243) mit dem Rastnippel(253) umschließt den Vakuumreserveraum und weist unten ein Rohr(255) auf, welches in den Ventiltrichter gesenkt werden kann, wobei seine seitliche Bohrungen(256) verschlossen werden. Diese Phase tritt erst ein, wenn nach nach Anhebung der Haut in die Kanülenspitze von dieser aus Blut in die Kapillare mit der Meßzone(5) gesaugt wurde. Nach einer festgesetzten Zeit wird die Saugglocke wiederbelüftet, der Hüllzylinder wieder hochgezogen und das Rohrende aus dem Ventiltrichter gehoben. Dabei werden die seitlichen Bohrungen wieder freigelegt und der Überdruck von der Kanüle treibt das Blut in den Vakuumreserveraum, welcher einen saugenden Belag aufweisen kann. Kanülenträger(235) und Hüllzylinder(243) sind über eine Dichtmanschette-(258) miteinander verbunden, welche etwas Spiel für gegenseitige Bewegung freiläßt. Es ist auf diese Weise nur ein Dichtungsring(248) in der Saugglocke erforderlich.

Die Möglichkeit, den Punktionsgriffel (und/oder Teile von ihm) über eine äußeres Rad(257) zur Achsendrehung zu veranlassen, ist in Figur 29 im Querschnitt angedeutet. Es kann so eine größere Blutmenge von der Kanüle aufgenommen werden; steigern läßt sich dieser Effekt mittels einer äußeren Schneidkante (z.B.im Bereich der Anschliffzone als eine den Schaftumfang überragende Fläche, wie in der Draufsicht auf die Kanülenanschliffzone übertrieben sichtbar gemacht wurde: Detail unten.) Der Rastnippel(253) weist eine Noppe(260), welche in einen Spiralschlitz(261) eines dünnwandigen und elastischen Schieberohres(262) bei stärker Druckeinwirkung von diesem her einrastet und eine Sektordrehung des gesamten Punktionsgriffels bewirkt.

Figur 31 zeigt im Maßstab 2 : 1 einen Punktions griffel in Kombination geschilderter Merkmale, welcher zur Injektion nach Messung geeignet ist. Aus dem Ventiltrichter(257) füllt sich die angeschlossene Kapillare mit der Meßzone(5), welche (wie unten im Querschnitt zu sehen) eine Strecke weit bogig auf dem Kanülenträger(235) verläuft, um sich nach außen in den Saugglockenraum zu öffnen. Der quere Schenkel dieser Kapillare liegt auf einer Art Amboß, über welcher sich eine Art Klemmkante(241) des Hüllzylinders(243) befindet. Nach der Blutansaugung wird der Hüllzylinder gesenkt und dabei die Kapillare von der Klemmkante gedichtet. Das Rohr(255) des Hüllzylinders dient in diesem Falle der anschließenden Flüssigkeitsinjektion. Die Blutabsaugung aus der Kapillare kann über die zu Fig.30 beschriebenen Weise erfolgen. Die Höhenfixation der Kanülenspitze zur Blutansaugung wird durch einen Teller(263) bewirkt, welcher hinter der Anschliffzone dem Schaft aufgeklebt oder aufgeklemmt ist und bei gewaltsamer Senkung des Punktionsgriffels gegen die Haut nach oben geschoben wird, so daß die Kanülenspitze sicher unterhalb der Haut gelegen ist und der Einspritzung dienen kann. In Figur 32 werden diese beiden Phasen als I und II abgebildet (im Längsschnitt).

Figur 33 gibt ebenfalls im Längsschnitt eine Saugglocke wieder, welche bei Soganschluß im Kuppenbereich dort eine Art Sieb(264) aufweist zur besseren Anlage der Haut. Eine Strahlenquelle für diaphanische Diagnostik mittels Laserlichtes ist so tangential im Kuppenbereich angeordnet, daß der Lichtstrahl eine festgelegte Strecke innerhalb der Haut zurücklegt, was die Meßwertkorrektur erleichtert. Das Prinzip kann aber auch für die Untersuchung der Hauteignung zur Punktion abgewandelt werden.

In Figur 34 wird das Prinzip aus Fig.33 dadurch abgewandelt, daß die Kuppenpartie vom Teller(263) einer Kanüle gebildet wird.

Figur 35 gibt in A die Draufsicht auf eine Einrichtung zur Saugdiagnostik bei einer Gehäusegestaltung ähnlich wie in den Figuren 9 und 10 wieder; Die wesentlichste Verbesserung liegt in der Möglichkeit, bei Unabhängigkeit von einer Druckgasquelle, bei Bedarf eine Art Dauersog zur Verfügung zu haben. In der linken unteren Ecke im Aufbruch ist unter der Deckel-Wippe(559) das Dach des Gehäusekastens ein Stück weit zu erkennen; in der Tiefe folgen Batterie (54) und Steuereinheit(560) mit der Steckbuchse(561) für eine Verbindung mit einer Meßvorrichtung in getrenntem Gehäuse. Unterhalb dieser Steckbuchse hat man sich weitere, z.B. für die Stromzufuhr zur Batterie vorzustellen. Im Zentrum ist drehbar über der Deckelwippe der Klappbügel(170) in flach angelegtem Zustand erkennbar.

Die Ansicht B bringt einen Querschnitt der Einrichtung hauptsächlich längs der Schnittlinie D - E der Ansicht C. Vier Stangen(564), welche fest an der Unterseite der Deckel-Wippe befestigt sind, erstrecken sich in das Gehäuseinnere. Die beiden hinteren sind bei Stellung des Kolbens der Zylinderpumpe(562) in linker Position dargestellt. Die Kontaktfeder berührt den rechten Kontaktring(563) auf dem zum Pumpenzylindern gedichteten Pumpenstößel. Letzterer steht mit dem Schiebergestänge(582) in Verbindung, dessen Lagerbuchsen(583) an der Gehäusevorderwand befestigt sind. Das linke der beiden Transportträder(63) für den Transport der Punktionsgriffel(oder Kanülenkörper,(1) liegt reifenartig um die Batterie(54). Der Kraftantrieb erfolgt über die Transportscheibe(44) und wird wie links oben neben dem Schaltgetriebe in Fig.37 erläutert, vom Elektromotor(51) angetrieben. Die Transportscheibe bringt jeweils einen Punktionsgriffel mit der zentralen Öffnung im Saugglockendach in Deckung. Der untere Gehäuseaufbruch -längs der Schnittlinie F - G im Längsschnitt C dargestellt- läßt in gespanntem Zustand die Druckfeder (567) erkennen, deren Schubstange(575) sich gegen die Profilnase der Schalthülse(581) anlehnt. Der Rasterbolzen(574) dieser Schalthülse liegt innerhalb einer Ausfräsung in der Gehäusewand. Die Drehung der Schalthülse erfolgt über das Zahnrad(572), welches vom Zahnrad(571) mit Kraftschluß zum Schaltgetriebe betätigt wird. Die Verbindungslaschen(565) zwischen der Kolbenstange und dem Schiebergestänge, welche unterhalb der Schnittebene liegen, sind gestrichelt dargestellt.

Der Längsschnitt C zeigt im linken Aufbruch -längs der Schnittlinie H - J des Querschnittes B- die Anlage der Stange(564) am Teller des Schiebegestänges(584). Der mittlere Aufbruch - entsprechend der Schnittlinie K - L des Querschnittes B- zeigt die Saugglocke(14) mit den Durchlaßschlitzen(579) für die Griffelkörper(1), welche von dort über die Führungsschiene(59) weitertransportiert werden. Mit der Saugglocke ist die Trägerplatte(577) für die Transportscheibe(44) festverbunden. Die Höhenverschiebung des Punktionsgiffels erfolgt über einen vom Schaltgetriebe her betätigten Bowdenzug mit dem Bowdenzuglager-(578). In Stellung gehalten wird die Saugglocke durch schräg angeordnete Druck- oder Balancier-Federn(580). Die bereits erwähnte Führungsschiene ist im rechten vorderen Längsschnitt dargestellt(C). Die Funktionsbeschreibung der Einrichtung erfolgt nach der des Schaltgetriebes-(Fig.37). Figur 36 zeigt eine Einrichtung ähnlich derjenigen der Fig.35, welche jedoch auch zur Injektion geeignet ist. Die Vakuumerzeugung erfolgt mittels einer Kolben-Zylinder-Pumpe, die mittels Druckgases angetrieben wird. Im Querschnitt A -längs der Schnittlinie D - E des Frontalschnittes C dargestellt-die Druckgaspatronen(26), von denen die oberste ge zeigt wird, liegen innerhalb von getrennten Gehäusezylindern, welche durch Schraubverschlüsse(584) rechts abgedichtet sind, deren Öffnungsdorne(28) die Weicheisenkappen der Druckgaspatronen eröffnet haben. Über die Sitzventile(584) ver-einigen sich Druckgasrohre, welche dann über ein vom Schaltgetriebe betätigtes Sitzventil(586) (nach Rückschlagventilen) und den Druckmeßer das Druckgas über das gesteuerte Verteilerventil(588) den Zuführungsleitungen(585,589) zuführen. Jede von diesen endet an je einem Pumpen-Doppelzylinders(590). Die inneren Verteilungsleitungen in Nähe der Zylindertrennwand führen ebenfalls über das Verteilerventil(586) und die Unterdruckmeßvorrichtung(591) im Nebenschluß über einen Schlauch zur Saugglocke(Teilquerschnitt B- in Höhe D - E des Frontalschnittes C). Vorn im Querschnitt A -in Höhe F - G des Frontalschnittes C-kann man den zweigeteilten Zylinder für die vier elastischen Flüssigkeitsbehälter(701), je zwei für verschiedene Arzneien, erkennen, welche durch die Schraubdeckel(728) gedichtet verschlossen sind. Die Flüssigkeitsbehälter befinden sich innerhalb von Kappen(595), deren Wandungen aus gleichbreiten Zinken(198) bestehen, welche jeweils bis in die Zwischenräume zwischen den Zinken der nächstliegenden Kappe reichen (linksstehend im Querschnitt dargestellt). Die Verbindungsschläuche(594) zu den Dosiervorrichtungen. der durch die Dichtung im Schraubdeckel-(728) hindurch mit dem Einstecktrichter(596) verbunden ist. Die Darstellung von Batterie, Steuervorrichtung, Motor und Schaltgetriebe (in der Mitte vor und hinter der Saugglocke) wurde unterlassen; letztere finden sich in Fig.37 verdeutlicht.

Der Frontalschnitt C zeigt die Lage der bereits beschriebenen Gehäusezylinder, die Abstützung der Saugglocke(14) durch die auf dem Umkreis entsprechend verteilten Balancier-Federn(580), eine der Dosiervorrichtungen(599) mit Verbindungsschlauch (594) zum entsprechenden Flüssigkeitsbehälter und die Schlauchverbindung(600) zwischen Saugglocke und Pumpen-Doppelzylinder. Die zentrale rinnenförmige Ausmuldung der Gehäuseunterfläche um die Saugglocke herum wird gezeigt sowie die Lichtschranke(7-8). Der Kanülenwechsel erfolgt über den Antrieb des Transportrades(650), während die Transportscheibe (s44) durch einen Kurvenbogen(405) der Transportschiene ersetzt ist. Die reflexphotometrische Anordnung wurde als anderswo behandelt(z.B.zu Fig.59) ebenfalls weggelassen.

Figur 37 bringt rechts im Querschnitt etwa im Maßstab 2 : 1 den Elektromotor(51) und das Schaltgetriebe(571), wobei die Einzelheiten des Elektromotors weggelassen wurde. Aus ihm führt um die Zentralachse(601) die Antriebshülse(602), welches das Ritzel(603)antreibt. Dieses wiederum treibt das Zahnrad(604) mit dem Ritzel(605), welches das Zahnrad(606) antreibt und mit ihm das Antriebsrohr(607). Auf letzterem sitzt das Sperrzahnrad(608) fest, von dem die Sperrklinke-(609) am Zahnrad(610) nur Rechtsdrehungen abgreift und über das Zahnrad(611) an die Zentralachse(601) weitergibt. Dessen Ritzel (612), welches mit dem Schaltritzel(613) in Eingriff steht; treibt über das Ritzel(614) die Arbeitswelle(615). Die Verschiebung des Schaltritzels (613) erfolgte gegen die einem Spalt- oder Bensingring auf der Arbeitswelle aufliegende Blattfeder(617) durch die seitliche Keilprofilnase(616) am Zahnrad (618), welches vom Zahnrad(619)- beide auf ihren Achsen freidrehend- angetrieben wird. Zahnrad(619) holt sich nur linksgerichtete Drehungen von dem Sperrzahnrad(620) über die Sperrklinke(621) am Zahnrad(619), welches -sektorverschoben zum Keil auf Zahnrad(618)- ebenfalls eine Keilprofilnase (621) aufweist, welche die benachbarten Schaltritzel verschieben kann. Auf diese Weise ist die Betätigung von vier Arbeitswellen nacheinander bei Änderung der Antriebsrichtung des Elektromotors demonstriert. Die Anordnung weiterer Antriebsachsen zur Betätigung weiterer Schaltfunktionen erscheint beliebig; vier weitere Schaltritzel analog zu (613) können auf Schiebeachsen parallel und unter sowie oberhalb der Ritzel(612,622) von diesen betätigt vorgestellt werden.

Linksseitig vom Schaltgetriebe ist oben die Übersetzung der Arbeitswellendrehung in eine Sektorbewegung der Transportscheibe(44) für die Kanülen wiedergegeben. Während jeder Linksbewegung des Rades(623) greift dessen Schaltnocke-(624) in eine Schrägnut der Trommel(625) ein, deren Sektorbewegung sich der Transportscheibe mitteilt.

Links unten wird eine Schubbewegung demonstriert, wie sie zur Senkung des Schiebestiftes für die Kanüle stufenweise erforderlich ist. Die um ihre zentrale Achse gedrehte Profilscheibe(626) treibt eine Exzenterstange(627) an und diese wieder das buchsengeführte Stoßgestänge(628). Das Ausmaß der einzelnen Sektorenbewegungen wird durch einen zum Drehzentrum hin federnden Bolzen(629) reguliert, welcher bei Ausweichbewegung in Einsenkungen der Profilscheibe einen Schaltkontakt betätigt, um den Elektromotor abzuschalten. Ein Zuviel an Drehung kann durch leichte Rückdrehung der Profilscheibe unter Bolzeneinwirkung ausgeglichen werden.

Zur Inbetriebnahme einer Einrichtung nach Fig.35 wird nach Abnahme eines Gerätedeckels die Batterie eingeschoben und die durch ihre Haltefäden elastisch federnde Kette der Punktionsgriffel über Transportträder(54)und Transportscheibe(44) eingelegt und das Gehäuse wieder verschlossen. Die Deckelwippe (559) wird mit der Handfläche nach rechts gedrückt, wobei die Enden der rechten Stangen(564) eine über Einwirkung auf die senkrechten Leisten an den Laschen(565,566) eine Linksverschiebung des Pumpenkolbens über das Schiebegestänge(582) bewirken sowie eine Linksverschiebung der Schalthülse(581). Letztere wurde nach Steuerimpuls bei Betätigung eines (nicht dargestellten) Elektro-Kontaktes an der Deckelwippe durch das Schaltgetriebe leicht gedreht, so daß ihre Profilnase hinter der Schubstange(575) zur Anlage kam und so die Druckfeder(567) gespannt wurde. Eine weitere Drehung der Schalthülse ließ den Rasterbolzen(574) in die Ausfräsung der Gehäusewandung eintreten, so daß die Entspannung der Druckfeder verhindert wird. Wird die Saugglocke gegen die Haut gedrückt, so wird diese gegen die Balancier-Federn (580) angehoben und die Startkontakte(576) werden bei senkrechter Aufwärtsbewegung der Saugglocke gleichzeitig betätigt und über das Schaltgetriebe die Schalthülse-(581) soweit gedreht, daß der Rasterbolzen die Bewegung der Druckfeder(567) freigibt, welche sich über Schubstange(575) und Profilnase der Schalthülse auch dem Ende der Stange(564) mitteilt und über die Verbindung der Lasche(566) mit dem Schiebergestänge(582) dem Pumpenkolben. Dessen Rechtsbewegung bewirkt die Erzeugung eines Luftunterdruckes links, welches sich nach Öffnen eines (nicht dargestellten) Rückschlagventiles über die Schlauchverbindung(600) der Saugglocke (14) mitteilt und die Hautansaugung bewirkt.

Eine (nicht dargestellte) Lichtschranke innerhalb der Saugglocke überprüft die Haut auf Eignung zur Punktion und kontrolliert den Höhenstand der Hautkuppe. Sobald der Unterdruck in der Saugglocke nachläßt -auch mittels eines Unterdruckprüfers(Fig.36B, (591) kontrolliert- warnt ein Summer im Zusammenhang mit der Steuereinheit(560) und über die Betätigung der Deckelwippe kann der Unterdruck ergänzt werden. Zwei Kanäle an den Enden des Pumpenzylinders sind nicht nur über Rückschlagventile mit der Saugglocke, sondern auch mit der Außenluft verbunden. Letztere Ventile werden geöffnet, um die Luft vor dem Kolben herauszulassen, während der Sog hinter dem Kolben sich jeweils dem Saugglockenraum mitteilt. Der Mechanismus für den Punktionsgriffelwechsel wurde schon vor der Auslösung der Druckfeder(567) betätigt und der Punktionsgriffel soweit mit Hilfe des Schiebestiftes(24) (24)-abgesenkt und das Saugglockendach abgedichtet.

Es erfolgt nun die Hautpunktion und Messung nach den zu den betreffenden Beispielen zu diesem Mechanismus aufgezeigten Gesichtspunkten. Die Wiederbelüftung der Saugglocke kann durch Anhebung des Punktionsgriffels erfolgen, der die zentrale Öffnung im Saugglockendach frei gibt. Es ist zweckmäßig, die Mitbewegung der Deckelwippe unter Einfluß der Druckfeder (567) auszuschalten, indem ein Mechanismus vorgesehen wird, welcher über das Schaltgetriebe beispielsweise gegen eine Rückfederung nach Spannung dieser Druckfeder eine Verschiebung der Deckelwippe längs ihrer Drehachse(568) dergestalt bewirkt, daß die Stangen (564) aus ihrer Angriffsfläche mit dem Schiebergestänge(562) in vorgesehene Schlitze verdrängt werden. Nach Gebrauch kann über Drehung des hochgezogenen Klappbügels(570) die Verriegelung der Teleskophülsen (568) untereinander gelöst werden; dieselben werden gegen die zentrale Druckfeder unter Annäherung der Deckelwippe an das Gehäuse zusammengeschoben und in diesem Zustand durch Gegendrehung am Klappbügel wieder verriegelt.

Die Betriebsvorbereitungen für die Einrichtung nach Fig.36 sind ähnliche wie diejenigen zu Fig.35; das Kanülenmagazin und die Transportvorrichtung hat man sich entsprechend vorzustellen, zusätzlichen Platzbedarf bedingen der Insulinvorrat und die Druckgaspatronen und zusätzliche Ventile. Auch hier ist das eigentliche Glukosemeßgerät in getrenntem Gehäuse untergebracht und steht über Lichtleitkabel und und elektrischem Kabel mit Steckerverbindung mit dem Injektorgehäuse in Verbindung. Bei nur diagnoschen Gebrauch oder wenn die Meßergebnisse erst nach der Injektion verwertet werden sollen, kann der Kontakt zwischen den beiden Gehäusen(vg.Fig.54) auch nach der Punktion innerhalb der Bluteinwirkungszeit auf die Indikatorschicht hergestellt werden (z.B. über Steckkontakt übereinanderliegend). Die bekannte Technik der Reflexphotometrie wird hier nicht abgehandelt. Die reflexphotometrischen Anordnungen wurden ebenfalls weggelassen und können beispielsweise der der Fig.59 entnommen werden.

Die Dosiervorrichtungen -beispielsweise nach Fig.23 oder 48- werden mit der Saugglocke(14) oder dieser aufgesteckt zusammen mit den Verbindungsschläuchen von Zeit zu Zeit ausgetauscht. Der fortlaufende Ersatz der Flüssigkeitsbehälter(701) bewirkt eine ständige Reinigung der Schläuche und Dosiervorrichtungen- (siehe auch Fig.65;Fig.66). Die Anzeige nahender Vorratserschöpfung - auch für Punktionsgriffel und Druckgas erforderlich- kann rechnerisch über den Verbrauch und auch durch manigfaltige Kontrollvorrichtungen, insbesondere Berührungskontakte für die Druckwirkung auf Schläuche oder bewegte Teile in Flüssigkeit und Gas und über Lichtschranken oder Lichtreflexion erfolgen. Da die Gas-und Flüssigkeitsvorräte doppelt angelegt sind, kann nach Warnung durch die Steuervorrichtung die Vorratsergänzung rechtzeitig erfolgen. Nach Erschöpfung eines Druckgasvorrates wird über das Schaltgetriebe(Fig.37) das Sitzventil(586) des angeschlos senen Druckgasbehälters geöffnet; Rückschlagventile (584) verhindern den Druckgasabstrom rückwärts beim Wechseln der Druckgaspatrone. Die Arznei wird mittels Gasdruckes aus den Flüssigkeitsbehältern befördert, welcher aus der Druckgasleitung zur Pumpe über das Druckreduzierventil(630) vor den Rückschlagventilen der zwei Einzelzylinder für die Kappen(595) abgeleitet wird. Ein rutscharmer Überzug (beispielsweise aus Gummi)(C,636) beidseits längs der Ausmuldung der Gehäuseunterseite ist zweckmäßig. Wird diese Unterseite mit der Muldenachse parallel zur Achse der Wölbung der Körperoberfläche der haut angedrückt und werden die Startkontakte(576) am Saugglockenrand gleichzeitig betätigt, so wird zunächst über die Steuereinheit das Schaltgetriebe zur Betätigung des Kanülentransportes(Fig.37 links oben) aktiviert und dann eine neue Kanüle durch den Schiebestift in die Dichtung im Zentrum der Saugglocke eingeführt und dann durch Öffnung des Sitzventiles(586) die Zylinderpumpe(590) betätigt. Je nach Rückmeldung des Kontaktes eines Kolbens mit der Kontaktdruckfeder über die Signalleitungen(585,589) wird dabei das Verteilerventil(588) durch Rotation so eingestellt, daß die Druckgaszufuhr über den Druckprüfer(587) über die linke oder rechte äußere Kanalverbindung, nämlich hinter den Kolben welcher die Kontaktfeder berührt, erfolgt. Die Sogkanäle aus der Zylinderpumpe in Nähe der Trennwand(636) mit der Dichtung um die Zwischenkolbenstange werden so abwechselnd mit der Außenluft oder mit der Saugglocke durch das Verteilerventil verbunden, daß der jeweils nicht gasbeaufschlagte Kolben saugend die Luft aus der Saugglocke über den Verbindungschlauch (C,594) aufnimmt. Hierzu sind zwei Kanäle nahe der Scheidewand der Pumpenzylinder über Rückschlagventile(407.408) mit der Außenluft verbunden, die luftableitenden Kanäle über ein Rückschlagventil(409) mit der Saugglocke. Die in die Saugglocke sich erhebende Haut kann leicht gegen den Druck der Balance-Federn(580) an der glatten Oberfläche des Saugglockenrandes(40) vorbeigezogen werden und nimmt den nach oben hin beweglich innerhalb der Saugglocke gelagerten Dichtungsring(633) innen leicht nach oben mit-(bzw.wird bei harter Beschaffenheit desselben ringförmig von diesem eingeschnitten). Die Lichtschranke(7-8) überprüft die Eignung der Hautkuppe für die Punktion. Bei Bestätigung durch die Steuereinheit wird die Kanüle zur Punktion abgesenkt und die Stoffwechselmessung nach einer der gesondert beschriebenen Funktionen und besonderen Anordnungen vollzogen. Nach Vergleich der Meßwerte mit dem Dosierprogramm werden gegebenfalls nacheinander die Dosiervorrichtungen(199) für schnell und langsam wirkendes Insulin taktweise betätigt und die Saugglocke dann wiederbelüftet. Die Sichtbarmachung des Meßwertes(oder die akustische oder palpierbare Zahlenwiedergabe) kann am Meßgerätgehäuse erfolgen. Die Registrierung der Meßwerte, der verabreichten Mengen und anderer wichtiger Benutzungsparameter erfolgt, wenn schon das Meßgerät mit dem Injektor zur Punktion verbunden werden kann, zweckmäßigerweise in einem dritten Gehäusezusammenhang mit dem elektrischen Ladegerät. Die Absaugung der Saugglokke wird häufig nicht über einen besondere Schlauchverbindung(600), sondern über die Kanüle oder den Punktionsgriffel erfolgen. Das Prinzip der Arzneibevorratung wird zu den Figuren 65 bis 68 ausführlicher beschrieben, die Einzelheiten im Kanülenbereich können den verschiedenen Zeichnungen(z.B. Fig.59) entnommen werden.

Figur 39 zeigt im Längsschnitt im Maßstab 2 : 1 eine Saugglocke(14) mit innerer Dichtungstülle-(638) für eine Anlage der hochgezogenen Haut mit höher ge zogenem ringförmig diese Tülle umbendem Reserveraum. in welchen eine besonders elastische Haut ausweichen kann. Der Transporthebel(639) befindet sich innerhalb einer Membranzone im Saugglockendach gedichtet, welche unter Sogeinfluß gesenkt wird. Ist nach Überprüfung der Hautkuppe durch die Lichtschranke(7-8) die Kanüle abgesenkt, so greift an der Riffelung des unteren Randes der gesenkten Kanüle der Transporthebel an und bewirkt die Kanülendrehung über eine vom Schaltgetriebe her gesteuerte Pleuelstangenbewegung. Die Abbildung zeigt den Zustand vor der Punktion.

Fig.40 zeigt im Längsschnitt im Maßstab 2 : 1 eine Saugglocke mit einer Art aufblasbarem Randring, (340) links in schlaffem Zustand desselben im Stadium beginnender Hautanhebung, rechts mit aufgeblähtem Randring bei bereits angehobener Haut.

Fig.41 zeigt im Längsschnitt im Maßstab 4 : 1 einen Punktionsgriffel für die Blutabnahme mit einem Loch(641) in der Kanülenspitze; dieses folgt bei Rotation des Schaftes der Schneide und bleibt den verletzten Gefäßen zugewandt, was die Blutaufnahme in den Schaft unter Sogwirkung von der Kapillare(3) her an der Meßzone(5) vorbei begünstigt. Im dargestellten Beispiel ist die Kapillare so im Schaftende verkittet, daß von der Kanülenschneide her kein Gewebe eingesaugt werden kann, welches den Blutstrom behindern könnte.

Figur 42 zeigt im Längsschnitt etwa in natürlicher Größe eine Saugglocke(14) mit gummielastischem Kanülenkörper(644), in welchen ein durchsichtiger Kanülenträger(235) mit dessen Außengewinde eingeschraubt ist, wobei die Gewindegänge nach oben und unten verschlossen sind. Der Kanülenschaft endet oben in diesem Gewinderaum während unten der Querkanal(643) einmal mit der Saugquelle und später mit der Dosierpumpe verbunden wird. Mittels eines vom Schaltgetriebe bewegten Transportrades(645) mit Achsdichtung zum Saugglockendach, das innerhalb der Saugglocke eine Art Schneckenprofil aufweist, kann der Kanülenträger zugleich mit einem Trägerstab für eine Lichtquelle(7) der Hautkuppe entgegen gesenkt werden. Der Lichtempfänger(8) links ist in ein fächerartig verbreitere Lichtleiste aufgeteilt. Der Anschluß zum Reflexphotometer(18) ist linksseitig eingezeichnet.

Figur 43 zeigt in nahezu natürlicher Größe im Längsschnitt eine Saugglocke(14) mit zur ihr kolbenartig gedichtetem Dach(648), welches in der links dargestellten Version von einer Zugfeder(648) von der Haut bis zu deren Anhebung durch Sogwirkung in Abstand gehalten wird. Die Rechte Hälfte gibt eine Version wieder, bei welcher unter Steuerung die im Dach festmontierten Lichtschranke(7-8) vom Schaltgetriebe mittels der Transportschraube(665) der Haut entgegen gesenkt werden kann.

In Figur 44 wird etwa im Maßstab 4 : 1 im Längsschnitt eine Sensorkanüle dargestellt, dessen Aufnahmebecher(669) im Kontakt mit dem schiebestift (24) sich befindet und in der Dichtung der zentralen Öffnung der Saugglocke feststeht, während der untere der die Trägerhülse(266) zu diesem Aufnahmebecher drehbeweglich angeordnet ist, wobei diese Drehung innerhalb der Saugglocke über das Transportrad (650) vom Schaltgetriebe her bewirkt wird. Vom Becher(268) aus ragt ein Schläuchchen mit der Meßschicht(5) durch eine senkrechte Bohrung in der Trägerhülse(266) durch das oberhalb des Kanülenspitzenbereiches befindliche Loch(641) im Kanülenschaft bis zur Kanülenöffnung hin. Der Kanülenschaft selbst endet in Richtung Dosierpumpe in der Ringfurche für Arzneiflüssigkeit(6). Nach Ansaugung des des Blutes über den (nicht dargestellten) Druckausgleichkanal(12) wird die Trägerhülse(266) gegen im Becher(268) feststehenden Schaft gedreht, wobei das Schläuchchen aus diesem herausgezogen wird; schließlich wird auch das Loch(641) am Ovalschlitz des Kanülenkörpers vorbei verschlossen und die Injektion kann erfolgen.

Im Querschnitt darunter erkennt man die Lichtschranke(7-8) und den gegen die Meßzone(5) gerichteten Sender-Empfänger(48), welcher auch die Funktion der Lichtschranke übernehmen kann.

Figur 45 zeigt etwa im Maßstab 2 : 1 im Längsschnitt eine andere Variante der Sensorkanüle, die wiederum aus Aufnahmebecher(268) und Trägerhülse (266) besteht. Nach Abhebelung des Deckels(252) um die Kanülenspitze und Herstellung der Verbindung zwischen röhrigem Schiebestift(24) mit anschließendem Ventil vor einem Vakuumstauraum mit dem Einfüllstutzen (251), wird über Sogeinwirkung nach Ventileröffnung Blut in den Raum zwischen Schaft(2) und Binnenkanüle (653) angesaugt. Dann wird der Becher(268) gesenkt und mit ihr die Binnenkanüle, welche in den Raum unter der Haut eindringt, in welchen jetzt über die Binnenkanüle injiziert werden kann(Phase II,rechts).

Figur 46 zeigt im Längsschnitt etwa im Maßstab 2 : 1 eine weitere zweiteilige Sensorkanüle. Die Trägerhülse(249) sitzt in diesem Falle unten, weist ein Innengewinde auf und läßt sich vom in der Dichtung feststehenden oberen Aufnahmebecher(268) durch die Drehung des Transportrades(650) innerhalb der Saugglocke gegen die Haut herabdrehen; dabei wird die an der Trägerhülse gedichtet befestigte Kanüle mitgenommen. Die Höhe des Blutspiegels(über die Kanüle) auf der Meßzone(5) und der Meßwert werden durch in den Aufnahmebecher eingelassene Lichtfasern kontrolliert.

Figur 47 zeigt etwa im Maßstab 2 : 1 im Längsschnitt eine Art Kanüle mit schraubenwindungen an der Oberfläche, welche über die Drehung des Transportrades (650) bis in die Muskulatur absenken läßt. Ist die Kanüle dabei an eine Vakuumquelle angeschlossen, so kann mittels der Lichtschranke(7-8) bei Durchsichtigkeit des spitzenabgewandten Schaftteiles der Anstieg der Blutsäule festgestellt und die Rotationsrichtung des Transportrades umgekehrt werden. Die Meßzone kann streifenförmig in den Schaft eingebracht sein. Die Vorrichtung kann auch ohne Anhebung der Haut innerhalb einer Saugglocke angewandt werden, ja ohne Hautfalte.

Figur 48 zeigt im Längsschnitt eine Dosiervorrichtung im Kontakt mit einer Sensorkanüle(86). Innerhalb des Pflockes(654) als Ende eines Verbindungsschlauches(599) aus einem Arzneibehälter führt der Zuflußkanal(655) an einer Stelle an die Oberfläche des Pflockes, in deren Nähe der Querkanal(656) in die kugelförmige Meßkammer-(657) führt. Innerhalb dieser Meßkammer befindet sich ein Druckgasballon, welcher mit einem größeren Reserveballon(659) in Hohlraumverbindung steht, dessen Außenfläche mit seiner Pflockwandung fest verklebt ist. Aus der Dosierkammer setzt sich der Querkanal auf die Gegenseite der Pflockoberfläche fort, von welcher aus der Abflußkanal-(659) zur Ringnut der Sensorkanüle führt. Die über das Schaltgetriebe drehbare Schalthülse(660) zeigt sektorverschoben auf der Innenseite zwei Überbrückungsnischen(661). Während je einer Umdrehung der Schalthülse wird diesmal die unter Druck stehende Arzneiflüssigkeit in die Dosierkammer gepresst und verdrängt dabei unter Gaskompression den Druckgasballon bis zur Größe der in seinem Inneren befindlichen Gitterkugel(662); in der Folgephase gibt die andere Überbrückungskammer den Abfluß über die Kanüle frei, welcher unter Wiederentfaltung des Druckgasballons von diesem bewirkt wird. Die Ventilfunktionen werden in dieser Kontruktion besser aufeinander abgestimmt als bei derjenigen der Fig.23.

Die als Beispiel gewählte Sensorkanüle weist einen in die Ringfurche führenden Schaft(2) auf, hinter dessen Spitzenanschrägung eine Auffräsung besteht, in welchen der Schlauch(3) mit Ultrafiltrationseigenschaften in seinem unteren Ende mit der Schaft innenfläche verklebt ist. Der Schlauch setzt sich durch die Reaktionskammer-(11) mit Reagentien hindurch in den Druckausgleichskanal(12) fort, der unterhalb des Dichtringes der Saugglockenöffnung in eine Ringfurche führt; letztere steht also in Verbindung mit dem Saugglockenraum.

Nach der Punktion steigt Blut in dem Schlauch aufwärts, dessen farblose Flüssigkeit durch die Schlauchwandung hindurch mit der Flüssigkeit der Reaktionskammer(11) sich vermischt. Die Meßwerte können auf die dem Verfahren entsprechende Weise gewonnen werden (z.B. auch mittels UV.-Photometer außerhalb des Gerätes oder innerhalb des Gerätes durch tangentiale Reflexphotometrie an einer streifenförmigen Meßzone). Auch Reagentien eines aktiven oder passiven elektrischen Sensors können kanülenfern untergebracht und mit Blut beschichtet werden.

Figur 49 zeigt im Längsschnitt etwa im Maßstab 2 : 1 eine Saugglocke(14) mit einer Sensorkanüle(86), welche mit breiter tiefer Dichtungstülle(638) zur Aufnahme der Haut in einer Art zweiten Saugglocke und eine den Kanülenschaft umgebende Mulde(663)ausgestattet ist, welche in die Längsbohrung einer Reaktionskammer(11) mündet.

Auf dem Boden desselben befindet sich die fleckförmige Meßzone(5). Der Druckausgleichskanal führt über die Auffangkammer(664) weiter zum Sogschlauch(600). Auf diese Weise kann das nach der Kanülenrotation neben dem Schaft austretende Blut über der Meßzone aufgeschichtet werden. ein Vorgang welcher nach Erreichen der Lichtschranke(7-8) unterbrochen wird. Durch Zurückziehen des Kanülenschaftes nach der Punktion kann die Blutaustrittsöffnung vergrößert werden.

Es sind weitere Sogkanäle eingezeichnet, welche eine abwechselnde Sogverstärkung in den verschiedenen ringförmigen Hautkuppenbereichen zulassen. Solche Ringzonen sind: der Raum zwischen Saugglockenrand(40) und Dichtring(633), der Raum zwischen Dichtring(633) und Dichtungstülle-(638), der Raum zwischen Dichtungstülle und Mulde(663) und der Muldenraum selbst sind da zu nennen. Ähnlich wie der aufblasbare Randring (340) in Fig.40 kann die Blutverschiebung zur Punktionssstelle hin durch Änderungen der Druckeinwirkung auf die Hautkuppe - was zu einer Art Peristaltik oder Melkbewegung führt- gefördert werden.

Figur 50 zeigt in schematischem Längsschnitt etwa im Maßstab 4 : 1 den unteren Teil eines Kanülenschaftes(2) durch dessen Loch(641) eine Kapillare zur Blutnahme quer in die Gefäße führende Hautschicht vorgeschoben werden kann. Auch ein Draht(565)vorschub mag zur Verletzung von Gefäßen nützlich sein; außerdem kann eine sölche Vorrichtung dazu dienen, die Haut in angehobenem Zustand festzuhalten oder sogar hochzuziehen, selbst wenn in der Umgebung der Haut kein Unterdruck mehr vorherrscht.

In die oben offene Bohrung, welche in der Meßzone(5) endet, ist der Rohrstutzen(566), welcher mit der stärksten Sogquelle verbunden ist, dichtend eingeführt; an jenem ist eine Art Wattebausch(567) befestigt, welcher nach der Bluteinwirkung auf die Meßzone dieser zur Reinigung genähert und dann nach oben herausgezogen werden kann. Es wird dann in besagte Bohrung ein Dichtkolben(568) mit Lichtleitfasern eingeführt und nach technischem Bedürfnis für die Photometerauswertung der Meßzone genähert (siehe auch Fig.53).

Figur 50 zeigt in einem teilweisen Längsschnitt im Maßstab 4: 1 einen Kanülenschaft(2), dessen Spitzenbereich das Loch(541) aufweist, durch welches -nach Einführung der Spitze in die Haut- ein Draht quer zur Haut herausgeschoben wurde, welcher mit und ohne Kanülendrehung Kapillarzerreißungen verursacht und sowohl der Blutentnahme als auch dem Festhalten der Haut dienen kann.

Figur 51 zeigt im Längsschnitt im Maßstab 2 : 1 einen Kanülenschaft(2), von welchem sich als Reaktionskammer(11) eine zweite durchsichtige Kannüle abzweigt(die Einbettung in einen Körper(1) ist wegelassen. In den Schaft(2) wurde von oben ein hochelastisches Schläuchchen(569) eingeführt, welches mit der Dosiervorrichtung in Verbindung steht. Links im Stadium I liegt das Schläuchchen noch oberhalb halb der Kanalgabelung; es kommt zum Einströmen von Blut in die mit der Vakuumquelle verbundene Reaktionskammer(11). Rechts zeigt das Stadium II , wie sich -übrigens nachdem der Schaft(2) tiefer unter die Haut geschoben wurde- das Schläuchchen(569) unter dem Flüssigkeitsdruck gestreckt hat, so daß die Kanalgabelung verschlossen ist, und durch das geplatzte Ende des Schläuchchens die Injektion erfolgt.

Die Figur 52 zeigt oben im Längsschnitt, unten im Querschnitt etwa im Maßstab 2 : 1 eine Sensorkanüle mit Ähnlichkeiten zu derjenigen von Fig.1. Eine Art Schwammkissen(570) nimmt das nach Senkung des Kanülenschaftes(2) durch Rotation der Schneidkante(259) gewonnene Blut auf und streift dieses an einem Faden(571) hochgezogen, and die Meßzone(5) ab, welche innerhalb einer Verengung liegt, welche das Schwammkissen ausdrückt. Der Faltenbalg(572) muß dabei durch Zug von oben überreckt werden, um den am Deckel oben befestigten Faden(571) hochzuziehen; der Faltenbalg(572) kann auch zur Erzeugung von Luftströmungen innerhalb des Körpers(1) eingesetzt werden. In Bruchstücken gezeigte benachbarte Kanülenkörper zeigen durch ihre innerhalb von Längsnuten(573) verschieblichen dort schwalbenschwanzähnlich verbreiterte Haltearme(574) zur Kettenbildung längs deren die einzelnen Glieder abgesenkt werden können.

Im Querschnitt unten wird deutlich, daß mittels des evuentuell noch vorhandenen Zugseiles(575) ein Wattebausch(567) aus einer Mulde an der Unterfläche des Körpers(1) herausgezogen und an der Meßzone(1) wischend vorbeigezogen werden kann.

Figur 53 zeigt oben im Längsschnitt und unten im Querschnitt im Maßstab 2 : 1 eine weitere Variation einer Sensorkanüle mit zentralem dünnerem Schaft(2) einer Injektionskanüle. Etwas exzentrisch zu diesem findet sich die breitere Schneide in Verbindung mit der Bohrung der Reaktionskammer(11), welche durch den Dichtkolben(568) verschlossen ist. letzterer führt die Lichtleitfasern vom und zum Reflexphotometer nach Reflexion auf der -auch in anderer Größe auswählbaren- Meßzone(5). Die Kanülenkörperrotation entfaltet auch ohne Vorhandensein einer zentralen Injektionskanüle durch die exzentrische Lage der Schneide eine gute gefäßdurchtrennende Wirkung. Der Deckel(576) ist über seine exzentrische Achse (577) innerhalb des Körpers gelagert; und wurde mit seinem Kappenrand nach oben etwas abgezogen und gab nach Schwenkung die Bohrungen frei. Ein solcher Deckel kann auch vor Benutzung mit dem Körper scheibenartig verklebt sein; sein Gelenk zum Körper kann auch eine punktförmige Anschweißung sein; er kann auch zur Abdeckung der Öffnungen unten eingesetzt werden. Eine Sammelvorrichtung für Kanülenabdeckelungen wird so entbehrlich.

Figur 54 zeigt eine Schemaskizze des Gesamtgeräteaufbaues gegliedert in Gehäuse mit Saugglocke(14), Gehäuse mit Reflexphotometer(18) und ein Gehäuse mit Netzteil mit Transformator(468) für die Ladung der Batterie oder der Batterien, falls die beiden anderen Gehäuse eine selbständige Stromversorgung besitzen. Besonders im letzteren Falle kann die Übertragung von Daten des Meßwertaufnehmers zum Reflexphotometer auch drahtlos erfolgen(durch gestrichelte Linie symbolisiert). Das Gehäuse mit Reflexphotometer kann auch bestimmungsgemäß oder wahlweise (bei elektrischer oder optischer Koppelung über Kabel) über dem Gehäuse mit Saugglocke liegend dort verrastet werden. Das Gehäuse mit Netzteil enthält bevorzugt auch einen Drucker zur zeitproportionalen Sichtbarmachung der Meßwerte oder (bei Injektoreinsatz) anderer relevanter Daten.

Figur 55 zeigt im Längsschnitt im Maßstab von nahezu 2: 1 eine Sonerform einer Reaktionskanüle, bei welcher anstelle einer Kanüle eine Lanzette(265) benutzt wird innerhalb einer Saugglocke(14). Die Lanzette ist im Zentrum der Trägerhülse(266) befestigt, welche dichtend in den Aufnahmebecher(268) eingeschoben ist. Dieser wiederum weist an seiner Bodenwand einen Schlitz für den Durchtritt der Lanzette auf, welcher auch zum Durchtritt von Blut auf die Meßzone(5) dienen kann. In einer Variante befindet sich diese Meßzone(5) abgedeckt von einer Ibutaufsaugenden Watteschicht(267) an dr Unterseite der Bodenplatte und tritt dort direkt mit dem auf die Haut austretenden Blutstropfen in Kontakt. Ein nippelartiger Ansatz am Becher wird von zwei gelenkkig des Schiebestiftes(24) umfaßt. Die Senkung des Aufnahmebechers und damit die Punktion der Haut erfolgt aber unter Sogwinwirkung über den Sogkanal (670): Das Stadiumder Punktion ist dargestellt. Wird der Innenraum des Aufnahmebechers wiederbelüftet, wo kann die Lanzette über den Schiebstift leicht zurückgezogen werden, während die Haut durch den Sogring über den Sogkanal(269) als Kuppe festgehalten wird. Erneuter Sog über Sogkanal(270) läßt nun das Blut aus dem Stichkanal treten und nähert die Haut mit Blutstropfen der Meßzone(5).

Figur 56 zeigt im Längsschnitt etwa im Maßstab 3 : 1 einen Punktionsgriffel mit Lanzettenspitze(265) oder Kanüle ohne Saugglocke bei zentraler Lage der Meßzone(5) zur Erleichterung der optischen Justierung. rung. Der die Optik (Stahlenkanal oder Lichtfasern) bergende Schiebestift(24) zeigt eine Ringdichtung zum Innenrand der Trägerhülse(266), welche über die Rotation des Schiebestiftes(24) gedreht werden kann.

Die Abdeckhaube(271) liegt mit gummielastischen Rändern innen dem oberen Rand des Aufnahmebechers(268) dichtend an und stellt die Verbindung zu den Sogkanälen her, wobei Sogkanal(270) tiefer als Sogkanal (269) über seitliche Schlitze in die Trägerhülse(266) einmündet. Nach Rotation derselben über Drehung des Antriebsrades(650) vom Schaltgetriebe aus, wird der ursprünglich an die Sogquelle angeschlossene Sogkanal(269) wie auch die Rotation abgeschaltet, wenn die optische Kontrolle genügend Blut auf der Meßzone(5) bestätigt. Nach der eingestellten Einwirkungszeit wird das Blut dann über Sogkanal(270) abgesogen und die Messung angeschlossen.

Unten zeigt der Querschnitt durch die Trägerhülse(266) die Löcher(272) für den Bluteintritt und die seitlichen Schlitze(273) für die Absaugung.

Figur 57 zeigt schematisch etwa in natürlicher Größe oben in der Draufsicht, unten in einer Seitenansicht eine Variation der Vorrichtung zum Punktionsgriffelwechsel. Die Körper(1) der Punktionsgriffel, von denen zur Vereinfachfachung nur eine Reihe von acht vorn in der Mitte eingezeichnet sind, sind oben viereckig, unten aber zur Abdichtung der Saugglocke gerundet. Die Griffel wurden ohne Fadenverbindung untereinander (aus einem linearen Magazin oder aus Magazinbohrungen in langelliptischer Reihung, beides nicht dargestellt) in die Schiene(274) mit Endbogen eingelegt, deren Rück- oder Innenwand sowie Innenkante durch Herunterklappen im Scharnier(276) vom Rahmen(275) gebildet wird, der den Griffeln zugewandt zwei Randleisten aufweist, einen oberen(278) und einen unteren(279). Entsprechende Randleisten weisen auch die Griffel auf und zwar in der Weise, daß deren Aufnahme-Becher(268) nicht nach oben geschoben werden kann; die Randleiste an der Trägerhülse(266)-wenn vom Beispiel in Fig.55 ausgegangen wird-wiederum hindert später deren Tiefertreten. Die Trägerhülse des mittleren Griffels, der mit der Saugglocke(14) diese dichtend in Verbindung steht, wurde über Anhebung der Transportgabel(280) angehoben und mittels des Keilschiebers(281) nach rechts auf die obere Randleiste(279) verschoben. Dieser Keilschieber ist(in der Draufsicht) wieder zurückgeschoben, um den Eintritt der Meßstrahlen von Lichtquelle mit Detektor (48) zu ermöglichen. (Liegt die Meßzone unten, entfällt natürlich die Notwendigkeit einer Abhebung der Trägerhülse). Die Weiterbeförderung des Aufnahmebechers(268) nach Rückzug aus der Saugglocke mittels des (nicht dargestellten) Führungsrohres wird über die Einwirkung der Keilschräge des Schiebers(282) bewirkt. welcher(die Magazin-Schiene muß ja immer ganz besetzt sein) nur dann eine Verschiebung der Griffelreihe im Gegenuhrzeigersinne bewirken kann, wenn der Keilschieber(283) ganz zurückgezogen wurde(der ganz vorgeschoben in der Draufsicht dargestellt wurde). Innerhalb der Schiene wird linksseitig noch der Keilschieber(284) gezeigt, welcher -flach und gabelartig ausgebildet-den Keilschieber(281) unten und oben umgreift und -vor jenem betätigt- die Deckel (welche entsprechende Anschlagstifte(285) exzentrisch aufweisen können) beiseite schieben kann(Detail links in der Draufsicht Fig.59). In den Bogenteilen der Schiene ist diese etwas breiter gestaltet, um die Punktionsgriffel in gegenseitiger Kantenberührung hindurchzulassen. Die Keilschieber werden über eine Art Profilscheibe(626) vom Magnetschaltwerk aus betätigt.

Figur 58 bringt im Längsschnitt etwa im Maßstab 2 : 1 eine weitere Abwandlung der Sensorkanüle, welche als "Vibrationskanüle" bezeichnet werden könnte. Im dargestellten Beispiel wird als Punktionsspitze eine Lanzette(265) gewählt. Die Schwingung erfolgt, als seltener sich verstärkender Ausschlag, in der Frontalebene (zwischen vorn und hinten gegen den Betrachter), verursacht werden sie von zwei konischen Walzen (286) mit spiralig im Wechseltakt arbeitenden Nocken, von denen nur die hintere gezeigt wird (in der Praxis ersetzt durch zwei gegeneinander wirkende waagerechte Profilscheiben). Die Walze wird vom Schaltgetriebe aus gedreht und mittels einer Spiralnutführung (287)-bei stehendem Führungsstift-so verschoben, daß die Noppen an Höhe ansteigend an einem (nicht dargestellten) Vorsprung an der Abdeckhaube(271) vorbeigeführt werden. Im gewählten Beispiel steht der Schiebestift(24) durch welches hindurch auch die Messung auf zentraler Meßzone(5) erfolgt, fest. Die Schwingungen des Aufnahmebechers(268) werden dadurch ermöglicht, daß die Griffeldichtung im Saugglockendach von einem elastischen Ring(304) umgeben ist. Die Einstichöffnung in der mitschwingenden Haut wird solange erweitert, bis die erwünschte Blutmenge an der Basisplatte mit der Meßzone(5) an der Oberseite und der umgebende Tellerrand aufgefüllt ist. Nach oben hin greifen klauenartig auf dem Kreisumfang verteilte Rasterarme(288) mit ihren Enden in eine Nut des Schiebestiftes. Die Wattеschicht(267) umgibt röhrenförmig das Ende des Schiebestiftes(24). Während der Einwirkungszeit wird die Vibration verstärkt, was eine bessere Blutbenetzung der Meßzone bewirkt (dem rollenden Schwenken des Teststreifens in der Hand bei jetzt üblichem Vorgehen zu vergleichen). Da die Wiederbelüftung schon nach der Blutaufnahme erfolgte, entfällt die stark hemmende Wirkung des Unterdruckes von der Saugglocke her auf den elastischen Ring. Vertikale Absenkung des Schiebestift nach der Messung bringt die Watte mit der Meßzone in Berührung, wobei verstärkte Bewegungsausschläge eine Watteberührung auch im Zentrum der Meßzone zur Folge haben. Nach Reinigung der letzteren erfolgt jetzt die Messung. Die Transportgabel(280) vereinigt nigt sich in eine Hubstange parallel zum Lager für den Schiebestift; auch teleskopartige Anordnung zu unabhängiger Hubbewegung der Abdeckhaube(271) und damit des Griffels als Ganzem und dessen zentraler Partie ist anwendbar. Die Meßstrahlen können direkt aus einer Lichtquelle oder aus Lichtleitfasern und in erforderliche Tiefe der Meßzone zugeführt werden.

Figur 59 gibt im Längsschnitt im Maßstab von etwa 2 : 1 eine anderen "Vibrationskanüle" wieder, wiederum mit sagitaler Schwingungsebene und unter Einsatz einer Lanzette(265), diesmal etwas exzentrisch angeordnet. Der Punktionsgriffel enthält den Kanülenschaftes (2), welcher unter Vermittlung des Kolbenstiftes(289) über den Schiebestift(24) gesenkt ist. Die seitliche Eintrittstelle(6) für Flüssigkeit wird dabei durch Einschnitte in das obere Schaftende in Richtung Dosiervorrichtung für den Arzneiabfluß durch den Schaft(2) freigehalten, wie auf der linken Saugglockenhälfte als Kanalführung zwischen den beiden Dichtungsringen für die Kanüle erkennbar ist. Bei dieser Lösung der Aufgabe ist der Aufnahmebecher(26 becher(268) feststehend, während der zentrale Schiebestift(24) mit der gegen die Meßzone(5) gerichteten Optik in der Frontalebene in Schwingungen versetzt werden kann. Innerhalb der becherförmigen Leiste(237), von welcher aus Öffnungen in den ringförmigen seitlichen tiefliegenden Auffangraum des Aufnahmenbechers vorhanden sind, ist eine Art Wattering-(291) befestigt, dessen der Haut zugewandte Fläche mit einer Folie abgedichtet sein kann. Die napfartig ausgehöhlte Mulde mit der zentralen Meßzone(5) gehört der Trägerhülse(266) an und wird nach oben hin von dem elastischen ringartigen Dichtkissen(298) begrenzt. Letzteres weist unten eine Fenstermembran(299) unter einem zentralen Lichtschacht auf. Der Schiebestift(24) kann (bei Bedarf von den Meßbedingungen her) bis zur Fenstermembran vorgeschoben werden oder diese mag durchbrochen werden bei nicht verschieblichem Dichtkissen, so daß die Optik direkt über der Meßzone liegt. Vorzugsweise ist der Schiebestift an der Durchtrittstelle durch den Aufnahmebecher mit diesem verrastet, um die Kanüle vertikal führen zu können. Um den Schiebestift, der möglichst mit einer scharfen Kante die hochelastische dünne Deckelmembran durchbricht, ist eine Kappenscheibe(301) gedichtet angeordnet. Letzere ist über dem Langloch des Becherauslasses auf einer Gleitschicht (z.B.Parafin) oder bei teflonartig selbstschmierenden Eigenschaften (oder auf Kugeln) mit seinem gewölbten Rand verschieblich (siehe auf dem Querschnitt rechts). Die genannte dünne Deckelmembran weist einen inneren ringfömigen Kleberand(301) über der Kappenscheibe, sowie einen äußeren(300) auf der Oberfläche des Aufnahmebechers auf. Auch bei noch nicht gesenktem Schaft(2) ist der Kolbenstift(289) von der elastischen Deckelmembran überzogen. Letztere überspannt dann -außer der Kappenscheibe- noch wenigstens eine Trichteröffnung für den Sogkanal-(269). Dabei kann die Öffnung der Deckelmembran -bei deren Verklebung der Feststellung von über die Mulde bestimmten Fassungsvermögens um die Meßzone(5) ausgelaufenes Blut dienen. Nach Unterbrechung der Vibration kann (beispielsweise wie zu Fig.5 5beschrieben) der Teilraum um die Lanzette wieder belüftet werden. Der Schaft(2) ragt jedoch in den Teil der Hautkuppe hinab, welcher über einen anderen Sogkanal weiter unterhalten wird. Unbehindert von der Hemmwirkung des Vakuums kann nach der Einwirkungszeit über heftige Vibrationen das Blut über der Meßzone in den Wattering ausgeschüttelt werden. Wenigstens ein Sogkanal (wie in Fig.56) oder eine Wischvorrichtung können außerdem zur Reinigung der Meßfläche vor der Messung herangezogen werden.

Der untere Deckel(390) ist über einen Faden so befestigt, daß nach Deckelabschub, dieser beim Verschluß der Saugglocke durch den Griffel nicht im Wege ist. Die Sogeinleitung kann über einen Kanal im Schiebestift(24) erfolgen: für den Bluteintritt müssen dann Löcher im Boden der Trägerhülse( wie im Querschnitt der Fig 56) vorgesehen sein. Die REaktionskammer soll -soweit die Meßtechnik dies zuläßt- sich möglichst im kapillären Bereich bleiben: jedenfalls dient die Anordnung mit Dichtkissen der Vorbeugung gegen ein Abtropfen des Blutes von der Meßzone vor Ablauf der Reaktionszeit infolge besonderer Gerätestellung-(z.B.Annäherung desselben an den Körper von unten her).

Die Montage der Trägerhülse erfolgt durch Einschub von unten in den Aufnahmebecher, wobei die Noppen (302) des Schwenkgelenkes in entsprechende Gruben innerhalb des Aufnahmezylinders einrasten. Für die Massenproduktion können aber bei Wahl entsprechend elstischen Materials Trägerhülse. und Aufnahmebecher verbunden durch die Bodenmembran von oben entkernt werden; als oberer Abschluß wird dann eine Art Deckelmaske aufgeschweißt. Eine Bodenmembran zwischen der Unterfläche von Aufnahmebecher und Trägerhülse -welche auch mit einer Membran des Watteringes(291) gemeinsame Flächen haben kann- muß die Kippbewegungen der Trägerhülse zulassen. Sie sperrt aber den Bluteintritt an unzweckemäßigen Stellen.

Figur 26 zeigt im schematischen Querschnitt in natürlicher Größe drei elektrisch aufheizbare Heißluftkessel(19) zur Unterdruckspeicherung, welche ein vom Magnetschaltwerk durch Rotation betätigtes Ventil(153) umschließen; letzteres erlaubt die Erneuerung des Unterdruckes innerhalb der Saugglocke bei Bedarf noch während ein und derselben Geräteanwendung. Die Luft aus der Saugglocke erreicht über einen Schlauch, das Rohrsegment(155) des Ventiles(153) über den Kanal(154) den abgekühlten linken oberen Heißluftkessel bei der gezeichneten Ventilstellung.

Figur 27 zeigt im Längsschnitt den unteren Teil des Gehäusekastens(155) eines Gerätes zur Saugdiagnostik über einer halbkugelförmigen Saugglocke (14) aus weich-elastischem Material. D erzur Annäherung an die Haut absenkbare Schiebestift-(24) enthält unten den Punktionsgriffel oder Kanülenkörper(1), welcher mittels des Schiebestiftes abgesenkt und gehoben werden kann. Über den Schaft(2) führt der Sogkanal über eine Bohrung im Schiebestift(24) und eine Schlauchverbindung über den Unterdruck-Meß-und-Regelzylinder-(155) zur Vakuumquelle. Wenn auch der manuelle Andruck dieser Saugglocke gegen die Haut bei vorrübergehender Abplattung der Saugglocke genügt, um eine Hautansaugung zu erzielen, so bietet die Unterdruckkontrolle erst Gewähr dafür, daß die Blutgewinnung vor Ablösung der Saugglocke von der Haut abgeschlossen ist. Mit optischer Blutmengenkontrolle (beispielsweise in Nähe der Meßzone) allein läßt sich die diagnostische Aufgabe ebenfalls lösen.

Die Figur 62 zeigt oben im Längsschnitt unten in einer Draufsicht eine Vorrichtung zur Klemmung der Haut. Die Klemmbacken(310) sind an ihrer Oberfläche rutscharm (oder sogar adhäsiv durch ein quadrati sches doppelseitig wirkendes Pflaster, analog zu Fig. in WO86/01781; die gestrichelte Linien(311) symbolisieren die Zugfedern, welche etwa oberhalb und seitlich der Backen unter Aufspreitzung derselben gespannt und dann durch Lösung des Sperrriegels(312) neben dem Gelenkscharnier ausgelöst werden. Die an der Scharnierachse zwischen den Scharnierbögen(314) befestigte Dachplatte(313) enthält den Durchlaß für die Punktionsgriffel, die Lichtquelle und den Detektor-(48) für die Überprüfung der Haut. Schlauchringe oder Schlauchringsegmente(340) innerhalb der Scharnierbögen können alternierend aufgeblasen werden, so daß Blutgefäße nach oben zur Hautkuppe hin ausgepreßt werden. Die Vakuumpumpe aus Fig.35 oder Fig.36 wird dann als Druckpumpe beispielsweise umfunktioniert. (Das übrige Gerät in Analogie zu den Vorrichtungen für Saugdiagnostik und Sauginjektion wurde weggelassen).

Links in Stadium I ist die Vorrichtung mit den Klemmbacken bei gespannten Zugfedern auf die Haut aufgesetzt, welche kissenartig etwas hochgewölbt wird.

Rechts im Stadium II sind die Zugfedern ausgelöst, die Klemmbacken einander genähert; die Hautfalte wurde angehoben und die Dachplatte-(313) genähert. Nach optischer Überprüfung der Hautfaltenkuppe auf Eignung können optische Stoffwechselmessungen oder auch Punktion oder Injektion vorgenommen werden.

Eine Art von Lamellenverschluß anstelle der Klemmbacken findet sich in WO86/01728 Fig.102, wobei dessen Öffnung (bei Anwendung hier zur Flüssigkeitsdosierung innerhalb eines Schlauches) wesentlich weiter sein muß bei Anwendung um die Haut (auch innerhalb einer Saugglocke). Die Einrichtung zur Reflexphotometrie impliziert geradezu auch deren Anwendung für die Überprüfung der Blutfüllung der Reaktionskammer. Reflexphotometrisch kann aber auch die Eignung der Haut zur Punktion überprüft werden. Lichtquelle und Detektor(48) können beispielsweise( wie dargestellt) vom Saugglockendach her auf die Haut gerichtet sein; aber auch durch die Kanüle hindurch, ja durch die Meßoptik selbst kann diese wichtige Aufgabe gelöst werden, wenn der Trog für die Meßschicht(5) durchsichtig ist und beispielsweise an letzterer vorbei das Licht von der Hautkuppe reflektiert werden kann. Die Erfindung besteht nicht nur aus der beschriebenen Kombination von neuen Einzelheiten, sondern auch deren vielfältig mögliche Kombination.

Überhaupt entspricht die Kombination der dargestellten Prinzipien und Baumerkmale auch mit früher dargestellten der Absicht dieser Erfindung und es sind auch einige leichtere Abwandlungen möglich, ohne die Grundkonzeption dieser Erfindung zu verlassen, welche unter Schutz gestellt werden soll. So ist es zweckmäßig derartige Vorrichtungen nicht nur zur Punktion, sondern auch zur Diagnostik mit einer Registriervorrichtung zu verbinden, welche auch außerhalb der Einrichtung sich befinden kann, so daß im Zuge der Batterieaufladung über die Ruhezeiten(z.B.nachts) die ermittelten Stoffwechselwerte und der Zeitpunkt ihrer Ermittlung übersichtlich und dauerhaft festgehalten werden.

Die bei den nachstehenden Ansprüchen angeführten Figuren sollen nur als das Verständnis erleichternde Hinweise verstanden werden und der Bezugnahme auf analoge Beispiele nicht einengend im Wege stehen. Ausdrücke, wie "Saugglocke", "Kanüle" etc. stehen häufig auch für analoge Mittel, wie "Vorrichtung zur Hautkuppenbildung", "Punktionsgriffel" etc..Blut immer auch für Gewebsflüssigkeit.

Als besondere Kombinationen mit bereits Bekanntem werden noch nachstehende Beispiele gegeben:

Figur 66 zeigt in natürlicher Größe einen schematischen Längsschnitt durch eine Hautkuppe-(strichpunktierte Kurve) innerhalb einer Saugglocke(14). Durch Höhenbegrenzung der Bewegung der Hautglocke in der Saugglocke (durch deren Gestaltung) ist der (gestrichelt dargestellte) Meßstrahl zwischen Lichtquelle(7) und -empfänger-(8) festgelegt. Eine Meßserie wird bei Erreichen der Lichtschranke durch die Hautkuppe durch Exstinktionsanstieg der Messung ausgelöst(I). Die Meßserie endet mit der dargestellten Messung (II). Die Exstinktionswerte der Messung(II) werden -sofern keine zu starke Abweichung von früheren Messungen (II), die gespeichert wurden, eintritt, den Meßwertvergleichen zugrunde gelegt. Erfolgt zwischen den Messungen(I) und (II) ein unverhältnismässiger Exstinktionsanstieg (ein leichter Kurvengipfel wird bei tangentialem Hautanschnitt erzeugt), so wird die Saugglocke ohne Absenkung der Kanüle wiederbelüftet. Pigmentgeschwülste, Warzen und Grützbeutel auf und in der Haut beispielsweise bewirken einen solchen unverhältnismäßigen Exstinktionsanstieg aber auch Venenansammlungen unter der Haut. Damit ist der Sicherungsvorgang gegenüber unbeabsichtigter Venenpunktion - wichtig besonders für die Injektionslöst(I). Die Meßserie endet mit der dargestellten Messung (II). Die Exstinktionswerte der Messung(II) werden -sofern keine zu starke Abweichung von früheren Messungen (II), die gespeichert wurden, eintritt, den Meßwertvergleichen zugrunde gelegt. Erfolgt zwischen den Messungen(I) und (II) ein unverhältnismässiger Exstinktionsanstieg (ein leichter Kurvengipfel wird bei tangentialem Hautanschnitt erzeugt), so wird die Saugglocke ohne Absenkung der Kanüle wiederbelüftet. Pigmentgeschwülste, Warzen und Grützbeutel auf und in der Haut beispielsweise bewirken einen solchen unverhältnismäßigen Exstinktionsanstieg aber auch Venenamsammlungen unter der Haut. Damit ist der Sicherungsvorgang gegenüber unbeabsichtigtem Ansicht von krankhafter Haut vereinfacht. Auch Meßwertvergleiche mit früheren, in der Kontrollvorrichtung gespeicherten, selbst über eine einzige Meßstelle können (auch gerade zum Ausschluß mehr flächiger Hautveränderungen von Nutzen sein. Im Rechner müssen die -eventuell auch den Erfahrungen im Einzelfall angegelichenen-Toleranzgrenzen ermittelt werden, bei deren Überschreitung eine Punktion unterbleibt. Der Benutzer muß diese Art von Kontrolle (beispielsweise nach Sonnenbrand) außer Kraft setzen können.

Figur 67 zeigt eine durch Zug angehobene Hautkuppe (vereinfacht mit der Hand bewerkstelligt), wobei ein Klebepflaster(495) sowohl die Verbindung mit der Haut als auch mit einem Überdruckinjektor herstellt. Eine wenigstens innen klebende Pflastertülle(496), deren Boden auch verdickt sein kann, umgibt den Spritzenzylinder auch noch nach oben hin und kann durch den Klemmring(497) festgedrückt werden. Durch die (nicht dargestellte) zentrale Düse kann nicht nur der Flüssigkeitsstrahl gegen die Haut gerichtet werden, sondern auch ein zentraler Lichtstrahl, welcher innerhalb der Spritzenkolbenstange geleitet (gestrichelte Linie) wird und nach Reflexion den Detektor(48) erreicht.

Die nachfolgenden Ansprüche stützen sich auf die bereits entwickelten "Wesensmerkmale", auf welche die arabischen Ziffern in Klammern verweisen, um das Verständnis in Zweifelsfällen zu erleichtern.

Wesenmerkmale der Erfindung

1. Einrichtung zur Stoffwechselkontrolle bei Mensch oder Tier mit während direkten Hautkontaktes wenigstens eingeleiteter Messung, welche auch -wenn beabsichtigt- mit einer Einspritzung verbunden sein kann,
**bestehend aus**
einem Gehäuse(155) mit wenigstens einer Steuervorrichtung(Fig.35A(560) und wenigstens einer Schaltvorrichtung(Fig.37), von einer Gestaltung-(Fig.36(670?, welche die sichere Auflage der Einrichtung während der notwendigen Dauer des Hautkontaktes gewährleistet und einer Punktionsvorrichtung(Fig.47;Fig.18;Fig.59) für die Aufnahme von Blutbestandteilen oder Gewebsflüssigkeit und eine Vorrichtung für deren Ableitung in eine Reaktionskammer(Fig.4(11); Fig.41(3),
bestehend weiterhin aus einem dort wirkenden Signalgeber und Signalempfänger(Fig.42(18), vorzugsweise als Strahlenquelle(7) und -detektor(8) eines Reflexphotometers(18) zur Signalverarbeitung (Fig.54(18), um Meßwerte über die zu ermittelnde Substanz zu erstellen und wenigstens eine Anzeigevorrichtung, die ermittelten Meßwerte erkennbar zu machen und in der Regel einer Registriervorrichtung(Fig.54(668) für die Meßwerte und andere Daten, wobei die signalverarbeitende Vorrichtung und die Registriervorrichtung wenigstens teilweise auf weitere Gehäuse verteilt sein können, bei wenigstens zeitweiser wenigstens funktioneller Koppelung derselben,
und bevorzugterweise aus einer Vorrichtung zur Kontrolle (Fig.47(7-8) ausreichender Füllung der Reaktionskammer, und wobei auch eine Vorrichtung zur Kontrolle der Eignung der Hautfalte zur Punktion vorhanden sein kann(Fig.59(7-8)(48), falls zusätzlich beabsichtigt verbunden mit Vorrichtung zur Einspritzung(Fig.36(592)(599);Fig.42 (643) von Flüssigkeit unter die Haut über wenigstens eine Dosiervorrichtung(Fig.36(599) bei Lagerung der Flüssigkeit in wenigstens zwei gleichartigen Behältern(Fig.36(701,702) je Flüssigkeitssorte.

2. Einrichtung zur Stoffwechselkontrolle bei Mensch oder Tier mit während direkten Hautkontaktes wenigstens eingeleiteter Messung, welche auch -wenn beabsichtigt- mit einer Einspritzung verbunden sein kann, **bestehend aus** einer Vorrichtung zur Anhebung einer Hautfalte (Fig.24-(71,72);Fig.27(14);Fig.35(14,562); Fig. 36 (14,590)-;Fig.61(310,311) und deren Fixierung im Zusammenhang mit einem Gehäuse mit einer Gestaltung-(Fig.36(570,636), welche die sichere Befestigung jener Vorrichtung zur Erzeugung einer Hautfalte während der notwendigen Dauer des Hautkontaktes gewährleistet, und einer Vorrichtung (Fig.33(264)-;Fig.34), welche gewährleistet, daß Laserlicht tangential durch die Hautfalte hindurch einem Detektor zur Vermittlung von Meßsignalen für die Bestimmung des Gehaltes der zu ermittelnden Substanz zugeleitet wird, wobei die Streckenlänge der Meßstrahlen innerhalb der Haut weitgehend durch tangentiale Strahlenführung zur in ihrer Krümmung am Strahlendurchtrittsort festgelegter Hautfalte weitgehend vorbestimmt ist, wobei der Photometer(Fig. 54 (18)und eine Registriervorrichtung(Fig.54(468) auf weitere Gehäuse, bei wenigsten zeitweise wenigstens funktioneller Koppelung, verteilt sein können, falls zusätzlich beabsichtigt verbunden mit einer Vorrichtung zur Einspritzung/Fig.36(599)-;Fig.42 (643) von Flüssigkeit unter die Haut über wenigstens eine Dosiervorrichtung(Fig.36(599) bei Lagerung der Flüssigkeit in wenigstens zwei gleichartigen Behältern(Fig.36 (701,702) je Flüssigkeitssorte.

3. (nach Anspruch 1 oder 2),
daß eine Art Klemmrahmen oder Klemmbacken vorhanden sind, welche die Haut an der Basis einer Hautkuppe, diese eventuell erst hervorbringend, von den Rändern her nähert(Fig.61).

4. (nach Anspruch 1)
daß eine Zugvorrichtung(Fig.24(71)(72);Fig.35(14)(590) zur Anhebung der Haut in Form einer Hautkuppe vorhanden ist.

5. (nach Anspruch 1, 2 und 4)
daß als Zugvorrichtung eine Saugglocke(Fig.5; Fig.27; Fig.36(14), in welcher Luftunterdruck wirksam wird, vorhanden ist.

6. (nach Anspruch 1 bis 5),
daß Vorrichtungen zur Unterdruckregulierung (Fig.25(78);Fig.36(591)(588) vorhanden sind, vorzugsweise aus Unterdruckprüfer und Verteilerventil-(Fig.36) bestehend und durch die Schaltvorrichtung(Fig.37) betätigt, wobei auch eine optische Kontrollvorrichtung(Fig.36(7-8) zur Überwachung der Folgen der Sogeinwirkung auf die Haut vorhanden sein kann.

7. (nach Anspruch 1 oder 2 und 5)
daß eine Warnvorrichtung bei absinkendem Unterdruck innerhalb der Saugglocke betätigt wird und mittels einer Handpumpe der erforderliche Unterdruck wieder hergestellt werden kann(Fig.35).

8. (nach Anspruch 1 oder 2 und 5)
daß mehrere Unterdruckspeicherräume vorhanden sind, welche bei Bedarf mittels wenigstens eines Ventiles (553) mit der Saugglocke in Verbindung gesetzt werden können(Fig.26).

9. (nach Anspruch 1 oder 2 und 5)
daß bei abfallendem Unterdruck .innerhalb der Saugglocke eine aus einem Kraftspeicher betriebene Vakuumpumpe betätigt wird(Fig.36).

10. (nach Anspruch 1 oder 2 und 4)
daß die Veränderung der Zugeinwirkung auf die Haut durch die Veränderung der Vorspannung einer Feder (72) bewirkt wird, welche die Zugwirkung hervorbringt(Fig.24).

11. (nach Anspruch 1 oder 2 und 5)
daß mit einer Saugglocke ein Druckregelventil(76) in Verbindung steht, welches sich öffnet, wenn der Luftdruck in der Saugglocke niedriger liegt als festgesetzt wurde(Fig.25).

12. (nach Anspruch 1 oder 2)
daß zur Regelung der Öffnung einer Gasdruckkapsel ein Ventilstift eingesetzt wird, dessen Entfernung zur Ventilbohrung über ein Schraubengewinde geregelt wird unter Verwendung eines Schwenkhebels quer zur Achse des Ventilstiftes und an diesem befestigt, welcher mittels wenigstens eines unter diesen vorbeibewegten Schiebers nacheinander in gegenläufige Drehbewegungen versetzt wird(Fig.60).

13. (nach Anspruch 1 und 2)
daß die Ebene (Fig.24(68);Fig.36(40)und(536) des zeitweisen Hautkontaktes mit der größten Ausdehnungslänge(Fig.9;10;35;36) des Gehäuses (Fig.35(559) parallel verläuft.

14. (nach Anspruch 1 oder 2)
daß die Funktionslelemente innerhalb eines teilweise wandbeweglichen Gehäuses so angeordnet sind, daß bei Abbiegung des Gehäuses im Umfang der vorgegebenen Beweglichkeit, das Zusammenspiel der Funktionen nicht gestört wird(Fig.9)

15. (nach Einrichtung 1 oder 2)
daß die Saugglocke innerhalb einer Längsmulde der Bodenfläche der Einrichtung angeordnet ist-(Fig.35,36).

16. (nach Anspruch 1 oder 2)
daß die Saugglocke mittels einer Art von Balancier-Federn(580) gegen die Haut gedrückt wird.während die Aufstützung(Fig.36(236) der Einrichtung auf die Haut weiter seitlich vom Saugglockenrand erfolgt.

17. (nach Anspruch 1 oder 2 und 16)
daß wenigstens zwei Federn, bevorzugterweise Druckfedern, so winkelverschieden zu einer Saugglocke angeordnet sind, daß sie nach einander in verschiedener und wechselnder Stärke in einem Zusammenwirken gemäß eines Kräfteparallelogrammes der Anhebung der Saugglocke entgegenwirken, um deren Hautandruck zu bewirken(Fig.59).

18. (nach Anspruch 1 oder 2)
daß um die Hautkuppe ringförmig wenigstens eine Art von elastischem Schlauchring, möglicherweise auch segmentartig unterbrochen, vorhanden ist, welcher durch Veränderung seines Füllungszustandes zeitweise Druck auf die anliegende Haut ausübt(Fig.40;Fig.61).

19. (nach Anspruch 1 oder 2 und 5)
daß bei glatter Beschaffenheit des Saugglockenrandes(40) ein weiterer Dichtring(633) sich innerhalb der Saugglocke befindet, der auch nach oben hin leicht elastisch beweglich sein kann-(Fig.42).

20. (nach Anspruch 1 oder 2)
daß zur Verschiebung des Punktionsgriffels wenigstens in der Senkrechten Bowdenzüge die Verbindung zur Schaltvorrichtung herstellen.

21. (nach Anspruch 1 oder 2 und 5)
daß das Schieberventil zur Verbindung der Kanalausgänge einer doppelten Zylinderpumpe mit der Druckgaspatrone einerseits und der Saugglocke andererseits auch dazu benutzt wird, Druckgas in den Dichtraum um die Flüssigkeitsbehälter einzuleiten (Fig.36B).

22. (nach Anspruch 1 oder 2)
daß insbesondere auch für den Injektionsgebrauch eine Art elektromagnetische Schaltvorrichtung vorhanden ist, welche durch Beeinflussung der Funktionsweise eines elektrischen Kraftantriebes durch Art und Weise der Stromzufuhr in wenigstens zwei Arbeitsfunktionen eingesetzt werden kann-(Fig.21;Fig.37)

23. Einrichtung nach Anspruch 1 oder 2 und 30,
dadurch gekennzeichnet,
daß als Kraftantrieb ein Elektromotor dient, dessen Drehung nur in einer Richtung wenigstens eine Arbeitswelle antreibt, während durch Wechsel der Laufrichtung die sonst gesperrte Sektordrehung wenigstens einer Schaltscheibe bewirkt wird, wobei letztere die Bewegungsübertragung auf je andere Arbeitswellen nach erneuter Umkehr der Drehrichtung des Elektromotors bewirkt (Fig.20;Fig.37)

24. Einrichtung nach Anspruch 1 oder 2 und 31,
dadurch gekennzeichnet,
daß nach Änderung der Laufrichtung des Kraftantriebes aus der Arbeitsfunktion zur Schaltfunktion durch Herstellung der Drehachsenkoppelung mit einem Ritzel in der Anfangsphase dieser Laufrichtungsänderung, bei nochmaligem Wechsel der Laufrichtung über die Mitnahme des Ritzels nur in dieser Arbeitslaufrichtung von diesem Ritzel aus eine Verstellung der Höhe des Arbeitshubes je nach Größe der Sektorbewegung des Ritzels und die Rückführung dieser Hubhöhenverstellung in die Ausgangsstellung am Ende einer vollen Ritzeldrehung bewirkt wird, während das Ritzel bei Fortsetzung des Kraftantriebes in der Schaltlaufrichtung das Ritzel wieder aus der Drehachsenkoppelung gelöst wird, was auch der Fall ist, wenn innerhalb der Umschaltdrehung für die Funktionswahl keine Unterbrechung durch Richtungsänderung der Drehung bewirkt wurde(Fig.20).

25. (nach Anspruch 1 oder 2 und 22)
daß als elektrischer Kraftantrieb ein Hubmagnet verwendet wird, wobei eine Höhendifferenz der Ankerbewegung zur Unterscheidung zwischen Arbeits- und Schalthub benutzt wird(Fig.20).

26. (nach Anspruch 1 oder 2 und 22)
daß durch die Hubbewegung des Magnetankers, neben der Übertragung des Arbeitshubes durch einen von der Schaltscheibe ausgehenden Stößel in eine von mehreren Bohrungen mit den Kraftübertragungsdrähten für die Arbeitsfunktion innerhalb einer feststehenden Trommel, über eine Kulissenführung die Sektorbewegung einer Pendelhülse bewirkt wird, welche Pendelhülse über eine über diese Sektorbewegung gespannte Feder mit der Schalthülse als Teil der Schaltscheibe in Verbindung steht, wobei die Federspannung durch eine Sperrasterverbindung zwischen Pendelhülse und Schaltscheibe erhalten bleibt, welche Sperrverbindung das Zurückpendeln der Pendelhülse ohne Schaltwirkung verhindert, so daß die gespannte Feder die Schaltscheibe in entgegengesetzter Richtung um eine Schaltsektorstufe dreht, sobald der von der Schaltscheibe ausgehende Stößel und ein in Bohrungen ohne Antriebsfunktion etwa zusätzlich bewegter Stift die feststehende Trommel verlassen hat, welches Verlassen durch vorzeitige Wiedererregung der Ankerspule solange verhindert wird, als noch Arbeitshübe in gleicher Schaltstellung der Schaltscheibe erfolgen sollen.(Fig.21)

27. (nach Anspruch 1 oder 2 und 22)
daß als elektrischer Kraftantrieb ein Hubmagnet verwendet wird und der Unterschied in der Geschwindigkeit der Ankerbewegung zur Unterscheidung zwischen Arbeits- und Schalthub benutzt wird(Fig.22).

28. (nach Anspruch 1 oder 2 und 22)
daß als elektrischer Antrieb ein Hubmagnet verwendet wird, wobei die zeitliche Verteilung von Ankerspulenerregungen zur Unterscheidung zwischen Arbeits- und Schalthub herangezogen wird(Fig.22).

29. (nach Anspruch 1 oder 2 und 22)
daß als elektrischer Antrieb ein Hubmagnet verwendet und durch diesen eine Schwungscheibe in Drehung versetzt wird, wobei das Ausmaß der Sektorbewegung dieser Schwungscheibe zur Unterscheidung zwischen Arbeits- und Schalthub benutzt wird(Fig.22).

30. (nach Anspruch 1 oder 2 und 22)
daß unter der Hubwirkung eines Arbeitshebels eine einer Scheibe benachbarte Feder unter Drehung der Scheibe gespannt wird, wobei diese Feder die zur Übertragung des Arbeitshubes dienende genannte Scheibe erst dann in der Drehachsenrichtung bewegen kann, wenn die Scheibe eine Drehstellung erreicht hat, bei welcher die Sperrwirkung eines achsenqueren Stiftes durch einen Schlitz aufgehoben wird(Fig.18;Fig.19).

31. (nach Anspruch 1 oder 2)
daß wenigstens ein Permanentmagnet vorhanden ist, um bei einem Hubantrieb die Endlagen der Hubbewegung zu stabilisieren(Fig.18;Fig.19).

30. (nach Anspruch 1 und 2)
daß die Punktionsgriffel oder Kanülen(Fig.1(1); Fig.4(1) zur Flüssigkeitsabnahme aus der Haut mittels eines Schiebestiftes (Fig.7(24); Fig.35(24)(571) in der Senkrechten angenähert und später wieder entfernt werden.

33. (nach Anspruch 1 oder 2)
daß der Körper des Punktionsmittels oben eine Bohrung zur Aufnahme eines Schiebestiftes, welcher der Beförderung in der Senkrechten oder der Messung durch Durchlaß der Meßstrahlen dient, aufweist(Fig.7).

34. (nach Anspruch 1 oder 2 und 30)
daß der Schiebestift eine Kappe aufweist, welche wenigstens Teile des Punktionsgriffels oder der Kanüle zum Transport umfaßt(Fig.10).

35. (nach Anspruch 1 oder 2)
daß der Punktionsgriffel oder die Kanüle in einer Hülse gelagert werden, welche vor Gebrauch beidseits luftdicht verschlossen ist(Fig.7).

36. (nach Anspruch 1 und 2)
daß mehrere Hülse oder Punktionsgriffel ohne Hülsen oder Kanülen nebeneinander als Kette gelagert werden(Fig.6;Fig.35(952);Fig.57).

37. (nach Anspruch 1 oder 2 und 34)
daß die Hülsen oder Punktionsgriffel oder Kanülen mittels wenigstens eines Fadens miteinander verbunden sind, wobei der Faden elastisch sein kann(Fig.35).

38. (nach Anspruch 1 oder 2 und 34)
daß zwischen den Hülsen oder Punktionsgriffeln oder Kanülen eine Art Gelenkverbindung bei Ermöglichung einer gegenseitigen Höhenverschiebung besteht (Fig.52).

39. (nach Anspruch 1 oder 2)
daß der Körper des Punkgstionsgriffels wenigstens eine zumindest teilweise Profilgestaltung aufweist, welche der Beförderung längs einer Schiene oder der Stellungssicherung gegenüber einer optischen Vorrichtung dienen kann(Fig.1;Fig12).

40. (nach Anspruch 1 oder 2)
daß die Saugglocke bei Anhebung mittels eines Stiftes, welcher auf eine Kulissenführung am Rand eines Transportrades einwirkt, die Seitwärtsbewegung der Punktionsmittel bewirkt(Fig.10).

41. (nach Anspruch 1 oder 2)
daß bei der Anhebung der Saugglocke bestimmungsgemäß unter Druckwirkung gegen die Haut mindestens eine Feder gespannt wird, welche zur Ausführung weiterer Gerätefunktionen benutzt wird(Fig.10).

42. (nach Anspruch 1 oder 2)
daß durch Anhebung der Saugglocke diese dem Punktionsmittel bis zur Abdichtung der Öffnung der Saugglocke durch das Punktionsmittel genähert wird(Fig.10).

43. (nach Anspruch 1 oder 2)
daß mehrere Federn an getrennten Schiebern hintereinander gestaffelt so angeordnet sind, daß sie durch die Bewegung eines Bügels nacheinander gespannt werden, wobei die einzelnen Schieber durch Raster festgestellt und vom Bügel überholt werden (Fig.16).

44. (nach Anspruch 1 oder 2)
daß beide Federendpunkte durch Raster festgestellt werden, von denen der eine zur Verrichtung der Arbeitsfunktion, der andere nach Verrichtung derselben ausgelöst wird(Fig.16).

45. (nach Anspruch 1 oder 2)
daß die Auslösung des Rasters nach Verrichtung der Arbeitsfunktion über die Bewegung des entsprechenden Schiebers selbst bewirkt wird(Fig.16).

46. (nach 1 oder 2)
daß meistens ein Teil der Transportmittel für die Positionierung der Punktionsmittel mit der Saugglocke verbunden ist und bei deren Anhebung mitangehoben werden(Fig.10;Fig.35).

47. (nach 1 oder 2)
daß der Transport der Punktionsgriffel von und zur Öffnung in die Saugglocke mittels wenigstens zweier Keilschieber(Fig.57(283)(282) erfolgt, wobei Keilschieber auch der Entfernung von wenigstens einem Deckel über Funktionsöffnungen im Punktionsgriffel dienen können(Fig.58)

48. (nach 1 oder 2 und 5)
daß innerhalb der Saugglocke eine Art Schale mit zentraler Öffnung zur Aufnahme der Hautkuppe vorhanden ist, welchem das Punktionsmittel angenähert wird(Fig.14).

49. (nach 1 oder 2 und 48)
daß jene Art Schale durch Magnetkraft bis zur Unterdruckansammlung vom Punktionsmittel in Abstand gehalten wird(Fig.14).

50. (nach 1)
daß wenigstens ein in die Haut eingedrungener schneidender Teil(Fig.18(236);Fig.19(259);Fig.50; Fig.59(265) des Schaft(2) des Punktionsgriffels oder der Kanüle zur Ableitung von Gewebsflüssigkeit oder Blut aus der Haut innerhalb derselben und auch mit wenigstens einem schneidenden Teil quer zu dieser bewegt wird, um Blutgefäße zu zerstören.

51. (nach 1) daß die Kanüle wenigstens eine den Schaftdurchmesser überragende Schneidkante aufweist, um die Eröffnung von Blutkapillaren zu befördern (Fig.30;Fig.52).

52. (nach 1) daß der Punktionsgriffel oder der Kanülenansatz als Ganzes oder in Teilen von außen her in Rotation gesetzt wird(Fig.46).

53. (nach 1 und ) daß die Rotation durch die Einwirkung von Mitteln, welche durch eine Dichtung im Saugglockendach hindurch innerhalb der Saugglocke wirken, erfolgt (Fig.39(639);Fig.42(645)).

54. (nach 1) daß die Rotation über die Einwirkung einer Spiralführung in dem den Punktionsgriffel oder die Kanüle umgebenden Führungsrohr zur Saugglocke hin durch gegenseitige relative Streckenverschiebung erfolgt(Fig.28).

55. (wie 1 oder 2 und 50) daß wenigstens eine Art Lanzette als schneidender Teil einer Punktionsvorrichtung verwendet wird (Fig.55;Fig.56).

56. (nach 1 und 50) daß eine Art Aufnahmeteil und eine Art Aufnahmebecher und eine Art Trägerhülse für den schneidenden Teil funktionell unterschieden werden können, indem beide Teile gegeneinander in wenigsten einer Seitwärtsrichtung quer zur Haut bewegt werden können(Fig.58;Fig.59)

57. (nach 1) daß eine Schneidfläche des Punktionsgriffels exzentrisch angeordnet ist(Fig.59).

58. (nach 1) daß im Bereich des Kanülenanschliffes dessen Öffnung gegenüber der Schaft(2) mindestens ein Loch(541) aufweist zur Aufnahme von Blutbestandteilen(Fig.41;Fig.50).

59. (nach 1 und 58) daß von dem Loch(541) ein Kanal zur Aufnahme von Blutbestandteilen im Schaft sich fortsetzt, während die Kanülenanschliföffnung verschlossen sein kann(Fig.41).

60. (nach 1) daß ein Kanülenschaft einen mindestens doppelten Anschliff aufweist, so daß sie nur gemäß der Länge der Zackentiefe in die Haut eindringen kann (Fig.38).

61. (nach 1) daß neben dem schneidenden Teil eines Punktionsgriffels mindestens eine Öffnung zur Aufnahme von Blutbestandteilen sich befindet (Fig.55;Fig.59).

62. (nach 1)

daß hinter der Kanülenspitze ein Teller sich befindet, welcher die Lage der Kanülenspitze innerhalb der Haut während der Blutaufnahme sichert (Fig.32).

63. (nach 1 und 108) daß dieser Teller über den Kanülenschaft hoch geschoben werden kann, um nach der Stoffwechselmessung eine Einspritzung unter die Haut zu ermöglichen(Fig.32).

64. (nach 1) daß innerhalb des in die Haut eingeführten Schaftes(2) eine Art innere Kanüle(553) liegt, welche zur Injektion unter die Haut gesenkt werden kann(Fig.45).

65. (nach 1) daß von innerhalb eines Schaftes(2) durch das Loch(541) im Schaft eine Art Draht(465) oder Kapillare quer zur Hautoberfläche in dieselbe eingeschoben werden kann(Fig.50).

66. (nach 1) daß eine seitliche Ableitungsöffnung, welche in die Reaktionskammer(11) führt, vor der Einspritzung durch ein Schläuchchen(566) verlegt wird, welches zumindest anfangs vorn verengt vom Flüssigkeitsstrom zur Einspritzung abwärts getrieben wird (Fig.51).

67. (nach 1 und 5) daß der Kanülenkörper saugglockenwärts eine Dichtungstülle(538) zur Aufnahme von Teilen der Hautkuppe aufweist(Fig.49).

68. (nach. 1)

daß der Kanülenkörper(1) saugglockenwärts eine Mulde(263) um oder neben dem Schaft(2) aufweist zur Aufnahme von um den Schaft austretenden Blutbestandteilen(Fig.49).

69. (nach 1 oder 2) daß Punktionsgriffel oder Kanülenkörper außen einen Vorsprung aufweisen, welcher deren Tiefstand zur Hautkuppe auch unter Unterdruckeinwirkung von dort festlegen(Fig.52).

70. (nach 1) daß eine Kanülenschaftbasis wenigstens eine Öffnung aufweist(Fig.3).

71. (nach 1) daß wenigstens ein Teile einer Punktionskanüle aus durchsichtigem Material besteht(Fig.47).

72. (nach 1) daß wenigstens ein Teil der Kanülenoberfläche eine gewindeartige eingeschnittene Oberfläche aufweist (Fig.41).

73. (nach 1) daß die Meßzone(5) auf der der Haut zugewandten Unter seite des Punktionsgriffels angeordnet ist in der Nach barschaft des schneidenden Teiles(Fig.55).

74. (nach 1) daß wenigstens eine Reaktionskammer(11) für Blutbestandteile außerhalb des Schaftes(2) vorhanden ist, in welchen die Blutbestandteile infolge des Hautkontaktes des Schaftes oder der Hautnähe einer Bohrung des Punktionsgriffels unter Wanderung verbracht werden(Fig.4;Fig 59).

75. (nach 1 und 74) daß zur Beförderung der Wanderung der Blutbestandteile wenigstens ein kapilläres Leitelement(3) in Fortsetzung des Schaftes vorhanden ist(Fig.2).

76. (nach 1 und 74) daß derartige kapilläre Leitelemente teilweise innerhalb eines Kanülenschaftes liegen (Fig.2;Fig.3).

77. (nach 1 und 74) daß derartige kapilläre Leitelemente beweglich angeordnet sind(Fig.2;Fig.5).

78. (nach 1 und 74

daß solche kapillären Leitelemente an einen Schaft anschließen(Fig.1;Fig.4).

79. (nach 1 und 74)

daß solche kapillären Leitelemente in einer Art Nische einer Schaftbasis enden(Fig.4).

80. (nach 1) daß der Schaft(2) ein Lücke aufweist, durch welche zeitweise von außen ein Schlauch eingeführt ist(Fig.44).

81. (nach 1) daß hinter der Anschliffzone eines Kanülenschaftes eine Hohlraumverbindung(Fig.3(12), Fig.7(24);Fig.47) zu einer Unterdruckquelle (Fig.27(14);Fig.26(19);Fig.35(565) vorhanden ist, um den Einstrom von Gewebsflüssigkeit oder Blut in eine Reaktionskammer(Fig.4(11) oder auf ˙eine Meßzone(Fig.1(5) zu begünstigen.

82. (nach 1 und 81) daß sich hinter der Reaktionskammer(11) ein Druckausgleichskanal(12) befindet, welcher mit dem Saugglockenraum in Verbindung steht, um den Bluttansport zu fördern(Fig.4;Fig.7).

83. (nach 1) daß die Reaktionskammer(11) mit einer Sogquelle in Verbindung gesetzt werden kann und zwar abseitig ˙von Öffnungen zur Flüssigkeits-oder Blutaufnahme(Fig.7;Fig.30;Fig.49).

84. (nach 1) daß ein Sog-oder Belüftungskanal(12) nur zeitweise mit einer Sogquelle, die auch ein Speicher sein kann, in Verbindung gebracht wird(Fig.7;Fig.29).

85. (nach 1) daß der Wiederbelüftungskanal der Saugglocke mit der Reaktionskammer verbunden ist, so daß dieselbe von Blutbestandteilen durch den Luftstrom gereinigt wird(Fig.7).

86. (nach 1) daß die in einer Reaktionskammer oder an kapilläre Leitelemente gebundenen Blutbestandteile aus dem Schaftträger oder Kanülenkörper entfernt und einer Messung außerhalb zugeführt werden(Fig.2).

87. (nach 1) daß innerhalb einer Reaktionskammer(11) wenigstens eine als Ultrafilter für feste Blutbestandteile wirkende Membran als Leitelement(3) angeordnet ist(Fig.48).

88. (nach 1) daß zur Messung von elektrischen Stromveränderungen geeignete Chemikalien,-auch in Fortsetzung eines Schaftes und dann mit Hüllschichten-im Kanülenkörper(1) angeordnet sind(Fig.8).

89. (nach 1) daß die Bohrung für den Durchtritt des senkrecht verschieblichen Schaftes durch den Körper(1) des Punktionsgriffels eine Dichtung(Fig.12(153); Fig.52(572)aufweist.

90. (nach 1 und 89) daß eine Dichtungstülle oberhalb des Körpers(1), zu diesem gedichtet, vorhanden ist, welche an einem Kanülenschaft befestigt ist(Fig.52)

91. (nach 1 und 89) daß diese Dichtung eine reckbare Membran ist, welche sowohl mit dem Körper als auch mit dem Schaft gedichtet fest verbunden ist(Fig.52).

92. (nach 1 und 89) daß dies Dichtung ein Faltenbalg ist welcher als Kappe dem Körper des Punktionsgriffels, zu Schaft und Körper gedichtet, aufsitzt(Fig.52).

93. (nach 1) daß der gesammte Schaft des Punktionsmittels vor der Benutzung in einer Bohrung des Körpers des Punktionsmittels zurückgezogen liegt bei zeitweisem Verschluß der Austrittsöffnung des Schaftes zur Hautkuppe hin(Fig.12).

94. (nach 1) daß hinter einer Anschliffzone eine Abwischzone und dann eine Akzeptorzone, letztere zur Aufnahme von Blutbestandteilen, liegen, wobei die Meßzone(5) mit den Indikatorsubstanzen im Körper des Punktionsmittels vor Benutzung oberhalb der in die Haut eintauchenden Schaftabschnitte gelagert ist und eine Wisch- oder Saugeinlage an der Meßzone vorbeibewegt wird(Fig.12).

95. (nach 1)

daß ein Punktionsgriffel im Anschluß an den in die Haut eindringenden Teil einer Kanüle sich in einen Kanal fortsetzt, dessen Fassungsvermögen für Blut nach oben hin durch eine Kalibererweiterung der Bohrung festgelegt ist(Fig.29).

96. (nach 1) daß oberhalb der Punktionskanüle im Punktionsgriffel sich eine Reaktionskammer befindet, welcher zu einer optischen Meßbelagsschicht oder Meßzone(5) durch Abplattung einen kapillären Spalt bildet, in

34

welchen das Blut über die Punktionskanüle hochgesaugt wird, und von welchem das Blut durch Drehung der Reaktionskammer, nach festgelegter Einwirkungszeit, entfernt wird, wobei eine besondere Wischzone in Nachbarschaft der Reaktionskammer unterstützend wirken kann (Fig.28).

97. (nach 1)
daß die Drehung eines Binnenzylinders über diejenige eines Hüllzylinders erfolgt, welcher zum Kanülenträger beweglich angeordnet ist (Fig.28).

98. (nach 1)
daß die Drehbewegung über eine Führungsnoppe in einer Spiralnut unter Verschiebung des Hüllzylinders auf dem Kanülenträger erfolgt(Fig.28).

daß ein Vakuumreserveraum im Inneren des Punktionsgriffels oder des Kanülenkörpers sich befindet, wobei eine Ventilvorrichtung zur Unterbrechung der Verbindung zu der Bohrung der Kanüle vorhanden ist(Fig.30).

100. (nach 1)
daß die Ventilvorrichtung aus einem Rohr im Hüllzylinder besteht, dessen Ende bei Absenkung den trichterförmigen Kalibersprung oberhalb der Kanüle verschließt und welcher nur zur Seite hin wenigstens eine Öffnung aufweist, durch welche die Bohrung der Kanüle mit dem Vakuumreserveraum in Verbindung steht, sobald Rohrende und Ventilsitz von einander entfernt sind(Fig.30).

101. (nach 1)
daß der Vakuumreserveraum aus dem Raum zwischen Dichtungsringen des Führungsrohres für den Punktionsgriffel oder den Kanülenkörper, nach innen von letzteren begrenzt, gebildet wird und die Ventilsteuerung des Kanals zur Bohrung der Kanüle hin durch Höhenverschiebung des Punktionsgriffels oder Kanülenkörpers bewerkstelligt wird(Fig.29).

102. (nach Anspruch 1)
daß die Meßzone(5) senkrecht und vorzugsweise zentral in einer Bohrung des Punktionsgriffel angeordnet ist innerhalb einer Art Sackloch(Fig.50;Fig.59).

103.(nach Anspruch 1)
daß eine Art Dichtungspfropf wenigstens eine Bohrung aufweist, welche auch durch eine durchscheinende Membran verschlossen sein kann, und sich oberhalb der Meßzone befindet(Fig.58; Fig.59).

104. (nach Anspruch 1 und 5)
daß um den in eine Bohrung innerhalb des Punktionsgriffels eintretende Führungsstift(42) von einer Dichtkappe umgeben ist, welche auf einem deckelartigen oberen Abschluß des Punktionsgriffels eine größere Bohrung in diesem oberen Abschluß für den Durchtritt des Führungsstiftes verschieblich und wenigstens zeitweise dichtend überdeckt, wobei für die Dichtkappe ein besonderes Gleitlager vorgesehen sein kann(Fig.59).

105. (nach Anspruch 1)
daß wenigstens ein Deckel um eine exzentrisch auf dem Kanülenkörper angeordnete Achse von Öffnungen in dem Kanülenkörper abschwenkbar sind(Fig.53).

106. (nach Anspruch 1)
daß die stärkste Sogquelle an den über die Reaktionskammer(11) führenden Kanal angeschlossen werden kann(Fig.49).

107. (nach 1 oder 2)
daß jeweils mindestens zwei gleichartige fortlaufend austauschbare Behälter(701,702), dieselbe Sorte von Flüssigkeit enthaltend, im Zusammenhang mit wenigstens einer Dosiervorrichtung(11) vorhanden sind, wobei ein Ventil(703,21) so gesteuert wird, daß nach annähernder Entleerung des einen Behälters(701) der andere Behälter(702), dessen Flüssigkeitsausflußbahn(707,720) bis dahin durch das Ventil(703,721) gesperrt war, nach Aufhebung dieser Sperre seinen Inhalt über die Dosiervorrichtung abgibt, wobei wenigstens eine Anzeigevorrichtung auf die Möglichkeit eines verlustarmen Behälterwechsels hinweist, so daß unterschied liche Dosen durch Ergänzung einer vor der Behälterentleerung(701) ungenügenden Teildosis zur gerade erforderlichen Gesamtdosis ohne Unterbrechung der Injektion verabreicht werden können (Fig.62;Fig63).

108. (nach 1 oder 2 und 107)
daß zwei Behälter(701,702) elastischer Wandbeschaffenheit an ihrem einen Ende einen Öffnungsstutzen(720) aufweisen, welcher durch eine Membran (721) verschlossen ist, und eine Art Kanüle (722) im Inneren des Behälters(701,702), wobei diese Membran(721) von der Schneidkante des Konus(727) eröffnet werden kann, welcher Konus über den Flüssigkeitsabflußschlauch(706) zuerst die Verbindung des Behälters(701) mit der Dosiervorrichtung(711) herstellt, und diese Verbindung über jene Art Kanüle(722) auch mit dem Behälter (702) hergestellt wird, sobald durch Wandannäherung infolge Entleerung des Behälters(701) dessen Kanüle(721) die Membran(721) im Behälter(702) eröffnet hat, wobei beide Behälter mindestens in Richtung der Lage jener Kanülen mittels Druckeinwirkung einander genähert werden, welcher auch mittels Zugfedern erreicht werden könnte. (Fig.64,Fig.65).

109. (nach 1 oder 2 und 108)
daß die Membran(721) durch ein anderes Ventil ersetzt ist.

110. (nach 1 oder 2 und 107)
daß zwei Dosiervorrichtungen für die Dosierung derselben Sorte von Flüssigkeit vorhanden sind, welche zur Flüssigkeitsmischung in einer sich zu einer Gesamtdosis ergänzenden Einzeldose geeignet sind.

111. (nach 1 oder 2 und 107) daß als Kontrollvorrichtung über die Behälterentleerung eine Art Schaltklemme(714) auf einem elastischen Abschnitt des Flüssigkeitsableitungsschlauches(706,707) vorgesehen ist (Fig.62).

112. (nach 1 oder 2 und 107) daß die Kanüle(722) innerhalb des Behälters (701) wenigstens eine seitliche Bohrung(719) zur Flüssigkeitsentleerung aufweist(Fig.64).

113. (nach 1 oder 2 und 107) daß an der Grenzfläche(719) zwischen den Behältern (701,702) eine lösbare Klebeverbindung vorgesehen ist(Fig.64).

114. (nach 1 oder 2 und 107) daß an der Trennfläche(719) zwischen zwei Behältern(701,702) eine Art lösbare Schraubverbindung vorgesehen ist(Fig.64).

115. (nach 1 oder 2 und 107) daß die Verbindung zwischen Bodendeckel (724) undFlüssigkeitsableitungsschlauch(706) eine unlösbare ist(Fig.64).

116. (1 oder 2 und 107) daß im Bereich des Gaszufuhrschlauches(725) zur Überdruckerzeugung über elastischen Behältern ein Rückschlagventil(726,Fig.66) vorgesehen ist (vgl.Fig.36A,630), um beim Auswechseln einzelner Behälter Gas zu sparen.

117. (nach 1 oder 2 und 108) daß die Flüssigkeitsbehälter(701,702) sich innerhalb der Kapseln(565) befinden, deren Bodenfläche den Einstecktrichter(596) für die Verbindung mit einem Einsteckkonus der Schlauchverbindung(710) zur Dosierpumpe umschließen, welcher Einsteckkonus zugleich zur Einführung des Steckkonus eines gleichartigen Behälters geeignet ist, während von der in der anderen Richtung offenen Kapsel(565) von freien Zwischenräumen unterbrochene Fortsätze sich erstrecken, welche in die freien Zwischenräumen der benachbart liegenden Kapsel eingreifen können(Fig.36,A).

118. (nach 1 oder 2) daß bei einer Injektionsvorrichtung die Dosiervorrichtung aus einer Kugel innerhalb eines Schlauches besteht, welche über einen Stift innerhalb einer runden die Kugel umgebenden Kammer taktweise bewegt wird, wobei die Flüssigkeit unter Überdruck in den Schlauch eingeführt wird, während die Kammerentleerung durch die Einwirkung eines Gasdruckpolsters erfolgt(Fig.23).

119. (nach 1 oder 2) daß als Dosiervorrichtung am Ende einer Schlauchverbindung(500) ein Pflock(554) sich befindet mit einer Dosierkammer(557) mit einem Gasballon in deren inneren und einem den Zufluß und Abfluß in diese Dosierkammer regelnden Schalthülse(560, Fig.48).

120. (nach 1 oder 2) daß im Bereich der durch die Vorrichtung erzeugten Hautfalte eine Vorrichtung zur Übermittlung von Meßsignalen bei Durchstrahlung mit Laserlicht vorhanden ist (Fig.33;Fig,36).

121. (nach 1 oder 2 und 5) daß im Kuppenbereich der Saugglocke ein an die Vakuumquelle angeschlossene siebartige Lochbildung vorhanden ist, welche die Anlage der Hautkuppe an die Formgebung der Saugglocke erleichtert, so daß im Kuppenbereich tangentiale Strahlen eine Streckenkonstanz beim Verlauf innerhalb der Haut aufweisen(Fig.33).

122. (nach 1 oder 2) daß das Zentrum der Hautkuppe durch einen schaftverbreiternden Teller gebildet wird, um durch Abstützung der Hautkuppe deren Formgebung für optische Meßzwecke festzulegen und eventuell nach der Messung eine Einspritzung zu ermöglichen, wozu der Teller dann auf dem Schaft verschieblich angeordnet ist.

123. (nach 1) daß mittels optischer Kontrolle die Füllung der Reaktionskammer(11) festgestellt wird(Fig.47).

124. (nach 1 und 123) daß nach Bestätigung der ausreichenden Füllung der Reaktionskammer die Kanülenrotation abgebrochen wird und bei Ausbleiben der Bestätigung unter Signalgebung der Abbruch der Anwendungsfunktionen eingeleitet wird(Fig.47).

125. (nach 1 und ) daß die Meßzone von einem eingebrachten Stanzteil gebildet wird, welches aus einem Teststreifen, beispielsweise für Glukose, gewonnen wird.

126. (nach 1 und 2) daß im Randbereich einer der Form nach durch die Gerätebeschaffenheit festgelegten Hautkuppe mindestens drei Lichtquellen so angeordnet sind, daß der mittlere auf das für die Punktion oder Messung bestimmte Hautareal gerichtet ist(Fig.67).

127. (nach 1 oder 2) daß auf das zur Punktion oder Messung vorgesehene Hautareal eine Lichtquelle gerichtet ist, deren Reflexionswerte durch Vergleich zur Hautkontrolle benutzt wird(Fig.66). wobei die Lichtquelle durch die Austrittsöffnung für die Flüssigkeit eines Injektors hindurch wirksam wird.

128. (nach 1 oder 2) daß bei einer optischen Hautüberwachung die früher erhobenen Meßwerte mit den aktuellen Werten verglichen werden und nach Abweichung von einer Toleranzgrenze, eine Hautpunktion unterbunden wird, wobei die Toleranzgrenze aus den nacheinander ermittelten Abweichungen mehrerer Meßstellen

festgelegt werden und der Benutzer die Sperrwirkung der Vorrichtung auf die Punktion unter festsetzbaren Bedingungen aufheben kann.

129. (nach 1 oder 2)
daß die Injektion wenigstens zeitweise auch vor Abschluß der Stoffwechselmessung durchgeführt werden kann, um die Zeit des Kontaktes der Einrichtung mit der Haut abzukürzen.

130. (nach 1 oder 2)
daß eine Saugglocke im Inneren wenigstens zwei ringförmige Kanten zur Hautanlagerung aufweist, bei getrennter Luftabsaugung in den so entstandenen ringförmigen Zonen, welche dazu benutzt werden kann, die so abgeteilten Hautzonen unter verschiedene Luftdruckbedingungen zu bringen (Fig.49).

131. (nach 1 oder 2)
daß die Saugglocke eine hochelastische Wandbeschaffenheit aufweist, so daß durch Andruck gegen die Haut, die von ihren Rändern eingeschlossene Luft wenigstens teilweise zeitwei lig entweichen kann(Fig.27).

## Ansprüche

1. Einrichtung zur Stoffwechselkontrolle bei Mensch oder Tier mit während direkten Hautkontaktes wenigstens eingeleiteter Messung, welche auch -wenn beabsichtigt- mit einer Einspritzung verbunden sein kann,
**bestehend aus**
einem Gehäuse(155) mit wenigstens einer Steuervorrichtung(Fig.35A(560) und wenigstens einer Schaltvorrichtung(Fig.37), von einer Gestaltung-(Fig.36(670), welche die sichere Auflage der Einrichtung während der notwendigen Dauer des Hautkontaktes gewährleistet und einer Punktionsvorrichtung(Fig.47;Fig.18;Fig.59) für die Aufnahme von Blutbestandteilen oder Gewebsflüssigkeit und eine Vorrichtung für deren Ableitung in eine Reaktionskammer(Fig.4(11); Fig.41(3),
bestehend weiterhin aus einem dort wirkenden Signalgeber und Signalempfänger(Fig.42(18), vorzugsweise als Strahlenquelle(7) und -detektor(8) eines Reflexphotometers(18) zur Signalverarbeitung (Fig.54(18), um Meßwerte über die zu ermittelnde Substanz zu erstellen und wenigstens eine Anzeigevorrichtung, die ermittelten Meßwerte erkennbar zu machen und in der Regel einer Registriervorrichtung(Fig.54(668) für die Meßwerte und andere Daten, wobei die signalverarbeitende Vorrichtung und die Registriervorrichtung wenigstens teilweise auf weitere Gehäuse verteilt sein können, bei wenigstens zeitweiser wenigstens funktioneller Koppelung derselben, und bevorzugterweise aus einer Vorrichtung zur Kontrolle (Fig.47(7-8) ausreichender Füllung der Reaktionskammer, und wobei auch eine Vorrichtung zur Kontrolle der Eignung der Hautfalte zur Punktion vorhanden sein kann(Fig.59(7-8)(48), falls zusätzlich beabsichtigt verbunden mit Vorrichtung zur Einspritzung(Fig.36-(592)(599);Fig.42 (643) von Flüssigkeit unter die Haut über wenigstens eine Dosiervorrichtung-(Fig.36(599) bei Lagerung der Flüssigkeit in wenigstens zwei gleichartigen Behältern(Fig.36(701,702) je Flüssigkeitssorte.

2. Einrichtung zur Stoffwechselkontrolle bei Mensch oder Tier mit während direkten Hautkontaktes wenigstens eingeleiteter Messung, welche auch -wenn beabsichtigt- mit einer Einspritzung verbunden sein kann, **bestehend aus** einer Vorrichtung zur Anhebung einer Hautfalte (Fig.24-(71,72);Fig.27(14);Fig.35(14,562); Fig.36 (14,590)-;Fig.61(310,311) und deren Fixierung im Zusammenhang mit einem Gehäuse mit einer Gestaltung-(Fig.36(570,636), welche die sichere Befestigung jener Vorrichtung zur Erzeugung einer Hautfalte während der notwendigen Dauer des Hautkontaktes gewährleistet, und einer Vorrichtung (Fig.33(264)-;Fig.34), welche gewährleistet, daß Laserlicht tangential durch die Hautfalte hindurch einem Detektor zur Vermittlung von Meßsignalen für die Bestimmung des Gehaltes der zu ermittelnden Substanz zugeleitet wird, wobei die Streckenlänge der Meßstrahlen innerhalb der Haut weitgehend durch tangentiale Strahlenführung zur in ihrer Krümmung am Strahlendurchtrittsort festgelegter Hautfalte weitgehend vorbestimmt ist, wobei der Photometer(Fig. 54(18) und eine Registriervorichtung(Fig.54(468) auf weitere Gehäuse, bei wenigsten zeitweise wenigstens funktioneller Koppelung, verteilt sein können, falls zusätzlich beabsichtigt verbunden mit einer Vorrichtung zur Einspritzung/Fig.36(599)-;Fig.42 (643) von Flüssigkeit unter die Haut über wenigstens eine Dosiervorrichtung(Fig.36(599) bei Lagerung der Flüssigkeit in wenigstens zwei gleichartigen Behältern(Fig.36 (701,702) je Flüssigkeitssorte.

3.Einrichtung nach Anspruch 1 oder 2,
dadurch gekennzeichnet,
daß eine Art Klemmrahmen oder Klemmbacken vorhanden sind, welche die Haut an der Basis einer Hautkuppe, diese eventuell erst hervorbringend, von den Rändern her nähert(3).

4. Einrichtung nach Anspruch 1 oder 2,
dadurch gekennzeichnet,
daß eine Zugvorrichtung(4) zur Anhebung der Haut in Form einer Hautkuppe vorhanden ist, wobei als Zugvorrichtung eine Saugglocke(5) dienen kann, welche auch elastisch sein kann(131).

5. Einrichtung nach Anspruch 1 oder 2 und 5,
dadurch gekennzeichnet,
daß Vorrichtungen zur Unterdruckregulierung vorhanden sind, vorzugsweise aus Unterdruckprüfer und Verteilerventil bestehend und durch eine

Schaltvorrichtung betätigt(6), und wobei der erforderliche Unterdruck mittels einer Vakuumquelle-(7,8,9) wieder hergestellt werden kann und wobei auch ein zu starker Überdruck mittels einer Regelvorrichtung (11), die aber auch die Vakuumquelle selbst sein kann, vermieden wird, wozu auch eine optische Kontrollvorrichtung zur Überwachung der Folgen der Sogeinwirkung auf die Haut vorhanden sein kann(6).

6. Einrichtung nach Anspruch 1 oder 2, dadurch gekennzeichnet, daß die Ebene des zeitweisen Hautkontaktes mti der größten Ausdehnungslänge des Gehäuses parallel verläuft(13), wobei die Funktionselemente innerhalb eines teilweise wandbeweglichen Gehäuses so angeordnet sein können, daß bei Abbiegung des Gehäuses im Umfang der vorgegebenen Beweglichkeit, das Zusammenspiel der Funktionen nicht gestört wird(14). wobei die Saugglocke innerhalb einer Längsmulde der Bodenfläche der Einrichtung angeordnet sein kann(15).

7.Einrichtung nach Anspruch 1 oder 2, dadurch gekennzeichnet, daß die Saugglocke mittels einer Art von Balancier-Federn gegen die Haut gedrückt wird, während die Aufstützung der Einrichtung auf die Haut weiter seitlich vom Saugglockenrand erfolgt(16), wobei wenigstens zwei Federn, bevorzugterweise Druckfedern, so winkelverschieden zu der Saugglocke angeordnet sein können, daß sie nacheinander in verschiedener und wechselnder Stärke in einem Zusammenwirken gemäß eines Kräfteparallelogrammes der Anhebung der Saugglocke entgegenwirken, um deren Hautandruck zu bewirken(17).

8. Einrichtung nach Anspruch 1 oder 2, dadurch gekennzeichnet, daß unterschiedliche wenigstens segmentweise ringförmige Zonen unterschiedlichen Druckes um eine Hautfalte erzeugt werden(18,130).

9. Einrichtung nach Anspruch 1 oder 2, dadurch gekennzeichnet, daß insbesondere auch für den Injektionsgebrauch eine Art elektromagnetische Schaltvorrichtung vorhanden ist, welche durch Beeinflussung der Funktionsweise eines elektrischen Kraftantriebes durch Art und Weise der Stromzufuhr in wenigstens zwei Arbeitsfunktionen eingesetzt werden kann(22).

10. Einrichtung nach Anspruch 1 oder 2, dadurch gekennzeichnet, daß bei der Bewegung mittels der Hand mehrere Transportsysteme der Einrichtung durch Federspannung in einem Zuge in Funktionsbereitschaft gesetzt werden(40,43,44,45).

11. Einrichtung nach Anspruch 1, dadurch gekennzeichnet, daß ein Schiebestift die Punktionsgriffel oder Kanülen und gegebenenfails auch Teile von diesen (93)-der Haut in der Senkrechten nähert und wieder von ihr entfernt(32), wobei mehrere Punktionsgriffel in einer Kette nebeneinander angeordnet sind(36) vor Gebrauch verschlossen, wobei sie auch innerhalb von Hülsen luftdicht gelagert sein können(35), und bei Verwendung von Deckeln dieselben über wenigstens einen Faden mit dem Punktionsgriffel verbunden sein können(37,38).

12. Einrichtung nach Anspruch 1 oder 2, dadurch gekennzeichnet, daß wenigstens ein in die Haut eingedrungener schneidender Teil des Schaft des Punktionsgriffels oder der Kanüle innerhalb der Haut und auch mit wenigstens einem schneidenden Teil quer zu dieser bewegt wird(50), wobei die Kanüle wenigstens eine den Schaftdurchmesser überragende Schneidkante aufweisen kann(51) und wobei eine Schneidfläche des Punktionsgriffels exzentrisch angeordnet sein kann(57), um die Eröffnung von Blutgefäßen zu fördern.

13. Einrichtung nach Anspruch 1 oder 2, dadurch gekennzeichnet, daß Mittel vorhanden sind, die gewährleisten, daß eine Kanüle zwar in die Haut eindringen, diese aber erst dann durchdringen kann, wenn die Blutaufnahme abgeschlossen ist, falls eine Einspritzmöglichkeit vorgesehen ist, wobei noch eine besondere Kanalführung für die Blutaufnahme vorgesehen sein kann(60,62,63,64,65,72;58,59,67,68,70 73,61), welche den Zufluß von Blut zur Meßzone oder Reaktionskammer durch besondere Gestaltungen zu fördern.

14.Einrichtung nach Anspruch 1, dadurch gekennzeichnet, daß im Anschluß an die Reaktionskammer, welche zur Abfilterung von Blutkörperchen auch eine Filtermembran aufweisen kann(87), wenigstens ein Sogkanal vorhanden ist, welcher zeitweise in mit einer Vakuumquelle verbunden wird, um den Einstrom von Blut zu begünstigen(74,75,81), wobei die Vakuumquelle auch die Saugglocke sein kann, und zur Ableitung des Blutes von der Meßzone von dort gespeicherter Unterdruck verwendet werden kann-(84).

15. Einrichtung nach Anspruch 1 oder 2, dadurch gekennzeichnet, daß zur Blutzuführung zur Meßzone und zur Reinigung derselben eine Art übertragendes kapilläres Leitelement verwendet wird(75,76,96) bei relativer Verschiebung der genannten Elemente gegeneinander.

16. Einrichtung nach Anspruch 1, dadurch gekennzeichnet, daß die Füllung der Reaktionskammer mittels einer Meßanordnung(123) festgestellt wird. welche weitere Funktionsabläufe steuert(124), wobei bevorzugterweise ein Photometer verwendet wird und vozugsweise bei einer Strahlenführung innerhalb einer Längsbohrung des Punktionsgriffels, was auch innerhalb eines Schiebestiftes stattfinden kann, und wobei vorzugsweise ein zeitweiseerzusätzlicher Verschluß der Öffnung der Reaktionskammer vorgesehen ist(103).

17. Einrichtung nach Anspruch 1 oder 2, dadurch gekennzeichnet, daß die optische Kontrolle der Hautoberfläche reflexphotometrisch durch den Bereich der Öffnung ·einer Injektionsvorrichtung erfolgt (127), wobei auch ein Vergleich aktueller Meßergebnisse mit früher gewonnenen ausgewertet werden kann, um die Punktion krankhaft veränderter Hautstellen auszuschließen.

18. Einrichtung nach Anspruch 1 oder 2, dadurch gekennzeichnet, daß jeweils mindestens zwei gleichartige fortlaufend austauschbare Behälter(701,702) dieselbe Sorte von Flüssigkeit enthaltend, im Zusammenhang mit wenigstens einer Dosiervorrichtung(11) vorhanden sind, wobei ein Ventil(703,21) so gesteuert wird, daß nach annähernder Entleerung des einen Behälters(701) der andere Behälter(702), dessen Flüssigkeitsausflußbahn(707,720) bis dahin durch das Ventil(703,721) gesperrt war, nach Aufhebung dieser Sperre seinen Inhalt über die Dosiervorrichtung abgibt, wobei wenigstens eine Anzeigevorrichtung auf die Möglichkeit eines verlustar men Behälterwechsels hinweist, so daß unterschiedliche Dosen durch Ergänzung einer vor der Behälterentleerung(701) ungenügenden Teildosis zur gerade erforderlichen Gesamtdosis ohne Unterbrechung der Injektion verabreicht werden können (107,108).

19. Einrichtung nach Anspruch 1 oder 2, dadurch gekennzeichnet, daß bei einer Injektionsvorrichtung die Dosiervorrichtung aus einer Kugel innerhalb eines Schlauches besteht, welche über einen Stift innerhalb einer runden die Kugel umgebenden Kammer taktweise bewegt wird, wobei die Flüssigkeit unter Überdruck in den Schlauch eingeführt wird, während die Kammerentleerung durch die Einwirkung eines Gasdruckpolsters erfolgt(118).

20. Einrichtung auch nach 2, dadurch gekennzeichnet, daß ein wenigstens teilweise adhäsives Zugpflaster zwischen eine Vorrichtung zur Überdruckeinspritzung und die Haut gebracht wird, so daß die Haut vor der Injektion durch eine Lücke dieses Pflasters vom übrigen Gewebe durch Anheben einer Falte entfernt werden kann.

Fig.1

Fig.2

Fig.3

Fig.4

Fig.5

Fig.7

Fig.6

Fig.8

Fig. 9

Fig.10

Fig.11

Fig.12

Fig.13

Fig.14

Fig.15

Fig.16

Fig.17

Fig.18

Fig.19

Fig. 20

Fig. 21

Fig. 22

Fig. 23

Fig. 24

Fig. 25

Fig. 26

Fig. 27

243
244
239
5
235
237
249
236 238

242
247
245 241 240

257

I II

*Fig.28*

*Fig. 29*

245
246
A B
248

I 251 248
250
241

II 253 252

III

Fig. 30

Fig. 31

Fig. 32

Fig. 33

Fig. 34

Fig. 35

Fig. 36

Fig. 37

Fig. 38

Fig. 39

Fig. 40

Fig. 41

Fig. 42

Fig. 43

Fig. 44

Fig. 45

Fig. 46

Fig. 47

Fig. 48

## Fig. 49

## Fig. 50

## Fig. 51

## Fig. 52

## Fig. 53

## Fig. 54

Fig. 55

Fig. 56

Fig. 57

Fig. 58

17/19

289
266
298
268
24
580
301
269
299
237
48
2    265    5

**Fig. 59**

301
293    300

228    218    225    213    226    224    223

221    227

**Fig. 60**

**Fig. 61**

313    I    II
340
314
310
48    311    314

*Fig.62*

*Fig.63*

*Fig.64*

*Fig.65*

Fig.66

Fig.67